# EUROPEAN PATENT APPLICATION

(11) **EP 2 298 764 A1**
(43) Date of publication of application: **23.03.2011**
(21) Application number: 10010919.8
(22) Date of filing: 21.11.2006
(51) Int. Cl.: C07D 413/04, C07D 413/14, A61K 31/454, A61P 25/00

(54) **4-Oxadiazolyl-piperidine compounds and use thereof**

(30) Priority: 21.11.2005 US 739107 P
(62) Divisional of application: 06829087.3
(71) Applicant: Purdue Pharma LP, Stamford, CT 06901-3431 (US)
(72) Inventor: Tafesse, Laykea, Robbinsville, NJ 08691 (US)
(74) Representative: Maiwald, Walter

(57) **Abstract**

4-Oxadiazolyl-piperidine compounds of formula (I) and (II), their compositions and use for the treatment of pain and diarrhoea.

## Description

### 1. Field of the Invention

The present invention relates to 4-Oxadiazolyl-piperidine Compounds, compositions comprising a 4-Oxadiazolyl-piperidine Compound and methods for preventing or treating pain or diarrhea in an animal comprising administering to an animal in need of such prevention or treatment an effective amount of a 4-Oxadiazolyl-piperidine Compound.

### 2. Background of the Invention

Pain is the most common symptom for which patients seek medical advice and treatment. Pain can be acute or chronic. While acute pain is usually self-limited, chronic pain can persist for 3 months or longer and lead to significant changes in a patient's personality, lifestyle, functional ability or overall quality of life (K.M. Foley, Pain, in Cecil Textbook of Medicine 100-107, J.C. Bennett and F. Plum eds., 20th ed. 1996).

Pain has been traditionally managed by administering a non-opioid analgesic, such as acetylsalicylic acid, choline magnesium trisalicylate, acetaminophen, ibuprofen, fenoprofen, diflusinal and naproxen; or an opioid analgesic, such as morphine, hydromorphone, methadone, levorphanol, fentanyl, oxycodone and oxymorphone. *Id.*

United States Patent No. 6,576,650 B1, United States Patent 6,166,039, and United States Patent No. 5,849,761, to Yaksh and United States Patent No. 6,573,282, to Yaksh et al. describe 1,4-substituted piperidine derivatives allegedly useful as peripherally active anti hyperalgesic opiates.

United States Patent No. 6,362,203 B1 to Mogi et al. describes 4-hydroxy-4-phenylpiperidine derivatives that are alleged to exhibit peripheral analgesic action.

Canadian Patent Publication No. 949560 of Carron et al. describes piperidine derivatives bearing substituents at the 1 and 4 positions that are alleged to be useful as analgesics.

International Publication No. WO 02/38185 A2 of Dunn et al*.* describes 1,4 substituted piperidine compounds that are allegedly useful as an antihyperalgesic opiate.

The Abstract of International Publication No. WO 01/70689 A1 also discloses piperidine derivatives carrying substituents at the 1 and 4 positions that are allegedly useful as opioid δ receptor agonists.

Traditional opioid analgesics exert their pharmacological activity once they have passed through the blood-brain barrier. But this passage through the blood-brain barrier can lead to undesirable central nervous system-mediated side effects, such as respiratory depression, increased drug tolerance, increased drug dependence, constipation and unwanted euphoria.

There remains a clear need for new drugs that are useful for treating or preventing pain or diarrhea and that reduce or avoid one or more side effects associated with traditional therapy for treating pain or diarrhea.

Citation of any reference in Section 2 of this application is not an admission that such reference is prior art to the present application.

### 3. Summary of the Invention

The present invention encompasses compounds having the formula (I): 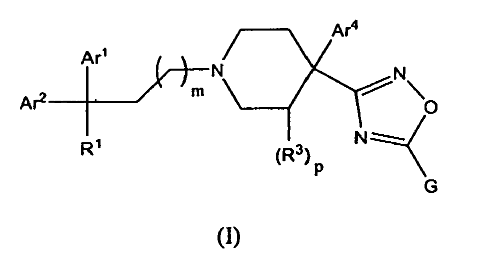 and pharmaceutically acceptable salts thereof, wherein:

Ar' is -C₃-C₈ cycloalkyl, phenyl, naphthyl, anthryl, phenanthryl or -(5- to 7-membered) heteroaryl, each being unsubstituted or substituted with one, two, or three R² groups;

Ar² is phenyl, naphthyl, anthryl, phenanthryl or -(5- to 7-membered) heteroaryl, each being unsubstituted or substituted with one, two, or three R² groups;

Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;

G is -H, -C(O)(CH₂)ₙCO₂R⁴ -C(O)(CH₂)ₙR⁵, -(C₁-C₅ alkylene)CO₂R⁴, or -(C₁-C₅ alkylene)R⁵;

R¹ = -H, -C(O)NH₂, -C(O)NHOH, -CO₂R⁴, -CHO, -CN, -(C₁-C₄ alkyl), -C(O)NH(C₁-C₄ alkyl), -C(O)N(C₁-C₄ alkyl)₂, -CF₃, -CHF₂, -CH₂F,

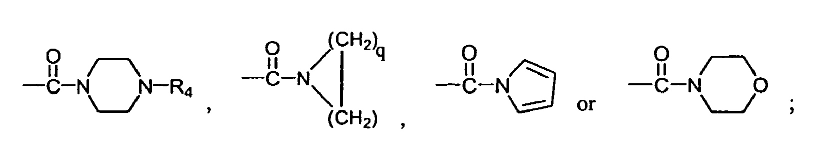

R² and R³ are each independently -halogen, -C₁-C₃ alkyl, -O(C₁-C₃ alkyl), -NH(C₁-C₃ alkyl), -N(C₁-C₃ alkyl)₂, -CF₃, or -OCF₃;

R⁴ = -H, -C₁-C₁₀ alkyl, -CH₂O(C₁-C₄ alkyl), -CH₂N(C₁-C₄ alkyl)₂, or -CH₂NH(C₁-C₄ alkyl);

R⁵ = -NH₂, -NHSO₂R₄, -C(O)NH₂, -C(O)NHOH, - SO₂NH₂, -C(O)NH(C₁-C₄ alkyl), -C(O)N(C₁-C₄ alkyl)₂, -SO₂NH(C₁-C₄ alkyl), -SO₂N(C₁-C₄ alkyl)₂, -H, -OH, -CN, or -C₃-C₈ cycloalkyl, phenyl, naphthyl, anthryl, phenanthryl, -(5- to 7-membered) heteroaryl each C₃-C₈ cycloalkyl, phenyl, naphthyl, anthryl, phenanthryl, -(5- to 7-membered) heteroaryl being unsubstituted or substituted with one or more R² groups;
m = an integer ranging from 0 to 4;
n = an integer ranging from 1 to 4;
p=0 or 1; and
q = an integer ranging from 1 to 6.

The present invention also encompasses compounds having the formula (II): 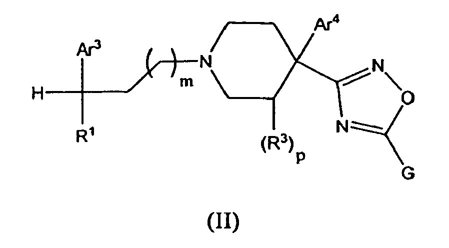 and pharmaceutically acceptable salts thereof, wherein:

Ar³ is phenyl, naphthyl, anthryl, phenanthryl, or -(5- to 7-membered) heteroaryl, each being unsubstituted or substituted with one, two, or three R² groups;

Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;

G = H, -C(O)(CH₂)ₙC(O)OR⁴, -C(O)(CH₂)ₙR⁵ -(C₁-C₅ alkylene)COOR⁴, or -(C₁-C₅ alkylene)R⁵;

R¹ = -H, -C(O)NH₂, -C(O)NHOH, -CO₂R⁴, -CHO, -CN, -(C₁-C₄ alkyl), -C(O)NH(C₁-C₄ alkyl), -C(O)N(C₁-C₄ alkyl)₂, -CF₃, -CHF₂, -CH₂F,

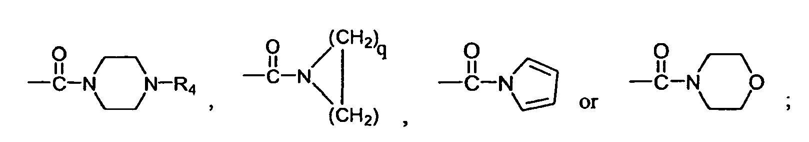

R² and R³ are each independently halogen, -C₁-C₃ alkyl, -O(C₁-C₃ alkyl), -NH(C₁-C₃ alkyl), -N(C₁-C₃ alkyl)₂, -CF₃, or -OCF₃;

R⁴= -H, -C₁-C₁₀ alkyl, -CH₂O(C₁-C₄ alkyl), -CH₂N(C₁-C₄ alkyl)₂, or -CH₂NH(C₁-C₄ alkyl);

R⁵ = -NH₂, -NHSO₂R⁴, -C(O)NH₂, -C(O)NHOH, - SO₂NH₂, -C(O)NH(C₁-C₄ alkyl), -C(O)N(C₁-C₄ alkyl)₂, -SO₂NH(C₁-C₄ alkyl), -SO₂N(C₁-C₄ alkyl)₂, -H, -OH, -CN, or -C₃-C₈ cycloalkyl, phenyl, naphthyl, anthryl, phenanthryl, -(5- to 7-membered) heteroaryl each -C₃-C₈ cycloalkyl, phenyl, naphthyl, anthryl, phenanthryl, -(5- to 7-membered) heteroaryl being unsubstituted or substituted with one or more R² groups;
m = an integer ranging from 0 to 4;
n = an integer ranging from 1 to 4;
p = 0 or 1; and
q = an integer ranging from 1 to 6.

A compound of formula (I) or (II) or a pharmaceutically acceptable salt thereof (each being a "4-Oxadiazolyl-piperidine Compound") is useful for treating or preventing pain or diarrhea in an animal.

The invention also relates to compositions comprising a 4-Oxadiazolyl-piperidine Compound and a pharmaceutically acceptable carrier or excipient. The present compositions are useful for treating or preventing pain or diarrhea in an animal.

The invention also relates to kits comprising a container containing an effective amount of a 4-Oxadiazolyl-piperidine Compound and instructions for using it to treat or prevent pain or diarrhea.

The invention further relates to methods for preventing pain or diarrhea in an animal, comprising administering to an animal in need thereof an effective amount of a 4-Oxadiazolyl-piperidine Compound. In further aspects of this embodiment, these methods for preventing pain or diarrhea in an animal further comprise administering an effective amount of an opiod analgesic, a non-opioid analgesic, an anti-emetic agent, or a combination thereof.

The invention further relates to methods for treating pain or diarrhea in an animal, comprising administering to an animal in need thereof an effective amount of a 4-Oxadiazolyl-piperidine Compound. In further aspects of this embodiment, these methods for treating pain or diarrhea in an animal further comprise administering an effective amount of an opiod analgesic, a non-opioid analgesic, an anti-emetic agent, or a combination thereof.

The invention also relates to the use of a 4-Oxadiazolyl-piperidine Compound in preparing a medicament useful to treat or prevent pain or diarrhea.

The invention still further relates to methods for stimulating opioid-receptor function in a cell, comprising contacting a cell capable of expressing an opioid receptor with a 4-Oxadiazolyl-piperidine Compound.

The invention still further relates to methods for preparing a pharmaceutical composition, comprising the step of admixing a 4-Oxadiazolyl-piperidine Compound with a pharmaceutically acceptable carrier or excipient.

The present invention may be understood more fully by reference to the following detailed description and illustrative examples, which are intended to exemplify non-limiting embodiments of the invention.

### 4. Detailed Description of the Invention

### 4.1 Definitions

As used herein, the terms used above have the following meaning:

"-C₁-C₃ alkyl" means a straight or branched non-cyclic hydrocarbon chain having from 1 to 3 carbon atoms. Representative straight chain and branched chain -C₁-C₃ alkyls include -methyl, -ethyl, -*n*-propyl and isopropyl.

"-C₁-C₄ alkyl" means a straight or branched non-cyclic hydrocarbon chain having from 1 to 4 carbon atoms. Representative straight chain -C₁-C₄ alkyls include methyl, -ethyl, -*n*-propyl, and -*n*-butyl. Representative branched chain -C₁-C₄ alkyls include -isopropyl, -*sec*-butyl, -isobutyl, and -tert-butyl.

"-C₁-C₆ alkyl" means a straight or branched non-cyclic hydrocarbon chain having from 1 to 6 carbon atoms. Representative straight chain -C₁-C6 alkyls include methyl, -ethyl, -n-propyl, -*n*-butyl, -n-pentyl and-n-hexyl. Representative branched chain -C₁-C₆ alkyls include -isopropyl, -*sec*-butyl, -isobutyl, -*tert*-butyl, -isopentyl, -neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl and 3,3-dimethylbutyl.

"-C₁**-**C₁₀ alkyl" means a straight chain or branched non-cyclic hydrocarbon having from 1 to 10 carbon atoms. Representative straight chain -(C₁-C₁₀) alkyls include methyl, -ethyl, -n-propyl, -*n*-butyl, -n-pentyl, -*n*-hexyl, -*n*-heptyl, -*n*-octyl, -*n*-nonyl, and -n-decyl. Representative branched -(C₁**-**C₁₀) alkyls include isopropyl, *sec*-butyl, isobutyl, -tert-butyl, isopentyl, neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-methylhexyl, 2-methylhexyl, 3-methylhexyl, 4-methylhexyl, 5-methylhexyl, 1,2-dimethylpentyl, 1,3-dimethylpentyl, 1,2-dimethylhexyl, 1,3-dimethylhexyl, 3,3-dimethylhexyl, 1,2-dimethylheptyl, 1,3-dimethylheptyl, and 3,3-dimethylheptyl.

"-C₁-C₅ alkylene" means a straight chain or branched, non-cyclic, divalent hydrocarbon having from 1 to 5 carbon atoms. Representative straight chain -C₁-C₅ alkylene groups are -CH₂-, -(-CH₂)₂-, -(CH₂)₃-, -(CH₂)₄- and -(C₂)₅-. Representative branched -C₂-C₅ alkylene groups include -CH(CH₃)-, -C(CH₃)₂--CH(CH₃)CH₂-, -CH₂CH(CH₃)-, -CH₂C(CH₃)₂-, -C(CH₃)₂CH₂-, -CH(CH₃)CH(CH₃)-, -CH₂C(CH₃)₂CH₂-, -(CH₂ )₂C(CH₃)₂-, -(CH₃)₂(CH₂)₂C- and -CH(CH₃)CH₂CH(CH₃)-.

"-C₃-C₈ cycloalkyl" means a saturated cyclic hydrocarbon having from 3 to 8 carbon atoms. Representative -C₃-C₈ cycloalkyls are -cyclopropyl, -cyclobutyl, -cyclopentyl, -cyclohexyl, -cycloheptyl and -cyclooctyl.

"-(5- to 7-membered)heteroaryl" means an aromatic heterocycle ring of 5 to 7 members, wherein at least one carbon atom of the ring is replaced with a heteroatom independently selected from nitrogen, oxygen, and sulfur. The -(5- to 7-membered)heteroaryl's ring contains at least one carbon atom. Representative -(5-to 7-membered)heteroaryls include pyridyl, furyl, thiophenyl, pyrrolyl, oxadiazolyl, imidazolyl, thiazolyl, pyrazolyl, isothiazolyl, pyridazinyl, pyrimidinyl, pyrazinyl, thiadiazolyl, and triazinyl.

"-Halogen" means -F, -Cl, -Br, or -I.

The term "animal," includes, but is not limited to, a cow, ape, monkey, chimpanzee, baboon, horse, sheep, pig, chicken, turkey, quail, cat, dog, mouse, rat, rabbit, guinea pig and human.

The phrase "pharmaceutically acceptable salt," as used herein, is a salt formed from an acid and the basic nitrogen group of a 4-Oxadiazolyl-piperidine Compound. Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate,p-toluenesulfonate, and pamoate (i.e., 1,1'-methylene-bis-(2-hydroxy-3-naphthoate)) salts. The term "pharmaceutically acceptable salt" also refers to a salt of a 4-Oxadiazolyl-piperidine Compound having an acidic functional group, such as a carboxylic acid functional group, and a pharmaceutically acceptable inorganic or organic base. Illustrative bases include, but are not limited to, hydroxides of alkali metals such as sodium, potassium and lithium; hydroxides of alkaline earth metal such as calcium and magnesium; hydroxides of other metals, such as aluminum and zinc; ammonia; and organic amines, such as unsubstituted or hydroxy substituted mono-, di-, or trialkylamines; dicyclohexylamine; tributylamine; pyridine; N-methyl-N-ethylamine; diethylamine; triethylamine; mono-, bis- or tris-(2-hydroxy-lower alkyl amines), such as mono- bis-or tris-(2- hydroxyethyl)amine, 2-hydroxy-*tert*-butylamine, or tris-(hydroxymethyl)methylamine, N, N-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

The terms "treat" "treatment of" and "treating" pain or diarrhea include the lessening of the severity of, or cessation of, pain or diarrhea. In one embodiment, "treat" "treating" or "treatment of" includes inhibiting, for example decreasing, the overall frequency of episodes of pain or diarrhea, respectively.

The terms "prevent" "prevention of'' and "preventing" pain or diarrhea include the avoidance of the onset of pain or diarrhea, respectively.

The phrase "opioid receptor" means a δ-opioid receptor, a κ-opioid receptor, a µ-opioid receptor or an ORL-1 receptor.

The phrase "effective amount" when used in connection with a 4-Oxadiazolyl-piperidine Compound means an amount of the 4-OxadiazolyL-piperidine Compound that is useful for treating or preventing pain or diarrhea in an animal or stimulating opioid-receptor function in a cell.

The phrase "effective amount" when used in connection with another therapeutic agent means an amount useful to providing the therapeutic effect of that particular therapeutic agent.

When a first group is "substituted with one or more" second groups, each of one or more of the first group's hydrogen atoms is replaced with a second group.

In one embodiment, a first group is substituted with up to three second groups.

In another embodiment, a first group is substituted with one or two second groups.

In another embodiment, a first group is substituted with only one second group.

### 4.2 The 4-Oxadiazolyl-piperidine Compounds

As stated above, the present invention encompasses 4-Oxadiazolyl-piperidine Compounds having the formula (I): 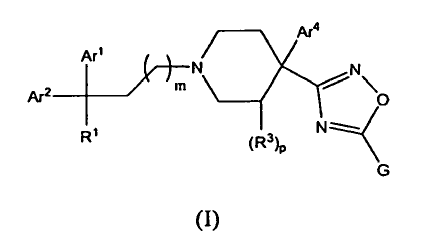 and pharmaceutically acceptable salts thereof, wherein Ar¹, Ar², Ar⁴, R¹-R³, G, n, m, p and q are as defined above.

In one embodiment, the 4-Oxadiazolyl-piperidine Compounds of formula (I) are those wherein G is -H.

In another embodiment, the 4-Oxadiazolyl-piperidine Compounds of formula (I) are those wherein G is -C(O)(CH₂)ₙCO₂R⁴.

In another embodiment, the 4-Oxadiazolyl-piperidine Compounds of formula (I) are those wherein G is -C(O)(CH₂)ₙC(O)OH.

In another embodiment, the 4-Oxadiazolyl-piperidine Compounds of formula (I) are those wherein G is -C(O)(CH₂)ₙC(O)OR⁴ and R⁴ = -C₁-C₁₀ alkyl.

In another embodiment, the 4-Oxadiazolyl-piperidine Compounds of formula (I) are those wherein G is -C(O)(CH₂)ₙC(O)OR⁴ and R⁴ = -CH₂O(C₁-C₄ alkyl).

In another embodiment, the 4-Oxadiazolyl-piperidine Compounds of formula (I) are those wherein G is -C(O)(CH₂)ₙC(O)OR⁴ and R⁴= -CH₂NH(C₁-C₄ alkyl) or -CH₂N(C₁**-**C₄ alkyl)₂.

In another embodiment, the 4-Oxadiazolyl-piperidine Compounds of formula (I) are those wherein G = -(C₁**-**C₅ alkylene)C(O)OR⁴.

In another embodiment, the 4-Oxadiazolyl-piperidine Compounds of formula (I) are those wherein G = -CH₂C(O)OR⁴.

In another embodiment, the 4-Oxadiazolyl-piperidine Compounds of formula (1) are those wherein G = -CH₂C(O)OCH₂CH₃-

In another embodiment, the 4-Oxadiazolyi-piperidine Compounds of formula (I) are those wherein G = -(CH₂)₂C(O)OR⁴.

In another embodiment, the 4-Oxadiazolyl-piperidine Compounds of formula (I) are those wherein G = -(CH₂)₃C(O)OR⁴.

In another embodiment, the 4-Oxadiazolyl-piperidine Compounds of formula (I) are those wherein G = -(CH₂)₄C(O)OR⁴.

In another embodiment, the 4-Oxadiazolyl-piperidine Compounds of formula (I) are those wherein G = -(CH₂)₅C(O)OR⁴.

In another embodiment, the 4-Oxadiazolyl-piperidine Compounds of formula (I) are those wherein G is -C(O)(CH₂)ₙR⁵ and R⁵ = -NHSO₂R⁴.

In another embodiment, the 4-Oxadiazolyl-piperidine Compounds of formula (I) are those wherein G is -C(O)(CH₂)ₙR⁵ and R⁵ = -C(O)NH₂, -C(O)NHOH, -C(O)NH(C₁-C₄ alkyl), or -C(O)N(C₁C₄ alkyl)₂.

In another embodiment, the 4-Oxadiazolyl-piperidine Compounds of formula (I) are those wherein G is -C(O)(CH₂)ₙR⁵ and R⁵ = -SO₂NH₂, -SO₂NH(C₁-C₄ alkyl), or -SO₂N(C₁-C₄ alkyl)₂.

In another embodiment, the 4-Oxadiazolyl-piperidine Compounds of formula (I) are those wherein G is -C(O)(CH₂)ₙR⁵ and R⁵ = -NHSO₂H.

In another embodiment, the 4-Oxadiazolyl-piperidine Compounds of formula (I) are those wherein G = -(C₁-C₅ alkylene)R⁵.

In another embodiment, the 4-Oxadiazolyl-piperidine Compounds of formula (I) are those wherein G = -CH₂-R⁵.

In another embodiment, the 4-Oxadiazolyl-piperidine Compounds of formula (I) are those wherein G = -(CH₂)₂-R⁵.

In another embodiment, the 4-Oxadiazolyl-piperidine Compounds of formula (I) are those wherein G = -(CH₂)₃-R⁵.

In another embodiment, the 4-Oxadiazolyl-piperidine Compounds of formula (I) are those wherein G = -(CH₂)₄-R⁵.

In another embodiment, the 4-Oxadiazolyl-piperidine Compounds of formula (I) are those wherein G = -(CH₂)₅-R⁵.

In another embodiment, the 4-Oxadiazolyl-piperidine Compounds of formula (I) are those wherein G = -(C₁-C₅ alkylene)R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (I) are those wherein G = -CH₂R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (I) are those wherein G = -CH₂C(O)N(CH₃)₂.

In another embodiment, the 4-Oxadiazolyl-piperidine Compounds of formula (I) are those wherein G = -CH₂C(O)NH₂.

In another embodiment, the 4-Oxadiazolyl-piperidine Compounds of formula (I) are those wherein G = -(CH₂)₂-R⁵ and R⁵ = -C(O)NH₂, -C(O)NHOH, -C(O)NH(C₁-C₄ alkyl), or -C(O)N(C₁-C₄ alkyl)₂-

In another embodiment, the 4-Oxadiazolyl-piperidine Compounds of formula (I) are those wherein G = -(CH₂)₃-R⁵ and R⁵ = -C(O)NH₂, -C(O)NHOH, -C(O)NH(C₁-C₄ alkyl), or -C(O)N(C₁-C4 alkyl)₂.

In another embodiment, the 4-Oxadiazolyl-piperidine Compounds of formula (I) are those wherein G = -(CH₂)₄-R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (I) are those wherein G = -(CH₂)₅-R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (I) are those wherein p = 0.

In another embodiment, the 4-Oxadiazolyl-piperidine Compounds of formula (I) are those wherein p = 1.

In another embodiment, the 4-Oxadiazolyl-piperidine Compounds of formula (I) are those wherein p = 1 and the carbon atom to which R³ is attached is in the (R)-configuration.

In another embodiment, the 4-Oxadiazolyl-piperidine Compounds of formula (I) are those wherein p = 1 and the carbon atom to which R³ is attached is in the (S)-configuration.

In another embodiment, the 4-Oxadiazolyl-piperidine Compounds of formula (I) are those wherein p = 1 and R³ is -C₁-C₃ alkyl.

In another embodiment, the 4-Oxadiazolyl-piperidine Compounds of formula (I) are those wherein p = 1 and R³ is -CH₃.

In another embodiment, the 4-Oxadiazolyl-piperidine Compounds of formula (I) are those wherein p = 1, R³ is -C₁-C₃ alkyl, and the carbon atom to which R₃ is attached is in the (R) configuration.

In another embodiment, the 4-Oxadiazolyl-piperidine Compounds of formula (I) are those wherein p = 1, R³ is -CH₃, and the carbon atom to which R³ is attached is in the (R) configuration.

In another embodiment, the 4-Oxadiazolyl-piperidine Compounds of formula (I) are those wherein p = 1, R³ is -C₁-C₃ alkyl, and the carbon atom to which R³ is attached is in the (S) configuration.

In another embodiment, the 4-Oxadiazolyl-piperidine Compounds of formula (I) are those wherein p = 1, and R³ is -CH₃, and the carbon atom to which R³ is attached is in the (S) configuration.

In another embodiment, the 4-Oxadiazolyl-piperidine Compounds of formula (I) are those wherein Ar⁴ is phenyl.

In another embodiment, the 4-Oxadiazolyl-piperidine Compounds of formula (I) are those wherein m = 0.

In another embodiment, the 4-Oxadiazolyl-piperidine Compounds of formula (I) are those wherein m = 1.

In another embodiment, the 4-Oxadiazolyl-piperidine Compounds of formula (I) are those wherein m = 0, and p = 0.

In another embodiment, the 4-Oxadiazolyl-piperidine Compounds of formula (I) are those wherein m = 1 and p = 0.

In another embodiment, the 4-Oxadiazolyl-piperidine Compounds of formula (I) are those wherein m = 0, and q = 0.

In another embodiment, the 4-Oxadiazolyl-piperidine Compounds of formula (I) are those wherein m = 1 and wherein Ar⁴ is phenyl.

In another embodiment, the 4-Oxadiazolyl-piperidine Compounds of formula (I) are those wherein m = 0, p = 0 and wherein Ar⁴ is phenyl.

In another embodiment, the 4-Oxadiazolyl-piperidine Compounds of formula (I) are those wherein m = 1, p = 0 and wherein Ar⁴ is phenyl.

In another embodiment, the 4-Oxadiazolyl-piperidine Compounds of formula (I) are those wherein R² is -Br, -Cl, -I, or -F.

In another embodiment, the 4-Oxadiazolyl-piperidine Compounds of formula (I) are those wherein R² is -O(C₁-C₃ alkyl).

In another embodiment, the 4-Oxadiazolyl-piperidine Compounds of formula (I) are those wherein R² is -C₁-C₃ alkyl.

In another embodiment, the 4-Oxadiazolyl-piperidine Compounds of formula (I) are those wherein R² is -CF₃ or -OCF₃.

In another embodiment, the 4-Oxadiazolyl-piperidine Compounds of formula (I) are those wherein R³ is -CF₃ or -OCF₃.

In another embodiment, the 4-Oxadiazolyl-piperidine Compounds of formula (I) are those wherein R² is -NH(C₁-C₃ alkyl) or -N(C₁-C₃ alkyl)₂.

In another embodiment, the 4-Oxadiazolyl-piperidine Compounds of formula (I) are those wherein R³ is -Br, -C1, -I, or -F.

In another embodiment, the 4-Oxadiazolyl-piperidine Compounds of formula (I) are those wherein R³ is -O(C₁-C₃ alkyl).

In another embodiment, the 4-Oxadiazolyl-piperidine Compounds of formula (I) are those wherein R³ is -C₁-C₃ alkyl.

In another embodiment, the 4-Oxadiazolyl-piperidine Compounds of formula (I) are those wherein R³ is -NH(C₁-C₃ alkyl), or -N(C₁-C₃ alkyl)₂.

In another embodiment, the 4-Oxadiazolyl-piperidine Compounds of formula (I) are those wherein R' is H.

In another embodiment, the 4-Oxadiazolyl-piperidine Compounds of formula (I) are those wherein R¹ is -C(O)NH₂, -C(O)NHOH, -C(O)NH(C₁-C₄ alkyl), or -C(O)N (C₁-C₄ alkyl)₂.

In another embodiment, the 4-Oxadiazolyl-piperidine Compounds of formula (I) are those wherein R' is -C(O)N(CH₃)₂.

In another embodiment, the 4-Oxadiazolyl-piperidine Compounds of formula (I) are those wherein R' is -C(O)N(CH₂CH₃)₂.

In another embodiment, the 4-Oxadiazolyl-piperidine Compounds of formula (I) are those wherein R¹ is -C(O)NHCH₃.

In another embodiment, the 4-Oxadiazolyl-piperidine Compounds of formula (I) are those wherein R' is -C(O)NH(CH₂CH₃)-

In another embodiment, the 4-Oxadiazolyl-piperidine Compounds of formula (I) are those wherein R¹ is -C(O)OR⁴.

In another embodiment, the 4-Oxadiazolyl-piperidine Compounds of formula (I) are those wherein R¹ is -CHO.

In another embodiment, the 4-Oxadiazolyl-piperidine Compounds of formula (I) are those wherein R¹ is -CN.

In another embodiment, the 4-Oxadiazolyl-piperidine Compounds of formula (I) are those wherein R¹ is -(C₁-C₄ alkyl).

In another embodiment, the 4-Oxadiazolyl-piperidine Compounds of formula (I) are those wherein R¹ is -CF₃, -CHF₂, or -CH₂F.

In another embodiment, the 4-Oxadiazolyl-piperidine Compounds of formula (I) are those wherein R¹ is

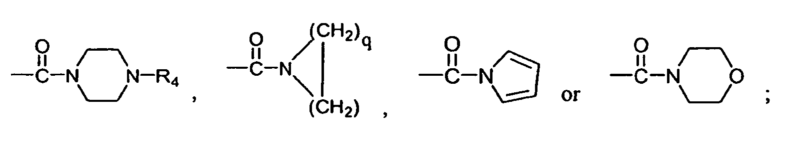

In another embodiment, the 4-Oxadiazolyl-piperidine Compounds of
formula (I) are those wherein
R¹ is

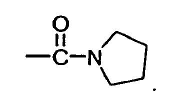

In another embodiment, the 4-Oxadiazolyl-piperidine Compounds of formula (I) are those wherein Ar¹ is -C₃-C₈ cycloalkyl.

In another embodiment, the 4-Oxadiazolyl-piperidine Compounds of formula (I) are those wherein Ar¹ is phenyl, naphthyl, anthryl, or phenanthryl.

In another embodiment, the 4-Oxadiazolyl-piperidine Compounds of formula (I) are those wherein Ar¹ is phenyl.

In another embodiment, the 4-Oxadiazolyl-piperidine Compounds of formula (I) are those wherein Ar¹ is -(5- to 7-membered) heteroaryl.

In another embodiment, the 4-Oxadiazolyl-piperidine Compounds of formula (I) are those wherein Ar¹ is substituted with one or more R² groups.

In another embodiment, the 4-Oxadiazolyl-piperidine Compounds of formula (I) are those wherein Ar² is phenyl, naphthyl, anthryl, or phenanthryl.

In another embodiment, the 4-Oxadiazolyl-piperidine Compounds of formula (I) are those wherein Ar² is phenyl.

In another embodiment, the 4-Oxadiazolyl-piperidine Compounds of formula (I) are those wherein Ar² is -(5- to 7-membered) heteroaryl.

In another embodiment, the 4-Oxadiazolyl-piperidine Compounds of formula (I) are those wherein Ar² is substituted with one or more R² groups.

In another embodiment, the 4-Oxadiazolyl-piperidine Compounds of formula (I) are those wherein Ar¹ and Ar² are phenyl.

In another embodiment, the 4-Oxadiazolyl-piperidine Compounds of formula (I) are those wherein Ar⁴ is substituted with one or more R² groups.

In another embodiment, the 4-Oxadiazolyl-piperidine Compounds of formula (I) are those wherein Ar⁴ is phenyl, naphthyl, anthryl, or phenanthryl.

In another embodiment, the 4-Oxadiazolyl-piperidine Compounds of formula (I) are those wherein Ar⁴ is phenyl.

In another embodiment, the 4-Oxadiazolyl-piperidine Compounds of formula (I) are those wherein Ar⁴ is -(5- to 7-membered) heteroaryl.

In another embodiment, the 4-Oxadiazolyl-piperidine Compounds of formula (I) are those wherein Ar¹, Ar², and Ar⁴ are phenyl.

In another embodiment, the 4-Oxadiazolyl-piperidine Compounds of formula (I) are those wherein Ar¹ is cyclohexyl.

In another embodiment, the 4-Oxadiazolyl-piperidine Compounds of formula (I) are those wherein Ar¹ is cyclohexyl and Ar² is phenyl.

In another embodiment, the 4-Oxadiazolyl-piperidine Compounds of formula (I) are those wherein G = H, and p = 0.

In another embodiment, the 4-Oxadiazolyl-piperidine Compounds of formula (I) are those wherein G = H, and q = 0.

In another embodiment, the 4-Oxadiazolyl-piperidine Compounds of formula (I) are those wherein G = H, and m = 0.

In another embodiment, the 4-Oxadiazolyl-piperidine Compounds of formula (I) are those wherein G = H, and m = 1.

In another embodiment, the 4-Oxadiazolyl-piperidine Compounds of formula (I) are those wherein G = H, and Ar¹ and Ar² are phenyl.

In another embodiment, the 4-Oxadiazolyl-piperidine Compounds of formula (I) are those wherein G = H, p = 0, and Ar⁴ is phenyl.

In another embodiment, the 4-Oxadiazolyl-piperidine Compounds of formula (I) are those wherein G = H, p = 0, and m = 0.

In another embodiment, the 4-Oxadiazolyl-piperidine Compounds of formula (I) are those wherein G = H, p = 0, and m = 1.

In another embodiment, the 4-Oxadiazolyl-piperidine Compounds of formula (I) are those wherein G = H, p = 0, and Ar¹ and Ar² are phenyl.

In another embodiment, the 4-Oxadiazolyl-piperidine Compounds of formula (I) are those wherein G = H, p = 0, Ar⁴ is phenyl, and m = 0.

In another embodiment, the 4-Oxadiazolyl-piperidine Compounds of formula (I) are those wherein G = H, p = 0, Ar⁴ is phenyl, and m = 1.

In another embodiment, the 4-Oxadiazolyl-piperidine Compounds of formula (I) are those wherein G = H, p = 0, Ar⁴ is phenyl, and Ar¹ and Ar² are phenyl.

In another embodiment, the 4-Oxadiazolyl-piperidine Compounds of formula (I) are those wherein G = H, p = 0, Ar⁴ is phenyl, m = 0, and Ar¹ and Ar² are phenyl.

In another embodiment, the 4-Oxadiazolyl-piperidine Compounds of formula (I) are those wherein G = H, p = 0, Ar⁴ is phenyl, m = 1, and Ar¹ and Ar² are phenyl.

Illustrative 4-Oxadiazolyl-piperidine Compounds of formula (I) have the following structure: 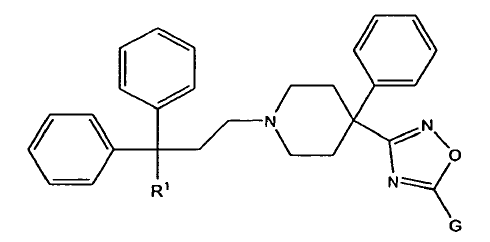 and pharmaceutically acceptable salts thereof,
wherein G and R¹ are as follows:

| **Compound Number:** | **G :** | **R¹:** |
|---|---|---|
| AAA | - H | -H |
| AAB | -CH₂C(O)NH₂ | -H |
| AAC | -CH₂C(O)N(CH₃)₂ | -H |
| AAD | -C(O)CH₂NHSO₂CH₃ | -H |
| AAE | -(CH₂)₂C(O)NH₂ | -H |
| AAF | -(CH₂)₂C(O)N(CH₃)₂ | -H |
| AAG | -C(O)(CH₂)₂NHSO₂CH₃ | -H |
| AAH | -CH₂CO₂CH₃ | -H |
| AAI | -CH₂CO₂CH₂CH₃ | -H |
| AAJ | -(CH₂)₂CO₂CH₃ | -H |
| AAK | -(CH₂)₂CO₂CH₂CH₃ | -H |
| AAL | -(CH₂)₃CO₂CH₃ | -H |
| AAM | -(CH₂)₃CO₂CH₂CH₃ | -H |
| AAN | -(CH₂)₄CO₂CH₃ | -H |
| AAO | -(CH₂)₄CO₂CH₂CH₃ | -H |
| AAP | -CH₂SO₂NH₂ | -H |
| AAQ | -CH₂SO₂N(CH₃)₂ | -H |
| AAR | -(CH₂)₂NHSO₂H | -H |
| AAS | -(CH₂)₂NHSO₂CH₃ | -H |
| AAT | - H | -C(O)NH₂ |
| AAU | -CH₂C(O)NH₂ | -C(O)NH₂ |
| AAV | -CH₂C(O)N(CH₃)₂ | -C(O)NH₂ |
| AAW | -CH₂NHSO₂CH₃ | -C(O)NH₂ |
| AAX | -(CH₂)₂C(O)NH₂ | -C(O)NH₂ |
| AAY | -(CH₂)₂C(O)N(CH₃)₂ | -C(O)NH₂ |
| AAZ | -(CH₂)₂NHSO₂CH₃ | -C(O)NH₂ |
| ABA | -CH₂CO₂CH₃ | -C(O)NH₂ |
| ABB | -CH₂CO₂CH₂CH₃ | -C(O)NH₂ |
| ABC | -(CH₂)₂CO₂CH₃ | -C(O)NH₂ |
| ABD | -(CH₂)₂CO₂CH₂CH₃ | -C(O)NH₂ |
| ABE | -(CH₂)₃CO₂CH₃ | -C(O)NH₂ |
| ABF | -(CH₂)₃CO₂CH₂CH₃ | -C(O)NH₂ |
| ABG | -(CH₂)₄CO₂CH₃ | -C(O)NH₂ |
| ABH | -(CH₂)₄CO₂CH₂CH₃ | -C(O)NH₂ |
| ABI | -CH₂SO₂NH₂ | -C(O)NH₂ |
| ABJ | -CH₂SO₂N(CH₃)₂ | -C(O)NH₂ |
| ABK | -(CH₂)₂NHSO₂H | -C(O)NH₂ |
| ABL | -(CH₂)₂NHSO₂CH₃ | -C(O)NH₂ |
| ABM | - H | -CO₂CH₃ |
| ABN | -CH₂C(O)NH₂ | -CO₂CH₃ |
| ABO | -CH₂C(O)N(CH₃)₂ | -CO₂CH₃ |
| ABP | -C(O)CH₂NHSO₂CH₃ | -CO₂CH₃ |
| ABQ | -(CH₂)₂C(O)NH₂ | -CO₂CH₃ |
| ABR | -(CH₂)₂C(O)N(CH₃)₂ | -CO₂CH₃ |
| ABS | -C(O)(CH₂)₂NHSO₂CH₃ | -CO₂CH₃ |
| ABT | -CH₂CO₂CH₃ | -CO₂CH₃ |
| ABU | -CH₂CO₂CH₂CH₃ | -CO₂CH₃ |
| ABV | -(CH₂)₂CO₂CH₃ | -CO₂CH₃ |
| ABW | -(CH₂)₂CO₂CH₂CH₃ | -CO₂CH₃ |
| ABX | -(CH₂)₃CO₂CH₃ | -CO₂CH₃ |
| ABY | -(CH₂)₃CO₂CH₂CH₃ | -CO₂CH₃ |
| ABZ | -(CH₂)₄CO₂CH₃ | -CO₂CH₃ |
| ACA | -(CH₂)₄CO₂CH₂CH₃ | -CO₂CH₃ |
| ACB | -CH₂SO₂NH₂ | -CO₂CH₃ |
| ACC | -CH₂SO₂N(CH₃)₂ | -CO₂CH₃ |
| ACD | -(CH₂)₂NHSO₂H | -CO₂CH₃ |
| ACE | -(CH₂)₂NHSO₂CH₃ | -CO₂CH₃ |
| ACF | - H | -CHO |
| ACG | -CH₂C(O)NH₂ | -CHO |
| ACH | -CH₂C(O)N(CH₃)₂ | -CHO |
| ACI | -C(O)CH₂NHSO₂CH₃ | -CHO |
| ACJ | -(CH₂)₂C(O)NH₂ | -CHO |
| ACK | -(Ch₂)₂C(O)N(CH₃)₂ | -CHO |
| ACL | -C(O)(CH₂)₂NHSO₂CH₃ | -CHO |
| ACM | -CH₂CO₂CH₃ | -CHO |
| ACN | -CH2CO₂CH₂CH₃ | -CHO |
| ACO | -(CH₂)₂CO₂CH₃ | -CHO |
| ACP | -(CH2)₂CO₂CH₂CH₃ | -CHO |
| ACQ | -(CH₂)₃CO₂CH₃ | -CHO |
| ACR | -(CH₂)₃CO₂CH₂CH₃ | -CHO |
| ACS | -(CH₂)₄CO₂CH₃ | -CHO |
| ACT | -(CH₂)₄CO₂CH₂CH₃ | -CHO |
| ACU | -CH₂SO₂NH₂ | -CHO |
| ACV | -CH₂SO₂N(CH₃)₂ | -CHO |
| ACW | -(CH₂)₂NHSO₂H | -CHO |
| ACX | -(CH₂)₂NHSO₂CH₃ | -CHO |
| ACY | - H | -CN |
| ACZ | -CH₂C(O)NH₂ | -CN |
| ADA | -CH₂C(O)N(CH₃)₂ | -CN |
| ADB | -C(O)CH₂NHSO₂CH₃ | -CN |
| ADC | -(CH₂)₂C(O)NH₂ | -CN |
| ADD | -(CH₂)₂C(O)N(CH₃)₂ | -CN |
| ADE | -C(O)(CH_{Z})_{Z}NHSO₂CH₃ | -CN |
| ADF | -CH₂CO₂CH₃ | -CN |
| ADG | -CH₂CO₂CH₂CH₃ | -CN |
| ADH | -(CH₂)₂CO₂CH₃ | -CN |
| ADI | -(CH₂)₂CO₂CH₂CH₃ | -CN |
| ADJ | -(CH₂)₃CO₂CH₃ | -CN |
| ADK | -(CH₂)₃CO₂CH₂CH₃ | -CN |
| ADL | -(CH₂)₄CO₂CH₃ | -CN |
| ADM | -(CH₂)₄CO₂CH₂CH₃ | -CN |
| AND | -CH₂SO₂NH₂ | -CN |
| ADO | -CH₂SO₂N(CH₃)₂ | -CN |
| ADP | -(CH₂)₂NHSO₂H | -CN |
| ADQ | -(CH₂)₂NHSO₂CH₃ | -CN |
| ADR | - H | -CH₃ |
| ADS | -CH₂C(O)NH₂ | -CH₃ |
| ADT | -CH₂C(O)N(CH₃)₂ | -CH₃ |
| ADU | -C(O)CH₂NHSO₂CH₃ | -CH₃ |
| ADV | -(CH₂)₂C(O)NH₂ | -CH₃ |
| ADW | -(CH₂)₂C(O)N(CH₃)₂ | -CH₃ |
| ADX | -C(O)(CH₂)₂NHSO₂CH₃ | -CH₃ |
| ADY | -CH₂CO₂CH₃ | -CH₃ |
| ADZ | -CH₂CO₂CH₂CH₃ | -CH₃ |
| AEA | -(CH₂)₂CO₂CH₃ | -CH₃ |
| AEB | -(CH₂)₂CO₂CH₂CH₃ | -CH₃ |
| AEC | -(CH₂)₃CO₂CH₃ | -CH₃ |
| AED | -(CH₂)₃CO₂CH₂CH₃ | -CH₃ |
| AEE | -(CH₂)₄CO₂CH₃ | -CH₃ |
| AEF | -(CH₂)₄CO₂CH₂CH₃ | -CH₃ |
| AEG | -CH₂SO₂NH₂ | -CH₃ |
| AEH | -CH₂SO₂N(CH₃)ₙ | -CH₃ |
| AEI | -(CH₂)₂NHSO₂H | -CH₃ |
| AEJ | -(CH₂)₂NHSO₂CH₃ | -CH₃ |
| AEK | - H | -C(O)NH(CH₃) |
| AEL | -CH₂C(O)NH₂ | -C(O)NH(CH₃) |
| AEM | -CH₂C(O)N(CH₃)₂ | -C(O)NH(CH₃) |
| AEN | -C(O)CH₂NHSO₂CH₃ | -C(O)NH(CH₃) |
| AEO | -(CH₂)₂C(O)NH₂ | -C(O)NH(CH₃) |
| AEP | -(CH₂)₂C(O)N(CH₃)₂ | -C(O)NH(CH₃) |
| AEQ | -C(O)(CH₂)₂NHSO₂CH₃ | -C(O)NH(CH3₎ |
| AER | -CH₂CO₂CH₃ | -C(O)NH(CH₃) |
| AES | -CH₂CO₂CH₂CH₃ | -C(O)NH(CH₃) |
| AET | -(CH₂)₂CO₂CH₃ | -C(O)NH(CH₃) |
| AEU | -(CH₂)₂CO₂CH₂CH₃ | -C(O)NH(CH₃) |
| AEV | -(CH₂)₃CO₂CH₃ | -C(O)NH(CH₃) |
| AEW | -(CH₂)₃CO₂CH₂CH₃ | -C(O)NH(CH₃) |
| AEX | -(CH₂)₄CO₂CH₃ | -C(O)NH(CH₃) |
| AEY | -(CH₂)₄CO₂CH₂CH₃ | -C(O)NH(CH₃) |
| AEZ | -CH₂SO₂NH₂ | -C(O)NH(CH₃) |
| AFA | -CH₂SO₂N(CH₃)₂ | -C(O)NH(CH₃) |
| AFB | -(CH₂)₂NHSO₂H | -C(O)NH(CH₃) |
| AFC | -(CH₂)₂NHSO₂CH₃ | -C(O)NH(CH₃) |
| AFD | - H | -C(O)N(CH₃)₂ |
| AFE | -CH₂C(O)NH₂ | -C(O)N(CH₃)₂ |
| AFF | -CH₂C(O)N(CH₃)₂ | -C(O)N(CH₃)₂ |
| AFG | -C(O)CH₂NHSO₂CH₃ | -C(O)N(CH₃)₂ |
| AFH | -(CH₂)₂C(O)NH₂ | -C(O)N(CH₃)₂ |
| AFI | -(CH₂)₂C(O)N(CH₃)₂ | -C(O)N(CH₃)₂ |
| AFJ | -C(O)(CH₂)₂NHSO₂CH₃ | -C(O)N(CH₃)₂ |
| AFK | -CH₂CO₂CH₃ | -C(O)N(CH₃)₂ |
| AFL | -CH₂CO₂CH₂CH₃ | -C(O)N(CH₃)₂ |
| AFM | -(CH₂)₂CO₂CH₃ | -C(O)N(CH₃)₂ |
| AFN | -(CH₂)₂CO₂CH₂CH₃ | -C(O)N(CH₃)₂ |
| AFO | -(CH₂)₃CO₂CH₃ | -C(O)N(CH₃)₂ |
| AFP | -(CH₂)₃CO₂CH₂CH₃ | -C(O)N(CH₃)₂ |
| AFQ | -(CH₂)₄CO₂CH₃ | -C(O)N(CH₃)₂ |
| AFR | -(CH₂)₄CO₂CH₂CH₃ | -C(O)N(CH₃)₂ |
| AFS | -CH₂SO₂NH₂ | -C(O)N(CH₃)₂ |
| AFT | -CH₂SO₂N(CH₃)₂ | -C(O)N(CH₃)₂ |
| AFU | -(CH₂)₂NHSO₂H | -C(O)N(CH₃)₂ |
| AFV | -(CH₂)₂NHSO₂CH₃ | -C(O)N(CH₃)₂ |
| AFW | - H | 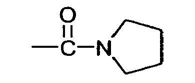 |
| AFX | -CH₂C(O)NH₂ | 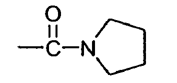 |
| AFY | -CH₂C(O)N(CH₃)₂ | 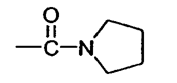 |
| AFZ | -C(O)CH₂NHSO₂CH₃ | 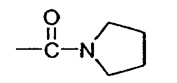 |
| AGA | -(CH₂)₂C(O)NH₂ | 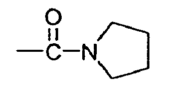 |
| AGB | -(CH₂)₂C(O)N(CH₃)₂ | 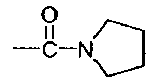 |
| AGC | -C(O)(CH₂)₂NHSO₂CH₃ | 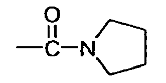 |
| AGD | -CH₂CO₂CH₃ | 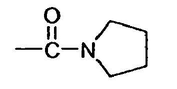 |
| AGE | -CH₂CO₂CH₂CH₃ | 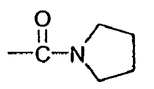 |
| AGF | -(CH₂)₂CO₂CH₃ | 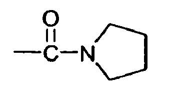 |
| AGG | -(CH₂)₂CO₂CH₂CH₃ | 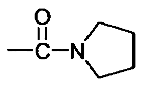 |
| AGH | -(CH₂)₃CO₂CH₃ | 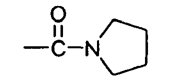 |
| AGI | -(CH₂)₃CO₂CH₂CH₃ | 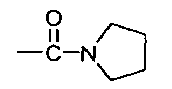 |
| AGJ | -(CH₂)₄CO₂CH₃ | 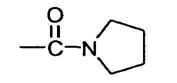 |
| AGK | -(CH₂)₄CO₂CH₂CH₃ | 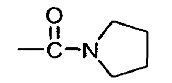 |
| AGL | -CH₂SO₂NH₂ | 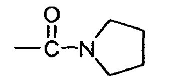 |
| AGM | -CH₂SO₂N(CH₃)₂ | 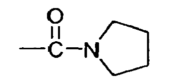 |
| AGN | -(CH₂)₂NHSO₂H | 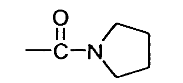 |
| AGO | -(CH₂)₂NHSO₂CH₃ | 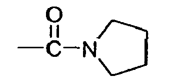 |
| AGP | -H | 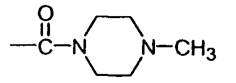 |
| AGQ | -CH₂C(O)NH₂ | 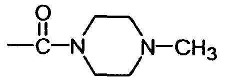 |
| AGR | -CH₂C(O)N(CH₃)₂ | 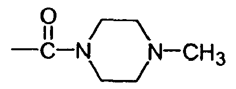 |
| AGS | -C(O)CH₂NHSO₂CH₃ | 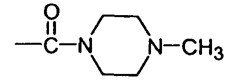 |
| AGT | -(CH₂)₂C(O)NH₂ | 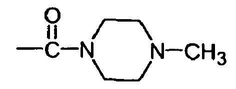 |
| AGU | -(CH₂)₂C(O)N(CH₃)₂ | 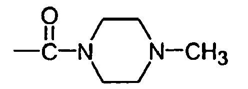 |
| AGV | -C(O)(CH₂)₂NHSO₂CH₃ | 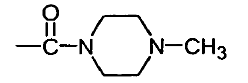 |
| AGW | -CH₂CO₂CH₃ | 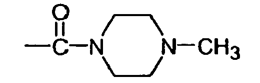 |
| AGX | -CH₂CO₂CH₂CH₃ | 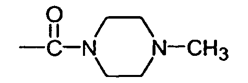 |
| AGY | -(CH₂)₂CO₂CH₃ | 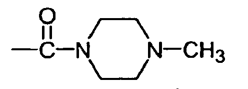 |
| AGZ | -(CH₂)₂CO₂CH₂CH₃ | 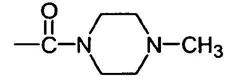 |
| AHA | -(CH₂)₃CO₂CH₃ | 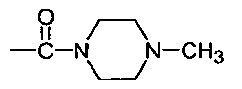 |
| AHB | -(CH₂)₃CO₂CH₂CH₃ | 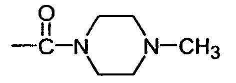 |
| AHC | -(CH₂)₄CO₂CH₃ | 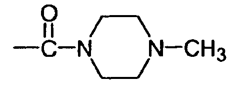 |
| AHD | -(CH₂)₄CO₂CH₂CH₃ | 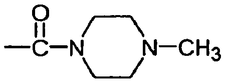 |
| AHE | -CH₂SO₂NH₂ | 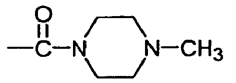 |
| AHF | -CH₂SO₂N(CH₃)₂ | 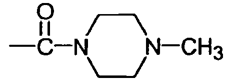 |
| AHG | -(CH₂)₂NHSO₂H | 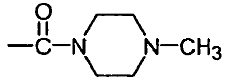 |
| AHH | -(CH₂)₂NHSO₂CH₃ | 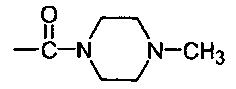 |
| AHl | -H | 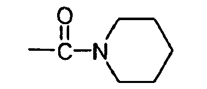 |
| AHJ | -CH₂C(O)NH₂ | 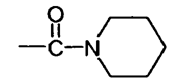 |
| AHK | -CH₂C(O)N(CH₃)₂ | 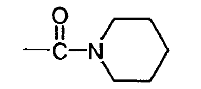 |
| AHL | -C(O)CH₂NHSO₂CH₃ | 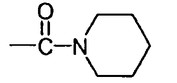 |
| AHM | -(CH₂)₂C(O)NH₂ | 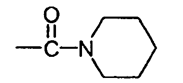 |
| AHN | -(CH₂)₂C(O)N(CH₃)₂ | 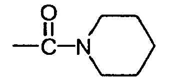 |
| AHO | -C(O)(CH₂)₂NHSO₂CH₃ | 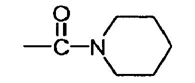 |
| AHP | -CH₂CO₂CH₃ | 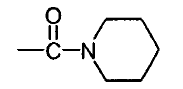 |
| AHQ | -CH₂CO₂CH₂CH₃ | 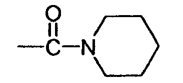 |
| AHR | -(CH₂)₂CO₂CH₃ | 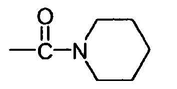 |
| AHS | -(CH₂)₂CO₂CH₂CH₃ | 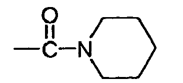 |
| AHT | -(CH₂)₃CO₂CH₃ | 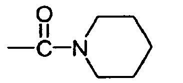 |
| AHU | -(CH₂)₃CO₂CH₂CH₃ | 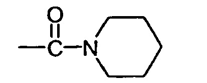 |
| AHV | -(CH₂)₄CO₂CH₃ | 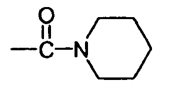 |
| AHW | -(CH₂)₄CO₂CH₂CH₃ | 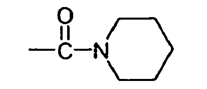 |
| AHX | -CH₂SO₂NH₂ | 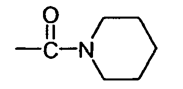 |
| AHY | -CH₂SO₂N(CH₃)₂ | 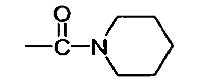 |
| AHZ | -(CH₂)₂NHSO₂H | 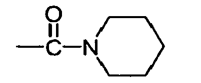 |
| AIA | -(CH₂)₂NHSO₂CH₃ | 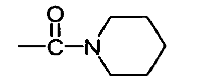 |
| AIB | -H | 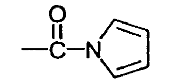 |
| AIC | -CH₂C(O)NH₂ | 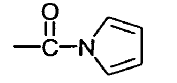 |
| AID | -CH₂C(O)N(CH₃)₂ | 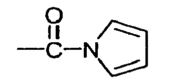 |
| AIE | -C(O)CH₂NHSO₂CH₃ | 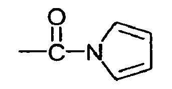 |
| AIF | -(CH₂)₂C(O)NH₂ | 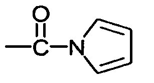 |
| AIG | -(CH₂)₂C(O)N(CH₃)₂ | 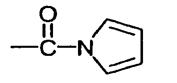 |
| AIH | -C(O)(CH₂)₂NHSO₂CH₃ | 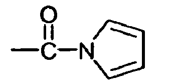 |
| All | -CH₂CO₂CH₃ | 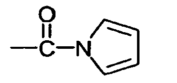 |
| AIJ | -CH₂CO₂CH₂CH₃ | 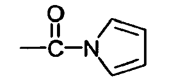 |
| AIK | -(CH₂)₂COCH₃ | 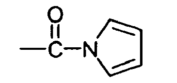 |
| AIL | -(CH₂)₂CO₂CH₂CH₃ | 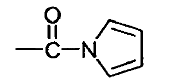 |
| AIM | -(CH₂)₃CO₂CH₃ | 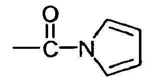 |
| AIN | -(CH₂)₃CO₂CH₂CH₃ | 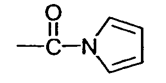 |
| AIO | -(CH₂)₄CO₂CH₃ | 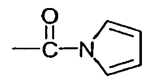 |
| AIP | -(CH₂)₄CO₂CH₂CH₃ | 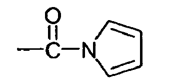 |
| AIQ | -CH₂SO₂NH₂ | 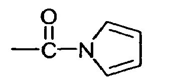 |
| AIR | -CH₂SO₂N(CH₃)₂ | 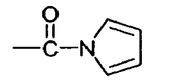 |
| AIS | -(CH₂)₂NHSO₂H | 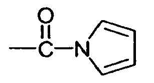 |
| AIT | -(CH₂)₂NHSO₂CH₃ | 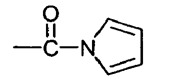 |
| AIU | -H | 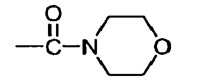 |
| AIV | -CH₂C(O)NH₂ | 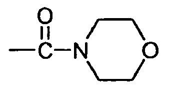 |
| AIW | -CH₂C(O)N(CH₃)₂ | 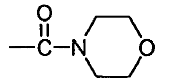 |
| AIX | -C(O)CH₂NHSO₂CH₃ | 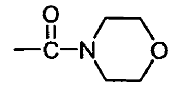 |
| AIY | -(CH₂)₂C(O)NH₂ | 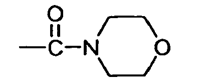 |
| AIZ | -(CH₂)₂C(O)N(CH₃)₂ | 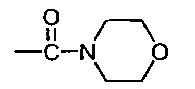 |
| AJA | -C(O)(CH₂)₂NHSO₂CH₃ | 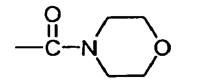 |
| AJB | -CH₂CO₂CH₃ | 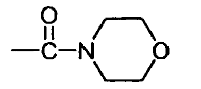 |
| AJC | -CH₂CO₂CH₂CH₃ | 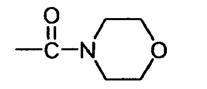 |
| AJD | -(CH₂)₂CO₂CH₃ | 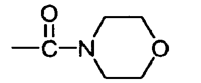 |
| AJE | -(CH₂)₂CO₂CH₂CH₃ | 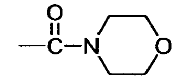 |
| AJF | -(CH₂)₃CO₂CH₃ | 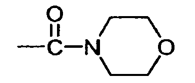 |
| AJG | -(CH₂)₃CO₂CH₂CH₃ | 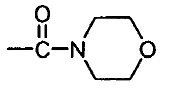 |
| AJH | -(CH₂)₄CO₂CH₃ | 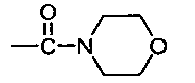 |
| AJI | -(CH₂)₄CO₂CH₂CH₃ | 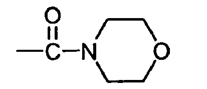 |
| AJJ | -CH₂SO₂NH₂ | 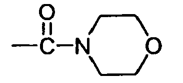 |
| AJK | -CH₂SO₂N(CH₃)₂ | 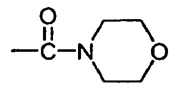 |
| AJL | -(CH₂)₂NHSO₂H | 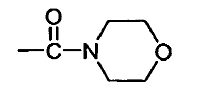 |
| AJM | -(CH₂)₂NHSO₂CH₃ | 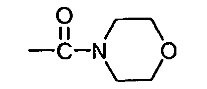 |
| AJN | -(CH₂)₃NHSO₂H | -H |
| AJO | -(CH₂)₃NHSO₂H | -C(O)NH₂ |
| AJP | -(CH₂)₃NHSO₂H | -CO₂CH₃ |
| AJQ | -(CH₂)₃NHSO₂H | -CHO |
| AJR | -(CH₂)₃NHSO₂H | -CN |
| AJS | -(CH₂)₃NHSO₂H | -CH₃ |
| AJT | -(CH₂)₃NHSO₂H | -C(O)NHCH₃ |
| AJU | -(CH₂)₃NHSO₂H | -C(O)N(CH₃)₂ |
| AJV | -(CH₂)₃NHSO₂H | 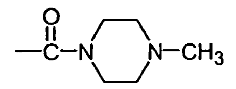 |
| AJW | -(CH₂)₃NHSO₂H | 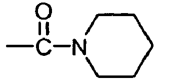 |
| AJX | -(CH₂)₃NHSO₂H | 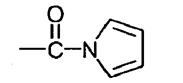 |
| AJY | -(CH₂)₃NHSO₂H | 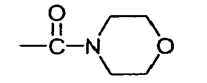 |

The present invention further encompasses compounds having the formula (II): 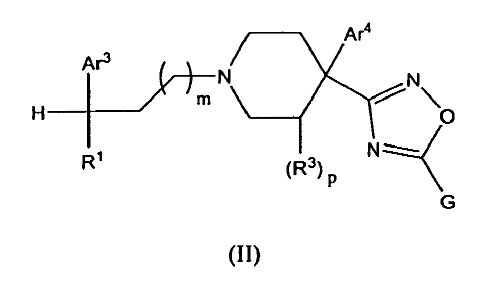 and pharmaceutically acceptable salts thereof, wherein Ar³, Ar⁴, R¹-R³, G, n, m, p and q are as defined above.

In one embodiment, the 4-Oxadiazolyl-piperidine Compounds of formula (II) are those wherein G is H.

In one embodiment, the 4-Oxadiazolyl-piperidine Compounds of formula (II) are those wherein G = -C(O)(CH₂)ₙC(O)OR ⁴

In one embodiment, the 4-Oxadiazolyl-piperidine Compounds of formula (II) are those wherein G = -(C₁-C₅ alkylene)R⁵.

In one embodiment, the 4-Oxadiazolyl-piperidine Compounds of formula (II) are those wherein G = -CH₂-R⁵.

In one embodiment, the 4-Oxadiazolyl-piperidine Compounds of formula (II) are those wherein G = -(CH₂)₂-R⁵.

In one embodiment, the 4-Oxadiazolyl-piperidine Compounds of formula (II) are those wherein G = -(CH₂)₃-R⁵.

In one embodiment, the 4-Oxadiazolyl-piperidine Compounds of formula (II) are those wherein G = -(CH₂)₄-R⁵

In one embodiment, the 4-Oxadiazolyl-piperidine Compounds of formula (II) are those wherein G = -(CH₂)₅-R⁵.

In one embodiment, the 4-Oxadiazolyl-piperidine Compounds of formula (II) are those wherein G = -C(O)(CH₂)ₙC(O)OR" and R⁴ = H.

In one embodiment, the 4-Oxadiazolyl-piperidine Compounds of formula (II) are those wherein G = -C(O)(CH₂)ₙC(O)OR⁴ and R⁴ = -C₁-C₁₀ alkyl.

In one embodiment, the 4-Oxadiazolyl-piperidine Compounds of formula (II) are those wherein G = -C(O)(CH₂)nC(O)OR⁴andR⁴ = -CH₂O(C₁-C₄ alkyl).

In one embodiment, the 4-Oxadiazolyl-piperidine Compounds of formula (II) are those wherein G = -C(O)(CH₂)ₙC(O)OR⁴ and R⁴ = -CH₂NH(C₁-C₄ alkyl) or -CH₂N(C₁-C₄ alkyl)_{2.}

In one embodiment, the 4-Oxadiazolyl-piperidine Compounds of formula (II) are those wherein G = -(C₁-C₅ alkylene)COOR⁴.

In one embodiment, the 4-Oxadiazolyl-piperidine Compounds of formula (II) are those wherein G = -CH₂-COOR⁴.

In one embodiment, the 4-Oxadiazolyl-piperidine Compounds of formula (II) are those wherein G = -CH₂COOCH₂CH₃.

In one embodiment, the 4-Oxadiazolyl-piperidine Compounds of formula (II) are those wherein G = -(CH₂)₂-COOR⁴.

In one embodiment, the 4-Oxadiazolyl-piperidine Compounds of formula (II) are those wherein G = -(CH₂)₃-COOR⁴.

In one embodiment, the 4-Oxadiazolyl-piperidine Compounds of formula (II) are those wherein G = -(CH₂)₄-COOR⁴.

In one embodiment, the 4-Oxadiazolyl-piperidine Compounds of formula (II) are those wherein G = -(CH₂)₅-COOR⁴.

In one embodiment, the 4-Oxadiazolyl-piperidine Compounds of formula (II) are those wherein G = -C(O)(CH₂)ₙR⁵ and R⁵ = -NHSO₂R⁴.

In one embodiment, the 4-Oxadiazolyl-piperidine Compounds of formula (II) are those wherein G = -C(O)(CH₂)ₙR⁵ and R⁵ = -C(O)NH₂, -C(O)NHOH, -C(O)NH(C1-C₄ alkyl), or -C(O)N(C₁-C₄ alkyl)₂.

In one embodiment, the 4-Oxadiazolyl-piperidine Compounds of formula (II) are those wherein G = -C(O)(CH₂)ₙR⁵ and R⁵ = -SO₂NH₂ -SO₂NH(C₁-C₄ alkyl), or -SO₂N(C₁-C₄ alkyl)₂.

In one embodiment, the 4-Oxadiazolyl-piperidine Compounds of formula (II) are those wherein G = -C(O)(CH₂)ₙR₅ and R⁵ = -NHSO₂H.

In one embodiment, the 4-Oxadiazolyl-piperidine Compounds of formula (II) are those wherein G = -C(O)(CH₂)ₙR⁵.

In one embodiment, the 4-Oxadiazolyl-piperidine Compounds of formula (II) are those wherein G = (C₁-C₅ alkylene)R⁵.

In another embodiment, the 4-Oxadiazolyl-piperidine Compounds of formula (II) are those wherein G = -(C₁-C₅ alkylene)R⁵ and R⁵ = -C(O)NH₂, -C(O)NHOH, -C(O)NH(C₁-C₄ alkyl), or -C(O)NH(C₁-C₄alkyl)₂.

In another embodiment, the 4-Oxadiazolyl-piperidine Compounds of formula (II) are those wherein G = -CH₂R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (II) are those wherein G = -CH₂C(O)N(CH₃)₂.

In another embodiment, the 4-Oxadiazolyl-piperidine Compounds of formula (I) are those wherein G = -CH₂C(O)NH₂.

In another embodiment, the 4-Oxadiazolyl-piperidine Compounds of formula (II) are those wherein G = -(CH₂)₂-R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (II) are those wherein G = -(CH₂)₃-R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (II) are those wherein G = -(CH₂)₄-R⁵ and R⁵ = -C(O)NH₂, -C(O)NHOH, -C(O)NH(C₁-C₄ alkyl), or -C(O)N(C₁-C₄ alkyl)₂.

In another embodiment, the 4-Oxadiazolyl-piperidine Compounds of formula (II) are those wherein G = -(CH₂)₅-R⁵ and R⁵ = -C(O)NH₂, -C(O)NHOH, -C(O)NH(C₁-C₄ alkyl), or -C(O)N(C₁-C₄ alkyl)₂.

In one embodiment, the 4-Oxadiazolyl-piperidine Compounds of formula (II) are those wherein p = 0.

In another embodiment, the 4-Oxadiazolyl-piperidine Compounds of formula (II) are those wherein p = 1.

In another embodiment, the 4-Oxadiazolyl-piperidine Compounds of formula (II) are those wherein p = 1 and the carbon atom to which R³ is attached is in the (R)-configuration.

In another embodiment, the 4-Oxadiazolyl-piperidine Compounds of formula (II) are those wherein p = 1 and the carbon atom to which R³ is attached is in the (S)-configuration.

In another embodiment, the 4-Oxadiazolyl-piperidine Compounds of formula (II) are those wherein p = 1 and R³ is -C₁-C₃ alkyl.

In another embodiment, the 4-Oxadiazolyl-piperidine Compounds of formula (II) are those wherein p = 1 and R³ is -CH₃.

In another embodiment, the 4-Oxadiazolyl-piperidine Compounds of formula (II) are those wherein p = 1, R³ is -C₁-C₃ alkyl, and the carbon atom to which R³ is attached is in the (R) configuration.

In another embodiment, the 4-Oxadiazolyl-piperidine Compounds of formula (II) are those wherein p = 1, R³ is -CH₃, and the carbon atom to which R³ is attached is in the (R) configuration.

In another embodiment, the 4-Oxadiazolyl-piperidine Compounds of formula (II) are those wherein p = 1, R³ is -C₁-C₃ alkyl, and the carbon atom to which R³ is attached is in the (S) configuration.

In another embodiment, the 4-Oxadiazolyl-piperidine Compounds of formula (II) are those wherein p = 1, R³ is -CH₃, and the carbon atom to which R₃ is attached is in the (S) configuration.

In one embodiment, the 4-Oxadiazolyl-piperidine Compounds of formula (II) are those wherein Ar⁴ is phenyl.

In one embodiment, the 4-Oxadiazolyl-piperidine Compounds of formula (II) are those wherein m = 0.

In one embodiment, the 4-Oxadiazolyl-piperidine Compounds of formula (II) are those wherein m = 1.

In one embodiment, the 4-Oxadiazolyl-piperidine Compounds of formula (II) are those wherein m = 0, and p = 0.

In one embodiment, the 4-Oxadiazolyl-piperidine Compounds of formula (II) are those wherein m = 1 and p = 0.

In one embodiment, the 4-Oxadiazolyl-piperidine Compounds of formula (II) are those wherein m = 0, and Ar⁴ is phenyl.

In one embodiment, the 4-Oxadiazolyl-piperidine Compounds of formula (II) are those wherein m = 1 and Ar⁴ is phenyl.

In one embodiment, the 4-Oxadiazolyl-pipefidine Compounds of formula (II) are those wherein m = 0, p = 0 and Ar⁴ is phenyl.

In one embodiment, the 4-Oxadiazolyl-piperidine Compounds of formula (II) are those wherein m = 1, p = 0 and Ar⁴ is phenyl.

In one embodiment, the 4-Oxadiazolyl-piperidine Compounds of formula (II) are those wherein R² is -Br, -CI, -I, or -F.

In one embodiment, the 4-Oxadiazolyl-pipefidine Compounds of formula (II) are those wherein R² is -O(C₁-C₃ alkyl).

In one embodiment, the 4-Oxadiazolyl-piperidine Compounds of formula (II) are those wherein R² is -C₁-C₃ alkyl.

In another embodiment, the 4-Oxadiazolyl-piperidine Compounds of formula (I) are those wherein R² is -CF₃ or -OCF₃.

In another embodiment, the 4-Oxadiazolyl-piperidine Compounds of formula (1) are those wherein R³ is -CF₃ or -OCF₃.

In one embodiment, the 4-Oxadiazolyl-piperidine Compounds of formula (II) are those wherein R³ is -NH(C₁-C₃ alkyl) or -N(C₁-C₃ alkyl)₂.

In one embodiment, the 4-Oxadiazolyl-piperidine Compounds of formula (II) are those wherein R³ is -Br, -Cl, -I, or -F.

In one embodiment, the 4-Oxadiazolyl-piperidine Compounds of formula (II) are those wherein R³ is -O(C₁-C₃ alkyl).

In one embodiment, the 4-Oxadiazolyl-piperidine Compounds of formula (II) are those wherein R³ is -C₁-C₃ alkyl.

In one embodiment, the 4-Oxadiazolyl-piperidine Compounds of formula (II) are those wherein R³ is -NH(C₁-C₃ alkyl) or -N(C₁-C₃ alkyl)₂.

In one embodiment, the 4-Oxadiazolyl-piperidine Compounds of formula (II) are those wherein R¹ is H.

In one embodiment, the 4-Oxadiazolyl-piperidine Compounds of formula (II) are those wherein R¹ is -C(O)NH₂, -C(O)NHOH, -C(O)NH(C₁-C₄ alkyl), or -C(O)N(C₁-C₄ alkyl)₂.

In one embodiment, the 4-Oxadiazolyl-piperidine Compounds of formula (II) are those wherein R¹ is -C(O)N(CH₃)₂.

In one embodiment, the 4-Oxadiazolyi-piperidine Compounds of formula (II) are those wherein R¹ is -C(O)N(CH₂CH₃)₂.

In one embodiment, the 4-Oxadiazolyl-piperidine Compounds of formula (II) are those wherein R¹ is -C(O)NHCH₃.

In one embodiment, the 4-Oxadiazolyl-piperidine Compounds of formula (II) are those wherein R¹ is -C(O)NHCH₂CH₃-

In one embodiment, the 4-Oxadiazolyl-piperidine Compounds of formula (II) are those wherein R¹ is -COOR₄.

In one embodiment, the 4-Oxadiazolyl-piperidine Compounds of formula (II) are those wherein R¹ is -CHO.

In one embodiment, the 4-Oxadiazolyl-piperidine Compounds of formula (II) are those wherein R¹ is -CN.

In another embodiment, the 4-Oxadiazolyl-piperidine Compounds of formula (I) are those wherein R¹ is -CF₃, -CHF₂, or -CH₂F.

In one embodiment, the 4-Oxadiazolyl-piperidine Compounds of formula (II) are those wherein R¹ is -(C₁-C₄ alkyl).

In one embodiment, the 4-Oxadiazolyl-piperidine Compounds of formula (II) are those wherein R¹ is

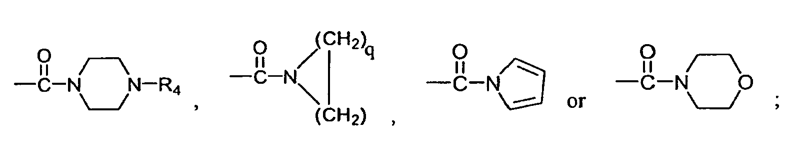

In another embodiment, the 4-Oxadiazolyl-piperidine Compounds of
formula (II) are those wherein
R¹ its

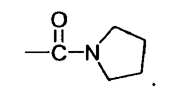

In one embodiment, the 4-Oxadiazolyl-piperidine Compounds of formula (II) are those wherein Ar³ is phenyl, naphthyl, anthryl, or phenanthryl.

In one embodiment, the 4-Oxadiazolyl-piperidine Compounds of formula (II) are those wherein Ar³ is phenyl.

In one embodiment, the 4-Oxadiazolyl-piperidine Compounds of formula (II) are those wherein Ar³ is -(5- to 7-membered) heteroaryl.

In one embodiment, the 4-Oxadiazolyl-piperidine Compounds of formula (II) are those wherein Ar³ is substituted with one, two, or three R² groups.

In one embodiment, the 4-Oxadiazolyl-piperidine Compounds of formula (II) are those wherein Ar⁴ is phenyl, naphthyl, anthryl, or phenanthryl.

In one embodiment, the 4-Oxadiazolyl-piperidine Compounds of formula (II) are those wherein Ar⁴ is phenyl.

In one embodiment, the 4-Oxadiazolyl-piperidine Compounds of formula (II) are those wherein Ar⁴ is -(5- to 7-membered) heteroaryl.

In one embodiment, the 4-Oxadiazolyl-piperidine Compounds of formula (II) are those wherein Ar⁴ is substituted with one, two, or three R² groups.

In one embodiment, the 4-Oxadiazolyl-piperidine Compounds of formula (II) are those wherein G = H, and p = 0.

In one embodiment, the 4-Oxadiazolyl-piperidine Compounds of formula (II) are those wherein G = H, and Ar⁴ is phenyl.

In one embodiment, the 4-Oxadiazolyl-piperidine Compounds of formula (II) are those wherein G = H, and m = 0.

In one embodiment, the 4-Oxadiazolyl-piperidine Compounds of formula (II) are those wherein G = H, and m = 1.

In one embodiment, the 4-Oxadiazolyl-piperidine Compounds of formula (II) are those wherein G = H, and Ar³ is phenyl.

In one embodiment, the 4-Oxadiazolyl-piperidine Compounds of formula (II) are those wherein G = H, p = 0, and Ar⁴ is phenyl.

In one embodiment, the 4-Oxadiazolyl-piperidine Compounds of formula (II) are those wherein G = H, p = 0, and m = 0.

In one embodiment, the 4-Oxadiazolyl-piperidine Compounds of formula (II) are those wherein G = H, p = 0, and m = 1.

In one embodiment, the 4-Oxadiazolyl-piperidine Compounds of formula (II) are those wherein G = H, p = 0, and Ar³ is phenyl.

In one embodiment, the 4-Oxadiazolyl-piperidine Compounds of formula (II) are those wherein G = H, p = 0, Ar⁴ is phenyl, and m = 0.

In one embodiment, the 4-Oxadiazolyl-piperidine Compounds of formula (II) are those wherein G = H, p = 0, Ar⁴ is phenyl, and m = 1.

In one embodiment, the 4-Oxadiazolyl-piperidine Compounds of formula (II) are those wherein G = H, p = 0, Ar⁴ is phenyl, and Ar³ is phenyl.

In one embodiment, the 4-Oxadiazolyl-piperidine Compounds of formula (II) are those wherein G = H, p = 0, Ar⁴ is phenyl, m = 0, and Ar₃ is phenyl.

Illustrative 4-Oxadiazolyl-piperidine Compounds of formula (II) have the following structure: 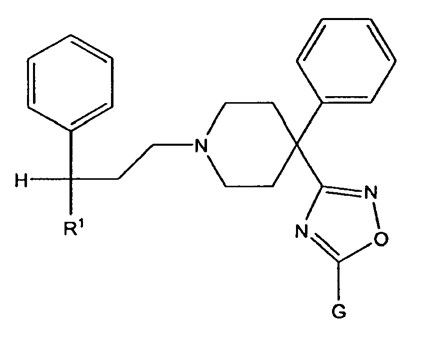 wherein G and R¹ are as follows:

| **Compound Number:** | **G :** | **R¹:** |
|---|---|---|
| BAA | - H | -H |
| BAB | -CH₂C(O)NH₂ | -H |
| BAC | -CH₂C(O)N(CH₃)₂ | -H |
| BAD | -C(O)CH₂NHSO₂CH₃ | -H |
| BAE | -(CH₂)₂C(O)NH₂ | -H |
| BAF | -(CH₂)₂C(O)N(CH₃)₂ | -H |
| BAG | -C(O)(CH₂)₂NHSO₂CH₃ | -H |
| BAH | -CH₂CO₂CH₃ | -H |
| BAI | -CH₂CO₂CH₂CH₃ | -H |
| BAJ | -(CH₂)₂CO₂CH₃ | -H |
| BAK | -(CH₂)₂CO₂CH₂CH₃ | -H |
| BAL | -(CH₂)₃CO₂CH₃ | -H |
| BAM | -(CH₂)₃CO₂CH₂CH₃ | -H |
| BAN | -(CH₂)₄CO₂CH₃ | -H |
| BAO | -(CH₂)₄CO₂CH₂CH₃ | -H |
| BAP | -CH₂SO₂NH₂ | -H |
| BAQ | -CH₂SO₂N(CH₃)₂ | -H |
| BAR | -(CH₂)₂NHSO₂H | -H |
| BAS | -(CH₂)₂NHSO₂CH₃ | -H |
| BAT | - H | -C(O)NH₂ |
| BAU | -CH₂C(O)NH₂ | -C(O)NH₂ |
| BAV | -CH₂C(O)N(CH₃)₂ | -C(O)NH₂ |
| BAW | -CH₂NHSO₂CH₃ | -C(O)NH₂ |
| BAX | -(CH₂)₂C(O)NH₂ | -C(O)NH₂ |
| BAY | -(CH₂)₂C(O)N(CH₃)₂ | -C(O)NH₂ |
| BAZ | -(CH₂)₂NHSO₂CH₃ | -C(O)NH₂ |
| BBA | -CH₂CO₂CH₃ | -C(O)NH₂ |
| BBB | -CH₂CO₂CH₂CH₃ | -C(O)NH₂ |
| BBC | -(CH₂)₂CO₂CH₃ | -C(O)NH₂ |
| BBD | -(CH₂)₂CO₂CH₂CH₃ | -C(O)NH₂ |
| BBE | -(CH₂)₃CO₂CH₃ | -C(O)NH₂ |
| BBF | -(CH₂)₃CO₂CH₂CH₃ | -C(O)NH₂ |
| BBG | -(CH₂)₄CO₂CH₃ | -C(O)NH₂ |
| BBH | -(CH₂)₄CO₂CH₂CH₃ | -C(O)NH₂ |
| BBI | -CH₂SO₂NH₂ | -C(O)NH₂ |
| BBJ | -CH₂SO₂N(CH₃)₂ | -C(O)NH₂ |
| BBK | -(CH₂)₂NHSO₂H | -C(O)NH₂ |
| BBL | -(CH₂)₂NHSO₂CH₃ | -C(O)NH₂ |
| BBM | - H | -CO₂CH₃ |
| BBN | -CH₂C(O)NH₂ | -CO₂CH₃ |
| BBO | -CH₂C(O)N(CH₃)₂ | -CO₂CH₃ |
| BBP | -C(O)CH₂NHSO₂CH₃ | -CO₂CH₃ |
| BBQ | -(CH₂)₂C(O)NH₂ | -CO₂CH₃ |
| BBR | -(CH₂)₂C(O)N(CH₃)₂ | -CO₂CH₃ |
| BBS | -C(O)(CH₂)₂NHSO₂CH₃ | -CO₂CH₃ |
| BBT | -CH₂CO₂CH₃ | -CO₂CH₃ |
| BBU | -CH₂CO₂CH₂CH₃ | -CO₂CH₃ |
| BBV | -(CH₂)₂CO₂CH₃ | -CO₂CH₃ |
| BBW | -(CH₂)₂CO₂CH₂CH₃ | -CO₂CH₃ |
| BB7C | -(CH₂)₃CO₂CH₃ | -CO₂CH₃ |
| BBY | -(CH₂)₃CO₂CH₂CH₃ | -CO₂CH₃ |
| BBZ | -(CH₂)₄CO₂CH₃ | -CO₂CH₃ |
| BCA | -(CH₂)₄CO₂CH₂CH₃ | -CO₂CH₃ |
| BCB | -CH₂SO₂NH₂ | -CO₂CH₃ |
| BCC | -CH₂SO₂N(CH₃)₂ | -CO₂CH₃ |
| BCD | -(CH₂)₂NHSO₂H | -CO₂CH₃ |
| BCE | -(CH₂)₂NHSO₂CH₃ | -CO₂CH₃ |
| BCF | - H | -CHO |
| BCG | -CH₂C(O)NH₂ | -CHO |
| BCH | -CH₂C(O)N(CH₃)₂ | -CHO |
| BCI | -C(O)CH₂NHSO₂CH₃ | -CHO |
| BCJ | -(CH₂)₂C(O)NH₂ | -CHO |
| BCK | -(CH₂)₂C(O)N(CH₃)₂ | -CHO |
| BCL | -C(O)(CH₂)₂NHSO₂CH₃ | -CHO |
| BCM | -CH₂CO₂CH₃ | -CHO |
| BCN | -CH₂CO₂CH₂CH₃ | -CHO |
| BCO | -(CH₂)₂CO₂CH₃ | -CHO |
| BCP | -(CH₂)₂CO₂CH₂CH₃ | -CHO |
| BCQ | -(CH₂)₃CO₂CH₃ | -CHO |
| BCR | -(CH₂)₃CO₂CH₂CH₃ | -CHO |
| BCS | -(CH₂)₄CO₂CH₃ | -CHO |
| BCT | -(CH₂)₄CO₂CH₂CH₃ | -CHO |
| BCU | -CH₂SO₂NH₂ | -CHO |
| BCV | -CH₂SO₂N(CH₃)₂ | -CHO |
| BCW | -(CH₂)₂NHSO₂H | -CHO |
| BCX | -(CH₂)₂NHSO₂CH₃ | -CHO |
| BCY | - H | -CN |
| BCZ | -CH₂C(O)NH₂ | -CN |
| BDA | -CH₂C(O)N(CH₃)₂ | -CN |
| BDB | -C(O)CH₂NHSO₂CH₃ | -CN |
| BDC | -(CH₂)₂C(O)NH₂ | -CN |
| BDD | -(CH₂)₂C(O)N(CH₃)₂ | -CN |
| BDE | -C(O)(CH₂)₂NHSO₂CH₃ | -CN |
| BDF | -CH₂CO₂CH₃ | -CN |
| BDG | -CH₂CO₂CH₂CH₃ | -CN |
| BDH | -(CH₂)₂CO₂CH₃ | -CN |
| BDI | -(CH₂)₂CO₂CH₂CH₃ | -CN |
| BDJ | -(CH₂)₃CO₂CH₃ | -CN |
| BDK | -(CH₂)₃CO₂CH₂CH₃ | -CN |
| BDL | -(CH₂)₄CO₂CH₃ | -CN |
| BDM | -(CH₂)₄CO₂CH₂CH₃ | -CN |
| BND | -CH₂SO₂NH₂ | -CN |
| BDO | -CH₂SO₂N(CH₃)₂ | -CN |
| BDP | -(CH₂)₂NHSO₂H | -CN |
| BDQ | -(CH₂)₂NHSO₂CH₃ | -CN |
| BDR | - H | -CH₃ |
| BDS | -CH₂C(O)NH₂ | -CH₃ |
| BDT | -CH₂C(O)N(CH₃)₂ | -CH₃ |
| BDU | -C(O)CH₂NHSO₂CH₃ | -CH₃ |
| BDV | -(CH₂)₂C(O)NN₂ | -CH₃ |
| BDW | -(CH₂)₂C(O)N(CH₃)₂ | -CH₃ |
| BDX | -C(O)(CH₂)₂NHSO₂CH₃ | -CH₃ |
| BDY | -CH₂CO₂CH₃ | -CH₃ |
| BDZ | -CH₂CO₂CH₂CH₃ | -CH₃ |
| BEA | -(CH₂)₂CO₂CH₃ | -CH₃ |
| BEB | -(CH₂)₂CO₂CH₂CH₃ | -CH₃ |
| BEC | -(CH₂)₃CO₂CH₃ | -CH₃ |
| BED | -(CH₂)₃CO₂CH₂CH₃ | -CH₃ |
| BEE | -(CH₂)₄CO₂CH₃ | -CH₃ |
| BEF | -(CH₂)4CO₂CH₂CH₃ | -CH₃ |
| BEG | -CH₂SO₂NH₂ | -CH₃ |
| BEH | -CH₂SO₂N(CH₃)₂ | -CH₃ |
| BEI | -(CH₂)₂NHSO₂H | -CH₃ |
| BEJ | -(CH₂)₂NHSO₂CH₃ | -CH₃ |
| BEK | - H | -C(O)NH(CH₃) |
| BEL | -CH₂C(O)NH₂ | -C(O)NH(CH₃) |
| BEM | -CH₂C(O)N(CH₃)₂ | -C(O)NH(CH₃) |
| BEN | -C(O)CH₂NHSO₂CH₃ | -C(O)NH(CH₃) |
| BEO | -(CH₂)₂C(O)NH₂ | -C(O)NH(CH₃) |
| BEP | -(CH₂)₂C(O)N(CH₃)₂ | -C(O)NH(CH₃) |
| BEQ | -C(O)(CH₂)₂NHSO₂CH₃ | -C(O)NH(CH₃) |
| BER | -CH₂CO₂CH₃ | -C(O)NH(CH₃) |
| BES | -CH₂CO₂CH₂CH₃ | -C(O)NH(CH₃) |
| BET | -(CH₂)₂CO₂CH₃ | -C(O)NH(CH₃) |
| BEU | -(CH₂)₂CO₂CH₂CH₃ | -C(O)NH(CH₃) |
| BEV | -(CH₂)₃CO₂CH₃ | -C(O)NH(CH₃) |
| BEW | -(CH₂)₃CO₂CH₂CH₃ | -C(O)NH(CH₃) |
| BEX | -(CH₂)₄CO₂CH₃ | -C(O)NH(CH₃) |
| BEY | -(CH₂)₄CO₂CH₂CH₃ | -C(O)NH(CH₃) |
| BEZ | -CH₂SO₂NH₂ | -C(O)NH(CH₃) |
| BFA | -CH₂SO₂N(CH₃)₂ | -C(O)NH(CH₃) |
| BFB | -(CH₂)₂NHSO₂H | -C(O)NH(CH₃) |
| BFC | -(CH₂)₂NHSO₂CH₃ | -C(O)NH(CH₃) |
| BFD | - H | -C(O)N(CH₃)₂ |
| BFE | -CH₂C(O)NH₂ | -C(O)N(CH₃)₂ |
| BFF | -CH₂C(O)N(CH₃)₂ | -C(O)N(CH₃)₂ |
| BFG | -C(O)CH₂NHSO₂CH₃ | -C(O)N(CH₃)₂ |
| BFH | -(CH₂)₂C(O)NH₂ | -C(O)N(CH₃)₂ |
| BFI | -(CH₂)₂C(O)N(CH₃)₂ | -C(O)N(CH₃)₂ |
| BFJ | -C(O)(CH₂)₂NHSO₂CH₃ | -C(O)N(CH₃)₂ |
| BFK | -CH₂CO₂CH₃ | -C(O)N(CH₃)₂ |
| BFL | -CH₂CO₂CH₂CH₃ | -C(O)N(CH₃)₂ |
| BFM | -(CH₂)₂CO₂CH₃ | -C(O)N(CH₃)₂ |
| BFN | -(CH₂)₂CO₂CH₂CH₃ | -C(O)N(CH₃)₂ |
| BFO | -(CH₂)₃CO₂CH₃ | -C(O)N(CH₃)₂ |
| BFP | -(CH₂)₃CO₂CH₂CH₃ | -C(O)N(CH₃)₂ |
| BFQ | -(CH₂)₄CO₂CH₃ | -C(O)N(CH₃)₂ |
| BFR | -(CH₂)₄CO₂CH₂CH₃ | -C(O)N(CH₃)₂ |
| BFS | -CH₂SO₂NH₂ | -C(O)N(CH₃)₂ |
| BFT | -CH₂SO₂N(CH₃)₂ | -C(O)N(CH₃)₂ |
| BFU | -(CH₂)₂NHSO₂H | -C(O)N(CH₃)₂ |
| BFV | -(CH₂)₂NHSO₂CH₃ | -C(O)N(CH₃)₂ |
| BFW | - H | 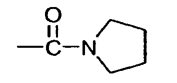 |
| BFX | -CH₂C(O)NH₂ | 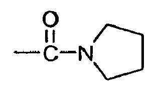 |
| BFY | -CH₂C(O)N(CH₃)₂ | 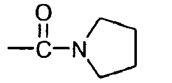 |
| BFZ | -C(O)CH₂NHSO₂CH₃ | 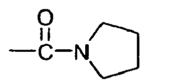 |
| BGA | -(CH₂)₂C(O)NH₂ | 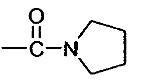 |
| BGB | -(CH₂)₂C(O)N(CH₃)₂ | 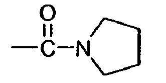 |
| BGC | -C(O)(CH₂)₂NHSO₂CH₃ | 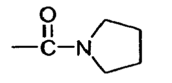 |
| BGD | -CH₂CO₂CH₃ | 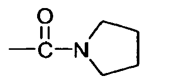 |
| BGE | -CH₂CO₂CH₂CH₃ | 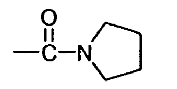 |
| BGF | -(CH₂)₂CO₂CH₃ | 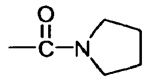 |
| BGG | -(CH₂)₂CO₂CH₂CH₃ | 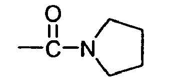 |
| BGH | -(CH₂)₃CO₂CH₃ | 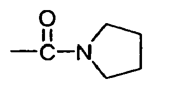 |
| BGI | -(CH₂)₃CO₂CH₂CH₃ | 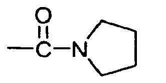 |
| BGJ | -(CH₂)₄CO₂CH₃ | 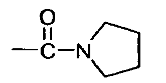 |
| BGK | -(CH₂)₄CO₂CH₂CH₃ | 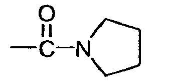 |
| BGL | -CH₂SO₂NH₂ | 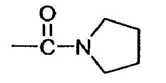 |
| BGM | -CH₂SO₂N(CH₃)₂ | 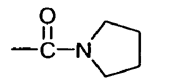 |
| BGN | -(CH₂)₂NHSO₂H | 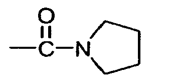 |
| BGO | -(CH₂)₂NHSO₂CH₃ | 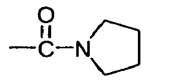 |
| BGP | -H | 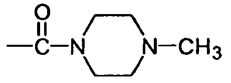 |
| BGQ | -CH₂C(O)NH₂ | 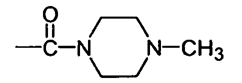 |
| BGR | -CH₂C(O)N(CH₃)₂ | 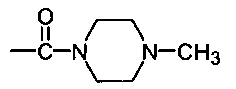 |
| BGS | -C(O)CH₂NHSO₂CH₃ | 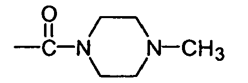 |
| BGT | -(CH₂)₂C(O)NH₂ | 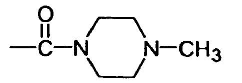 |
| BGU | -(CH₂)₂C(O)N(CH₃)₂ | 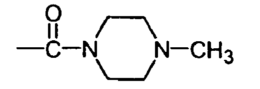 |
| BGV | -C(O)(CH₂)₂NHSO₂CH₃ | 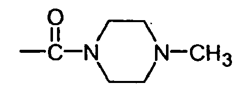 |
| BGW | -CH₂CO₂CH₃ | 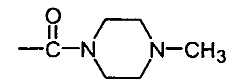 |
| BGX | -CH₂CO₂CH₂CH₃ | 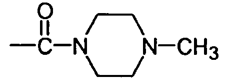 |
| BGY | -(CH₂)₂CO₂CH₃ | 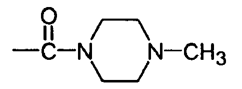 |
| BGZ | -(CH₂)₂CO₂CH₂CH₃ | 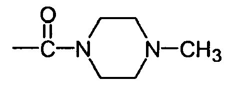 |
| BHA | -(CH₂)₃CO₂CH₃ | 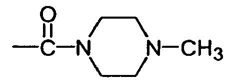 |
| BHB | -(CH₂)₃CO₂CH₂CH₃ | 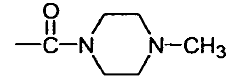 |
| BHC | -(CH₂)₄CO₂CH₃ | 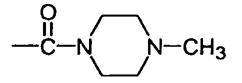 |
| BHD | -(CH₂)₄CO₂CH₂CH₃ | 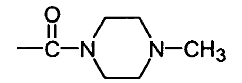 |
| BHE | -CH₂SO₂NH₂ | 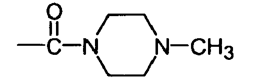 |
| BHF | -CH₂SO₂N(CH₃)₂ | 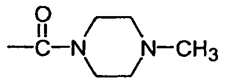 |
| BHG | -(CH₂)₂NHSO₂H | 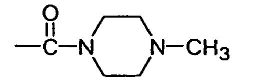 |
| BHH | -(CH₂)₂NHSO₂CH₃ | 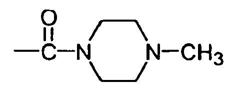 |
| BHI | -H | 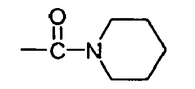 |
| BHJ | -CH₂C(O)NH₂ | 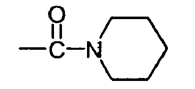 |
| BHK | -CH₂C(O)N(CH₃)₂ | 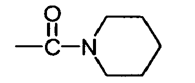 |
| BHL | -C(O)CH₂NHSO₂CH₃ | 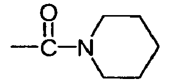 |
| BHM | -(CH₂)₂C(O)NH₂ | 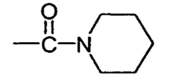 |
| BHN | -(CH₂)₂C(O)N(CH₃)₂ | 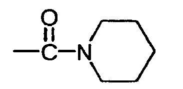 |
| BHO | -C(O)(CH₂)₂NHSO₂CH₃ | 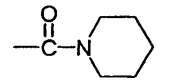 |
| BHP | -CH₂CO₂CH₃ | 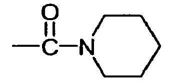 |
| BHQ | -CH₂CO₂CH₂CH₃ | 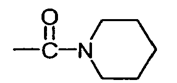 |
| BHR | -(CH₂)₂CO₂CH₃ | 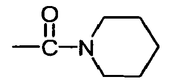 |
| BHS | -(CH₂)₂CO₂CH₂CH₃ | 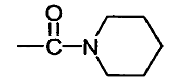 |
| BHT | -(CH₂)₃CO₂CH₃ | 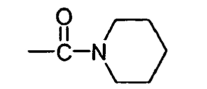 |
| BHU | -(CH₂)₃CO₂CH₂CH₃ | 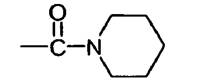 |
| BHV | -(CH₂)₄CO₂CH₃ | 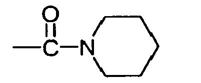 |
| BHW | -(CH₂)₄CO₂CH₂CH₃ | 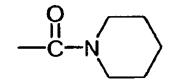 |
| BHX | -CH₂SO₂NH₂ | 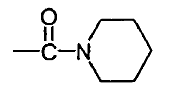 |
| BHY | -CH₂SO₂N(CH₃)₂ | 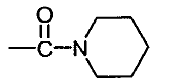 |
| BHZ | -(CH₂)₂NHSO₂H | 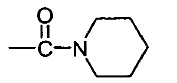 |
| BIA | -(CH₂)₂NHSO₂CH₃ | 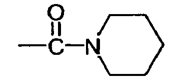 |
| BIB | -H | 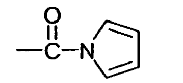 |
| BIC | -CH₂C(O)NH₂ | 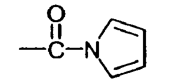 |
| BID | -CH₂C(O)N(CH₃)₂ | 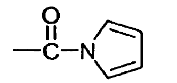 |
| BIE | -C(O)CH₂NHSO₂CH₃ | 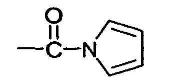 |
| BIF | -(CH₂)₂C(O)NH₂ | 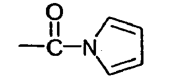 |
| BIG | -(CH₂)₂C(O)N(CH₃)₂ | 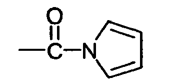 |
| BIH | -C(O)(CH₂)₂NHSO₂CH₃ | 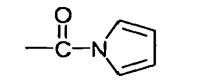 |
| BII | -CH₂CO₂CH₃ | 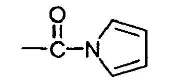 |
| BIJ | -CH₂CO₂CH₂CH₃ | 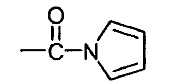 |
| BIK | -(CH₂)₂CO₂CH₃ | 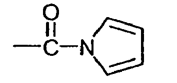 |
| BIL | -(CH₂)₂CO₂CH₂CH₃ | 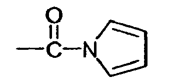 |
| BIM | -(CH₂)₃CO₂CH₃ | 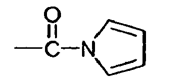 |
| BIN | -(CH₂)₃CO₂CH₂CH₃ | 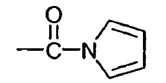 |
| BIO | -(CH₂)₄CO₂CH₃ | 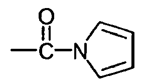 |
| BIP | -(CH₂)₄CO₂CH₂CH₃ | 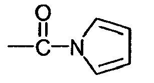 |
| BIQ | -CH₂SO₂NH₂ | 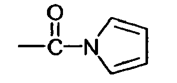 |
| BIR | -CH₂SO₂N(CH₃)₂ | 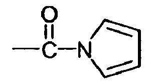 |
| BIS | -(CH₂)₂NHSO₂H | 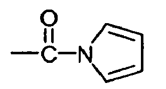 |
| BIT | -(CH₂)₂NHSO₂CH₃ | 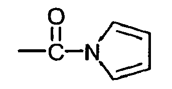 |
| BIU | -H | 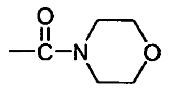 |
| BIV | -CH₂C(O)NH₂ | 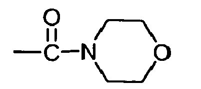 |
| BIW | -CH₂C(O)N(CH₃)₂ | 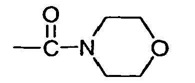 |
| BIX | -C(O)CH₂NHSO₂CH₃ | 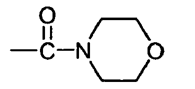 |
| BIY | -(CH₂)₂C(O)NH₂ | 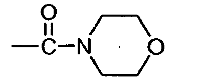 |
| BIZ | -(CH₂)₂C(O)N(CH₃)₂ | 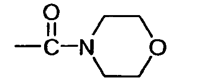 |
| BJA | -C(O)(CH₂)₂NHSO₂CH₃ | 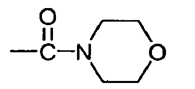 |
| BJB | -CH₂CO₂CH₃ | 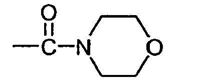 |
| BJC | -CH₂CO₂CH₂CH₃ | 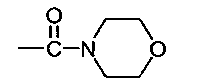 |
| BJD | -(CH₂)₂CO₂CH₃ | 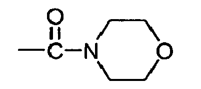 |
| BJE | -(CH₂)₂CO₂CH₂CH₃ | 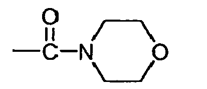 |
| BJF | -(CH₂)₃CO₂CH₃ | 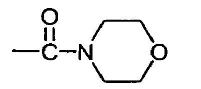 |
| BJG | -(CH₂)₃CO₂CH₂CH₃ | 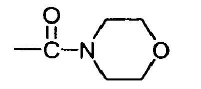 |
| BJH | -(CH₂)₄CO₂CH₃ | 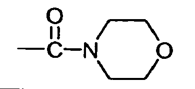 |
| BJI | -(CH₂)₄CO₂CH₂CH₃ | 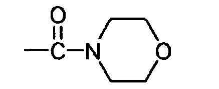 |
| BJJ | -CH₂SO₂NH₂ | 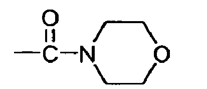 |
| BJK | -CH₂SO₂N(CH₃ₕ | 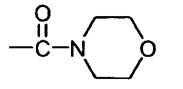 |
| BJL | -(CH₂)₂NHSO₂H | 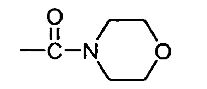 |
| BJM | -(CH₂)₂NHSO₂CH₃ | 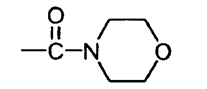 |
| BJN | -(CH₂)₃NHSO₂H | -H |
| BJO | -(CH₂)₃NHSO₂H | -C(O)NH₂ |
| BJP | -(CH₂)₃NHSO₂H | -CO₂CH₃ |
| BJQ | -(CH₂)₃NHSO₂H | -CHO |
| BJR | -(CH₂)₃NHSO₂H | -CN |
| BJS | -(CH₂)₃NHSO₂H | -CH₃ |
| BJT | -(CH₂)₃NHSO₂H | -C(O)NHCH₃ |
| BJU | -(CH₂)₃NHSO₂H | -C(O)N(CH₃)₂ |
| BJV | -(CH₂)₃NHSO₂H | 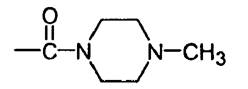 |
| BJW | -(CH₂)₃NHSO₂H | 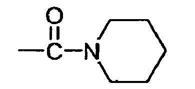 |
| BJX | -(CH₂)₃NHSO₂H | 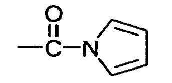 |
| BJY | -(CH₂)₃NHSO₂H | 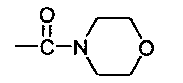 |

### 4.3 Methods for Making the 4-Oxadiazolyl-piperidine Compounds

The 4-Oxadiazolyl-piperidine Compounds of the present invention can be made using conventional organic syntheses as well as by the following illustrative methods.

### 4.3.1 Synthesis of Compounds of Structure 3

Scheme 1 depicts methods for making intermediates useful in the synthesis of 4-Oxadiazolyl-piperidine Compounds of formula I and formula II, in which R¹ is -C(O)NZ¹Z², where Z¹ and Z² are each independently a -(C₁-C₄ alkyl) group or Z¹ and Z² and the nitrogen atom to which they are attached are taken together to form N-(4-R⁴)-N'-1-piperazinyl, aziridyl, azetidyl, pyrrolidyl, piperidyl, homopiperidyl, pyrrolyl or morpholinyl.

Bromoacids 1 are converted to bromoacid chlorides 2 using thionylchloride (J.S. Pizey, Synthetic Reactions 2: 65 (1974)), or, as depicted, using oxalyl chloride. Bromoacid chlorides 2 are reacted with Z¹Z² NH, optionally in the presence of base such as Na₂CO₃, to provide reactive intermediates 3. 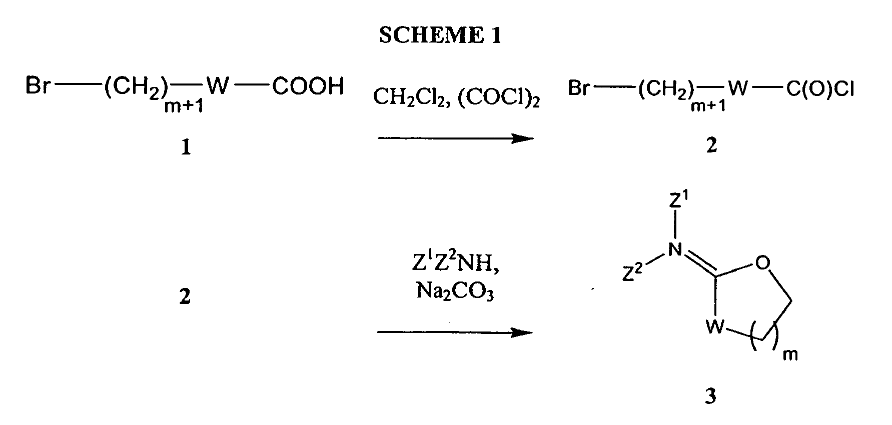 where -W- is -C(Ar¹)(Ar²)- or -C(H)(Ar³)-, and m, Ar¹, Ar², and Ar³ are as defined above.

### 4.3.2 Synthesis of Compounds of Structure 10

Scheme 2 depicts methods for making intermediates useful in the synthesis 4-Oxadiazolyl-piperidine Compounds of formula l and formula ll, in which G is, for example, -CH₂C(O)(CH₂)ₙCH₃ and where n, p, R³, and Ar⁴ are as defined above.

Nitriles 4 are added to a mixture of hydroxylamine hydrochloride and potassium carbonate. The resulting suspension is stirred at room temperature and then at reflux temperature to provide the amidoxime product 5. The amidoximes 5 are reacted with malonyl chloride derivates 6 to provide the product N-benzyl-piperidinyl compounds 7. The N-benzyl-piperidinyloxadiazoles 7 are heated in DMF in the presence of molecular sieves to provide the N-benzyl-oxadiazolyl-piperidine compounds 8, which are reacted with 1-chloroethyl chloroformate 9 to remove the benzyl moiety and provide the oxadiazolyl piperidines 10.

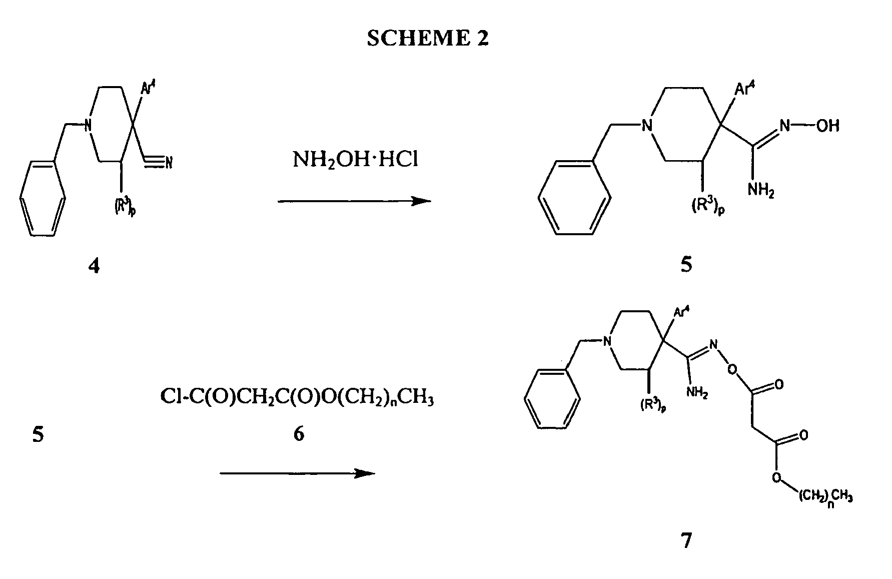

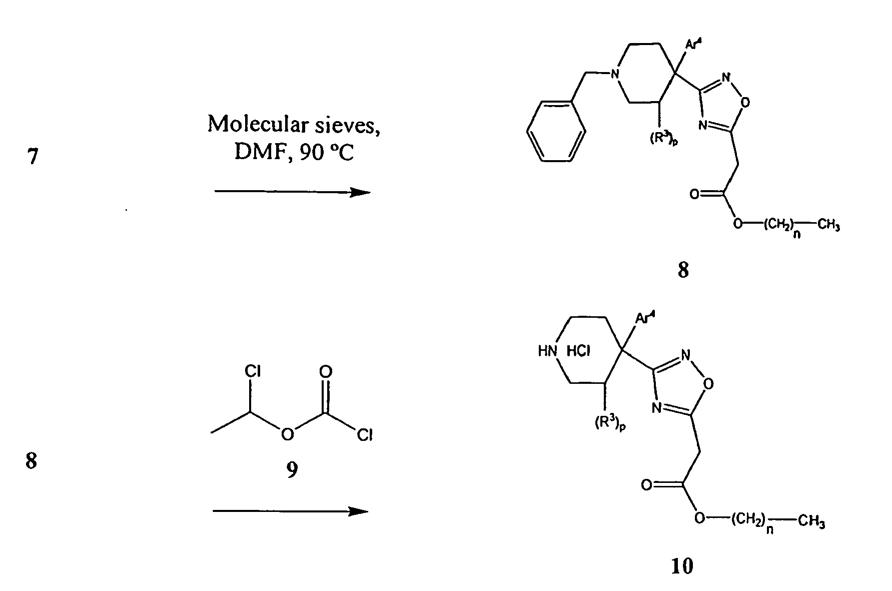

### 4.3.3 Synthesis of Compounds of Structure 12

Scheme 3 depicts methods for use in the synthesis 4-Oxadiazolyl-piperidine Compounds of formula I and formula II, in which R¹ is, for example, -C(O)NZ¹Z², where Z¹ and Z² are each independently a -(C₁-C₄ alkyl) group or Z¹ and Z² and the nitrogen atom to which they are attached are taken together to form N-(4-R⁴)-N'-1-piperazinyl, aziridyl, azetidyl, pyrrolidyl, piperidyl, homopiperidyl, pyrrolyl or morpholinyl; and in which G is, for example, -CH₂C(O)(CH₂)ₙCH₃; where -W- is -C(Ar¹)(Ar²)- or -C(H)(Ar³)- ; and n, m, p, Ar¹, Ar², Ar³, Ar⁴, and R³ are as defined above.

Reactive intermediates 3 and oxadiazolylpiperidines 10 are combined and stirred, initially at 0°C, and then at room temperature to provide 4-Oxadiazolyl-piperidine compounds 11. The 4-Oxadiazolyl-piperidine compounds 11, may be dissolved in ammonia in methanol (e.g. 7N, Aldrich) and stirred at reflux temperature to provide the corresponding amides 12.

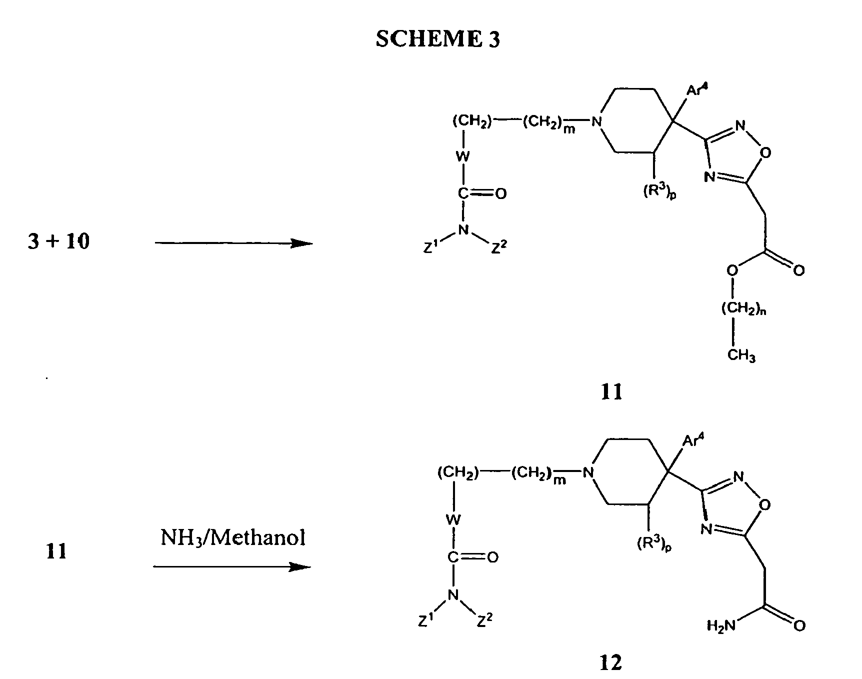

### 4.3.4 Synthesis of Compounds of Structure 14

Scheme 4 depicts methods for use in the synthesis 4-Oxadiazolyl-piperidine Compounds of formula I and formula 11, in which R¹ is -CO₂R⁴ and G is, for example, -CH₂C(0)(CH₂)ₙCH₃; where -W- is -C(Ar¹)(Ar²)- or -C(H)(Ar³)- ; and n, m, p, Ar¹, Ar², Ar³, Ar⁴, and R³ are as defined above.

Bromoacid chlorides 2 (Scheme 1) are reacted with R⁴0H, optionally in the presence of a base such as pyridine, 4-dimethylaminopyridine, triethylamine or Hünig's base, to provide bromoesters 13. Bromoesters 13 are reacted with oxadiazolylpiperidines 10 (Scheme 2) to provide 4-oxadiazolyl-piperidines 14.

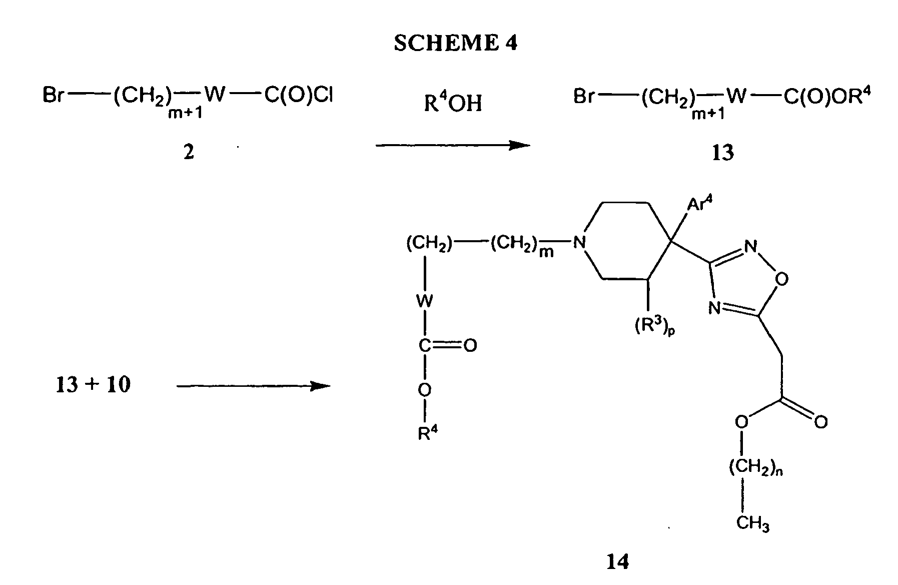

### 4.3.5 Synthesis of Compounds of Structure 4

In certain embodiments, compounds according to structure 4 *(e.g.* those in which where p is 0), which can be used in the synthesis of 4-Oxadiazolyl-piperidine Compounds of the invention, are prepared according to Scheme 5.

Commercially-available (Aldrich) benzyl compounds 25, where X is -Cl or -Br, are reacted with bis(2-chloroethyl)amine 26 (Aldrich) in DMF in the presence of triethylamine at a temperature within the range of room temperature to 80 °C to provide the benzyl-protected amine 27.

Substituted acetonitrile 28, dissolved in THF, is first treated with NaH at room temperature and then reacted with benzyl-protected amine 27 at reflux temperature in THF to provide the nitrile 4, which can be used, for example, in the synthesis depicted in Scheme 2, above.

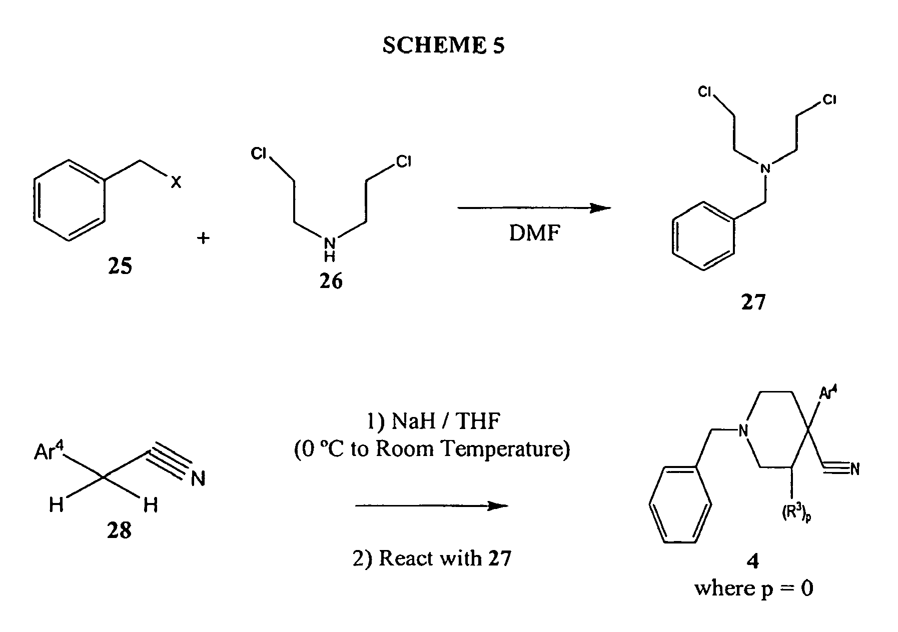

Each structure depicted herein is intended to encompass all stereoisomers and tautomers thereof, each of which is understood to be included in the present invention, whether specifically disclosed or not, and whether or not the stereoisomer or tautomer depicted herein represents a stereoisomer or tautomer in excess relative to any other stereoisomer ir tautomer thereof. Accordingly, the invention also encompasses 4-Oxadiazolyl-piperidine Compounds and their uses as described herein in the form of their individual stereoisomer or tautomers. In addition, one or more hydrogen, carbon or other atoms of a 4-Oxadiazolyl-piperidine Compound can be replaced by an isotope of the hydrogen, carbon or other atoms, respectively. Such compounds, which are encompassed by the present invention, are useful as research and diagnostic tools in metabolic pharmacokinetic studies and in binding assays.

### 4.4 Therapeutic Uses of the 4-0xadiazolyl-piperidine Compounds

In accordance with the invention, the 4-Oxadiazolyl-piperidine Compounds are administered to an animal, in one embodiment a mammal, in another embodiment a human, for the treatment or prevention of pain. The 4-Oxadiazolyl-piperidine Compounds can be used to treat or prevent acute or chronic pain. For example, the 4-Oxadiazolyl-piperidine Compounds can be used for, but are not limited to, treating or preventing cancer pain, central pain, labor pain, myocardial infarction pain, pancreatic pain, colic pain, post operative pain, headache pain, muscle pain, and pain associated with intensive care.

The 4-Oxadiazolyl-piperidine Compounds can also be used for inhibiting, preventing, or treating pain associated with inflammation or with an inflammatory disease in an animal. The pain to be inhibited, treated or prevented may be associated with inflammation associated with an inflammatory disease, which can arise where there is an inflammation of the body tissue, and which can be a local inflammatory response and/or a systemic inflammation. For example, the 4-Oxadiazolyl-piperidine Compounds can be used to inhibit, treat, or prevent pain associated with inflammatory diseases including, but not limited to: organ transplant rejection; reoxygenation injury resulting from organ transplantation (see Grupp et al. J. Mol. Cell Cardiol. 31:297 303 (1999)) including, but not limited to, transplantation of the heart, lung, liver, or kidney; chronic inflammatory diseases of the joints, including arthritis, rheumatoid arthritis, osteoarthritis and bone diseases associated with increased bone resorption; inflammatory bowel diseases, such as ileitis, ulcerative colitis, Barrett's syndrome, and Crohn's disease; inflammatory lung diseases, such as asthma, adult respiratory distress syndrome, and chronic obstructive airway disease; inflammatory diseases of the eye, including corneal dystrophy, trachoma, onchocerciasis, uveitis, sympathetic ophthalmitis and endophthalmitis; chronic inflammatory diseases of the gum, including gingivitis and periodontitis; tuberculosis; leprosy; inflammatory diseases of the kidney, including uremic complications, glomerulonephritis and nephrosis; inflammatory diseases of the skin, including sclerodermatitis, psoriasis and eczema; inflammatory diseases of the central nervous system, including chronic demyelinating diseases of the nervous system, multiple sclerosis, AIDS-related neurodegeneration and Alzheimer s disease, infectious meningitis, encephalomyelitis, Parkinson's disease, Huntington's disease, amyotrophic lateral sclerosis, and viral or autoimmune encephalitis; autoimmune diseases, including Type I and Type 11 diabetes mellitus; diabetic complications, including, but not limited to, diabetic cataract, glaucoma, retinopathy, nephropathy (such as microaluminuria and progressive diabetic nephropathy), polyneuropathy, mononeuropathies, autonomic neuropathy, gangrene of the feet, atherosclerotic coronary arterial disease, peripheral arterial disease, nonketotic hyperglycemic hyperosmolar coma, foot ulcers, joint problems, and a skin or mucous membrane complication (such as an infection, a shin spot, a candidal infection or necrobiosis lipoidica diabeticorum); immune-complex vasculitis, systemic lupus erythematosus (SLE); inflammatory diseases of the heart, such as cardiomyopathy, ischemic heart disease hypercholesterolemia, and atherosclerosis; as well as various other diseases that can have significant inflammatory components, including preeclampsia, chronic liver failure, brain and spinal cord trauma, and cancer. The 4-Oxadiazolyl-piperidine Compounds can also be used for inhibiting, treating, or preventing pain associated with inflammatory disease that can, for example, be a systemic inflammation of the body, exemplified by gram-positive or gram negative shock, hemorrhagic or anaphylactic shock, or shock induced by cancer chemotherapy in response to pro-inflammatory cytokines, e.g., shock associated with pro-inflammatory cytokines. Such shock can be induced, e.g., by a chemotherapeutic agent that is adminstered as a treatment for cancer.

In another embodiment, the 4-Oxadiazolyl-piperidine Compounds are administered to an animal, in one embodiment a mammal, in another embodiment a human, for the treatment or prevention of diarrhea. The 4-Oxadiazolyl-piperidine Compounds can be used to treat or prevent acute or chronic diarrhea. For example, the 4-Oxadiazolyl-piperidine Compounds can be used for, but are not limited to, treating or preventing acute diarrhea caused by a virus, such as but not limited to Norwalk-virus, Norwalk-like virus, Rotavirus, and Cytomegaloviurs: protozoa, such as but not limited to *Girardia lamlia, Crpytosporidium* and *Entamoeba histolytica;* and bacteria, including but not limited to *Stapylococcus aureus, Bacillus cereus, Clostridium perfringens,* enterotoxigenic *E. coli, Vibrio cholera,* enterohemmorrhagic *E. coli* 0157:H5, *Vibrio parahaemolyticus, Clostridium difficile, Campylobacter jejuni, Salmonella,* enteroinvasive *E. coli, Aeromonas, Plesiomonas, Yersinia enterocolitica, Chlamydia, Nisseria gonorrhoeae,* and *Listeria monocytogenes.* For example, the 4-0xadiazolyl-piperidine Compounds can be used for treating or preventing chronic diarrhea classified as including, but not limited to, osmotic diarrhea, secretory diarrhea, or resulting from an inflammatory condition, a malabsorption syndrome, a motility disorder, and a chronic infection.

The present inventor believes that unlike traditional opioid agonists and nonsteroidal anti-inflammatory agents, the 4-Oxadiazolyl-piperidine Compounds do not significantly cross the blood-brain barrier. Accordingly, the administration of an effective amount of a 4-Oxadiazolyl-piperidine Compound to an animal should result in fewer side effects, including, for example, respiratory depression, unwanted euphoria, sedation, increased drug tolerance, and increased drug dependence, that can result from the administration of traditional opioid agonists or nonsteroidal anti-inflammatory agents. In one embodiment, the administration of an effective amount of a 4-0xadiazolyl-piperidine Compound to an animal results in none of the aforementioned side effects. Therefore, in certain embodiments, the present methods encompass treating or preventing pain, while reducing or eliminating one or more of the aforementioned side effects.

Without wishing to be bound by theory, it is believed that the 4-Oxadiazolyl-piperidine Compounds are agonists for and, accordingly, are capable of stimulating an opioid receptor. In one embodiment, the opiod receptor is a µ receptor. In another embodiment, the opioid receptor is an ORL-1 receptor. In a further embodiment, the opioid receptor is a δ-opioid receptor.

The invention also relates to methods for stimulating opioid-receptor function in a cell comprising contacting a cell capable of expressing an opioid receptor with an effective amount of a 4-Oxadiazolyl-piperidine Compound. The method is also useful for stimulating opioid receptor function in a cell *in vivo,* in an animal, in one embodiment a human, by contacting a cell capable of expressing an opioid receptor, in an animal, with an effective amount of a 4-Oxadiazolyl-piperidine Compound. In one embodiment, the method is useful for treating or preventing pain or diarrhea in an animal. Brain tissue, spinal chord tissue, immune cells, cells of the gastrointestinal tract, and primary afferent nerve cells are examples of tissues and/or cells that are capable of expressing an opioid receptor. This method can be used *in vitro,* for example, as an assay to select cells that express an opioid receptor.

### 4.4.1 Therapeutic/Prophylactic Administration and Compositions of the Invention

Due to their activity, the 4-Oxadiazolyl-piperidine Compounds are advantageously useful in veterinary and human medicine. As described above, the 4-Oxadiazolyl-piperidine Compounds are useful for treating or preventing pain or diarrhea in an animal in need thereof.

When administered to an animal, the 4-Oxadiazolyl-piperidine Compounds can be administered as a component of a pharmaceutical composition that comprises a pharmaceutically acceptable carrier or excipient. The present compositions, which comprise a 4-Oxadiazolyl-piperidine Compound, are in one embodiment administered orally. The compositions of the invention can also be adapted for and administered by any other convenient route, for example, by infusion or bolus injection, by absorption through epithelial or mucocutaneous linings *(e.g.,* oral mucosa, rectal, and intestinal mucosa, *etc.)* and can be administered alone or together with another therapeutic agent. Administration can be systemic or local. Various delivery systems are known, e.g., encapsulation in liposomes, microparticles, microcapsules, capsules, etc., and can be used to administer the 4-Oxadiazolyl-piperidine Compounds.

Methods of administration include but are not limited to intradermal, intramuscular, intraperitoneal, intravenous, subcutaneous, intranasal, epidural, oral, sublingual, intracerebral, intravaginal, transdermal, rectal, by inhalation, or topical, particularly to the ears, nose, eyes, or skin. The mode of administration is left to the discretion of the practitioner. In most instances, administration will result in the release of a therapeutically effective amount of the 4-Oxadiazolyl-piperidine Compounds into the bloodstream.

In specific embodiments, it may be desirable to administer the 4-Oxadiazolyl-piperidine Compounds locally. This may be achieved, for example, by local infusion during surgery, topical application, e.g., in conjunction with a wound dressing after surgery, by injection, by means of a catheter, by means of a suppository, or by means of an implant, said implant being of a porous, non porous, or gelatinous material, including membranes, such as sialastic membranes, or fibers.

Pulmonary administration can also be employed, e.g., by use of an inhaler or nebulizer, and formulation with an aerosolizing agent, or via perfusion in a fluorocarbon or synthetic pulmonary surfactant. In certain embodiments, the 4-Oxadiazolyl-piperidine Compounds can be formulated as a suppository, with traditional binders and excipients such as triglycerides.

In another embodiment, the 4-Oxadiazolyl-piperidine Compounds can be delivered in a vesicle, in particular a liposome (see Langer, Science 249:1527-1533 (1990) and Treat et al., Liposomes in the Therapy of Infectious Disease and Cancer 317-327 and 353-365 (1989).

In yet another embodiment, the 4-Oxadiazolyl-piperidine Compounds can be delivered in a controlled-release system *(see, e.g.,* Goodson, in Medical Applications of Controlled Release, supra, vol. 2, pp. 115-138 (1984)). Other controlled release systems discussed in the review by Langer, Science 249:1527-1533 (1990) may be used. In one embodiment, a pump may be used (Langer, Science 249:1527-1533 (1990); Sefton, CRC Crit. Ref Biomed. Eng. 14:201 (1987); Buchwald et al., Surgery 88:507 (1980); and Saudek et al., N. Engl. J. Med. 321:574 (1989)). In another embodiment, polymeric materials can be used *(see* Medical Applications of Controlled Release (Langer and Wise eds., 1974); Controlled Drug Bioavailability, Drug Product Design and Performance (Smolen and Ball eds., 1984); Ranger and Peppas, J. Macromol. Sci. Rev. Macromol. Chem. 23:61 (1983); Levy et al., Science 228:190 (1985); During et al., Ann. Neurol. 25:351 (1989); and Howard et al., J. Neurosurg. 71:105 (1989)). In yet another embodiment, a controlled release system can be placed in proximity of a target of a 4-Oxadiazolyl-piperidine Compound thus requiring only a fraction of the systemic dose. In further embodiments, the 4-Oxadiazolyl-piperidine Compounds can be administered by controlled-release means or by delivery devices that are well known to those of ordinary skill in the art. Examples include, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566, each of which is incorporated herein by reference. Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the 4-Oxadiazolyl-piperidine Compounds. The invention thus encompasses single unit dosage forms suitable for oral administration such as, but not limited to, tablets, capsules, gelcaps, and caplets that are adapted for controlled release.

The present compositions can optionally comprise a suitable amount of a pharmaceutically acceptable excipient so as to provide the form for proper administration to the animal. Such pharmaceutical excipients can be liquids, such as water and oils, including those of petroleum, animal, vegetable, or synthetic origin, such as peanut oil, soybean oil, mineral oil, sesame oil and the like. The pharmaceutical excipients can be saline, gum acacia, gelatin, starch paste, talc, keratin, colloidal silica, urea, and the like. In addition, auxiliary, stabilizing, thickening, lubricating, and coloring agents may be used. When administered to an animal, the pharmaceutically acceptable excipients are preferably sterile. Water is a particularly useful excipient when the 4-Oxadiazolyl-piperidine Compound is administered intravenously. Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The present compositions, if desired, can also contain minor amounts of wetting or emulsifying agents, or pH buffering agents.

Suitable pharmaceutically acceptable carriers or excipients for intravenous administration of the 4-Oxadiazolyl-piperidine Compounds include, but are not limited to, normal (about 0.9%) saline, about 25 to about 30% polyethylene glycol ("PEG") diluted with saline or water, and about 2 to about 30% hydroxypropyl p-cyclodextrin diluted with water. In one embodiment, compositions for intravenous administration comprise the compound dissolved in sterile isotonic aqueous buffer. Where necessary, the compositions may also include a solubilizing agent. Compositions for intravenous administration can optionally include a local anesthetic such as lignocaine to lessen pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the 4-Oxadiazolyl-piperidine Compounds are to be administered by infusion, they can be dispensed, for example, from an infusion bottle containing sterile pharmaceutical grade water or saline. Where the 4-Oxadiazolyl-piperidine Compounds are administered by injection, an ampoule of sterile water for injection or saline can be provided so that the ingredients may be mixed prior to administration.

Suitable pharmaceutically acceptable carriers or excipients for intraperitoneal administration of the 4-Oxadiazolyl-piperidine Compounds include, but are not limited to, normal (about 0.9%) saline, about 25 to about 30% PEG diluted with saline or water, about 25 to about 30% propylene glycol (PG) diluted with saline or water, and about 2 to about 30% hydroxypropyl β-cyclodextrin diluted with water.

Suitable pharmaceutically acceptable carriers or excipients for subcutaneous and intramuscular administration of the 4-Oxadiazolyl-piperidine Compounds include, but are not limited to, water, normal (about 0.9%) saline, about 25 to about 30% PEG diluted with saline or water, and about 25 to about 30% PG diluted with saline or water.

Suitable pharmaceutically acceptable carriers or excipients for oral administration of the 4-Oxadiazolyl-piperidine Compounds include, but are not limited to, water, normal (about 0.9%) saline, about 25 to about 30% polyethylene glycol PEG diluted with saline or water, about 2 to about 30% hydroxypropyl β-cyclodextrin diluted with water, about 25 to about 30% PG diluted with saline or water, and about 1 to about 5% methylcellulose diluted with water.

Suitable pharmaceutically acceptable carriers or excipients for intracerebroventricular and intrathecal administration of the 4-Oxadiazolyl-piperidine Compounds include, but are not limited to, normal (about 0.9%) saline.

The present compositions can take the form of solutions, suspensions, emulsion, tablets, pills, pellets, capsules, capsules containing liquids, powders, sustained release formulations, suppositories, aerosols, sprays, suspensions, or any other form suitable for use. In one embodiment, the composition is in the form of a capsule *(see e.g.,* U.S. Patent No. 5,698,155). Other examples of suitable pharmaceutical excipients are described in Remington's Pharmaceutical Sciences 1447-1676 (Alfonso R. Gennaro ed., 19th ed. 1995), incorporated herein by reference.

In one embodiment, the 4-Oxadiazolyl-piperidine Compounds are formulated in accordance with routine procedures as a composition adapted for oral administration to an animal, particularly a human being. Compositions for oral delivery may be in the form of tablets, lozenges, aqueous or oily suspensions, granules, powders, emulsions, capsules, syrups, or elixirs, for example. Orally administered compositions can contain preserving agents, coloring agents, and one or more agents, for example, sweetening agents such as fructose, aspartame or saccharin and flavoring agents such as peppermint, oil of wintergreen, or cherry, to provide a pharmaceutically palatable preparation. Moreover, where in tablet or pill form, the compositions can be coated or otherwise formulated to delay disintegration and absorption in the gastrointestinal tract thereby providing a sustained action over an extended period of time. Selectively permeable membranes surrounding an osmotically active driving compound are also suitable for orally administered compositions. In these later platforms, fluid from the environment surrounding the capsule is imbibed by the driving compound, which swells to displace the agent or agent composition through an aperture. These delivery platforms can provide an essentially zero order delivery profile as opposed to the spiked profiles of immediate-release formulations. A time delay material such as glycerol monostearate or glycerol stearate may also be used. Oral compositions can include standard excipients such as mannitol, lactose, starch, magnesium stearate, sodium saccharin, cellulose and magnesium carbonate. In one embodiment, such excipients are of pharmaceutical grade.

Controlled release pharmaceutical compositions can have a common goal of improving drug therapy over that achieved by their non-controlled counterparts. In one embodiment a controlled-release composition comprises a minimal amount of a 4-Oxadiazolyl-piperidine Compound to cure or control the condition in a minimum amount of time. Advantages of controlled release compositions include extended activity of the drug, reduced dosage frequency, and increased patient compliance. In addition, controlled release compositions can favorably affect the time of onset of action or other characteristics, such as blood levels of the 4-Oxadiazolyl-piperidine Compound, and can thus reduce the occurrence of adverse side effects.

In one embodiment, controlled release compositions can initially release an amount of a 4-Oxadiazolyl-piperidine Compound that promptly treats or prevents pain or diarrhea, and then gradually and continually release another amount of the 4-Oxadiazolyl-piperidine Compound to maintain this level of therapeutic or prophylactic effect over an extended period of time. To maintain this constant level of the 4-Oxadiazolyl-piperidine Compound in the body, the 4-Oxadiazolyl-piperidine Compound can be released from the dosage form at a rate that will replace the amount of 4-0xadiazolyl-piperidine Compound being metabolized and excreted from the body. Controlled release of an active ingredient can be stimulated by various conditions including, but not limited to, changes in pH, changes in temperature, concentration or availability of enzymes, concentration or availability of water, or other physiological conditions or compounds.

The amount of the 4-Oxadiazolyl-piperidine Compounds that is effective in the treatment or prevention of pain or diarrhea can depend on the nature or severity of the disorder or condition causing the pain and can be determined by standard clinical techniques. In addition, in vitro or in vivo assays can optionally be employed to help identify optimal effective dosage amounts. The precise dose to be employed can also depend on the intended route of administration, and the degree or severity of the pain or diarrhea and can be determined according to the judgment of the medical practitioner in view of each patient's circumstances and published clinical studies. Suitable effective dosage amounts can range from about 10 micrograms to about 2500 milligrams about every 4 h, although typically about 100 mg or less. In one embodiment, the effective dosage amount ranges from about 0.01 milligrams to about 100 milligrams of a 4-Oxadiazolyl-piperidine Compound about every 4 h, in another embodiment about 0.020 milligrams to about 50 milligrams about every 4 h, and in another embodiment about 0.025 milligrams to about 20 milligrams about every 4 h. The dosage amounts described herein refer to total amounts administered; that is, if more than one 4-Oxadiazolyl-piperidine Compound is administered, the effective dosage amounts correspond to the total amount administered.

Where a cell capable of expressing an opioid receptor is contacted with a 4-Oxadiazolyl-pipeiidine Compound *in vitro,* the effective amount for opioid receptor function stimulation will typically range from about 0.01 mg to about 100 mg/L, in one embodiment from about 0.1 mg to about 50 mg/L, and in another embodiment from about 1 mg to about 20 mg/L, of a solution or suspension of a pharmaceutically acceptable carrier or excipient. In one embodiment, the opiod receptor is a µ receptor. In another embodiment, the opiod receptor is an ORL-1 receptor. In a further embodiment, the opioid receptor is a δ-opioid receptor.

Where a cell capable of expressing an opioid receptor is contacted with a 4-Oxadiazolyl-piperidine Compound *in vivo,* the effective amount for opioid receptor function stimulation will typically range from about 0.01 mg to about 100 mg/kg of body weight per day, in one embodiment from about 0.1 mg to about 50 mg/kg body weight per day, and in another embodiment from about 1 mg to about 20 mg/kg of body weight per day. In one embodiment, the opiod receptor is a µ receptor. In another embodiment, the opiod receptor is an ORL-1 receptor. In a further embodiment, the opioid receptor is a δ-opioid receptor.

The 4-Oxadiazolyl-piperidine Compounds can be assayed *in vitro* or *in vivo* for their ability to treat or prevent pain or diarrhea prior to use in humans. Animal model systems can be used to demonstrate the 4-Oxadiazolyl-piperidine Compounds' safety or efficacy.

The present methods for treating or preventing pain or diarrhea in an animal can further comprise administering to the animal an effective amount a 4-Oxadiazolyl-piperidine Compound in combination with an effective amount of another therapeutic agent.

The present methods for stimulating opioid-receptor function in a cell can further comprise contacting the cell with an effective amount of another therapeutic agent.

Examples of other therapeutic agents include, but are not limited to, an opioid agonist, a non-opioid analgesic, a non-steroid antiinflammatory agent, an antimigraine agent, a Cox-II inhibitor, an antiemetic, a β-adrenergic blocker, an anticonvulsant, an antidepressant, a Ca²⁺ -channel blocker, an anticancer agent, an anti-anxiety agent, an agent for treating or preventing an addictive disorder and mixtures thereof.

Effective amounts of the other therapeutic agents are well known to those skilled in the art. However, it is well within the skilled artisan's purview to determine the other therapeutic agent's optimal effective-amount range. In one embodiment of the invention, where another therapeutic agent is administered to an animal, the effective amount of the 4-Oxadiazolyl-piperidine Compound is less than its effective amount would be where the other therapeutic agent is not administered. In this case, without being bound by theory, it is believed that the 4-Oxadiazolyl-piperidine Compound and the other therapeutic agent act synergistically to treat or prevent pain or diarrhea.

Examples of useful opioid agonists include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof.

In certain embodiments, the opioid agonist is selected from codeine, hydromorphone, hydrocodone, oxycodone, dihydrocodeine, dihydromorphine, morphine, tramadol, oxymorphone, pharmaceutically acceptable salts thereof, and mixtures thereof.

Examples of useful non-opioid analgesics include non steroidal anti-inflammatory agents, such as aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, and pharmaceutically acceptable salts thereof, and mixtures thereof. Examples of other suitable non-opioid analgesics include the following, non limiting, chemical classes of analgesic, antipyretic, nonsteroidal antiinflammatory drugs: salicylic acid derivatives, including aspirin, sodium salicylate, choline magnesium trisalicylate, salsalate, diflunisal, salicylsalicylic acid, sulfasalazine, and olsalazin; para aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone. For a more detailed description of the NSAIDs, see Paul A. Insel, Analgesic Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti Inflammatory Drugs in Remington: The Science and Practice of Pharmacy Vol 11 1196-1221 (A.R. Gennaro ed. 19th ed. 1995) which are hereby incorporated by reference in their entireties. Suitable Cox-11 inhibitors and 5-lipoxygenase inhibitors, as well as combinations thereof, are described, among other places, in U.S. Patent No. 6,136,839. Cox II inhibitors include, but are not limited to, rofecoxib and celecoxib.

Examples of useful antimigraine agents include, but are not limited to, alpiropride, dihydroergotamine, dolasetron, ergocomine, ergocominine, ergocryptine, ergot, ergotamine, flumedroxone acetate, fonazine, lisuride, lomerizine, methysergide oxetorone, pizotyline, and mixtures thereof.

The other therapeutic agent can alternatively be an antiemetic agent. Useful antiemetic agents include, but are not limited to, metoclopromide, domperidone, prochlorperazine, promethazine, chlorpromazine, trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, and mixtures thereof.

Examples of useful β-adrenergic blockers include, but are not limited to, acebutolol, alprenolol, amosulabol, arotinolol, atenolol, befunolol, betaxolol, bevantolol, bisoprolol, bopindolol, bucumolol, bufetolol, bufuralol, bunitrolol, bupranolol, butidrine hydrochloride, butofilolol, carazolol, carteolol, carvedilol, celiprolol, cetamolol, cloranolol, dilevalol, epanolol, esmolol, indenolol, labetalol, levobunolol, mepindolol, metipranolol, metoprolol, moprolol, nadolol, nadoxolol, nebivalol, nifenalol, nipradilol, oxprenolol, penbutolol, pindolol, practolol, pronethalol, propranolol, sotalol, sulfinalol, talinolol, tertatolol, tilisolol, timolol, toliprolol, and xibenolol.

Examples of useful anticonvulsants include, but are not limited to, acetylpheneturide, albutoin, aloxidone, aminoglutethimide, 4-amino-3-hydroxybutyric acid, atrolactamide, beclamide, buramate, calcium bromide, carbamazepine, cinromide, clomethiazole, clonazepam, decimemide, diethadione, dimethadione, doxenitroin, eterobarb, ethadione, ethosuximide, ethotoin, felbamate, fluoresone, gabapentin, 5-hydroxytryptophan, lamotrigine, magnesium bromide, magnesium sulfate, mephenytoin, mephobarbital, metharbital, methetoin, methsuximide, 5-methyl-5-(3-phenanthryl)-hydantoin, 3-methyl-5-phenylhydantoin, narcobarbital, nimetazepam, nitrazepam, oxcarbazepine, paramethadione, phenacemide, phenetharbital, pheneturide, phenobarbital, phensuximide, phenylmethylbarbituric acid, phenytoin, phethenylate sodium, potassium bromide, pregabaline, primidone, progabide, sodium bromide, solanum, strontium bromide, suclofenide, sulthiame, tetrantoin, tiagabine, topiramate, trimethadione, valproic acid, valpromide, vigabatrin, and zonisamide.

Examples of useful antidepressants include, but are not limited to, binedaline, caroxazone, citalopram, (S)-citalopram, dimethazan, fencamine, indalpine, indeloxazine hydrocholoride, nefopam, nomifensine, oxitriptan, oxypertine, paroxetine, sertraline, thiazesim, trazodone, benmoxine, iproclozide, iproniazid, isocarboxazid, nialamide, octamoxin, phenelzine, cotinine, rolicyprine, rolipram, maprotiline, metralindole, mianserin, mirtazepine, adinazolam, amitriptyline, amitriptylinoxide, amoxapine, butriptyline, clomipramine, demexiptiline, desipramine, dibenzepin, dimetacrine, dothiepin, doxepin, fluacizine, imipramine, imipramine N-oxide, iprindole, lofepramine, melitracen, metapramine, nortriptyline, noxiptilin, opipramol, pizotyline, propizepine, protriptyline, quinupramine, tianeptine, trimipramine, adrafinil, benactyzine, bupropion, butacetin, dioxadrol, duloxetine, etoperidone, febarbamate, femoxetine, fenpentadiol, fluoxetine, fluvoxamine, hematoporphyrin, hypericin, levophacetoperane, medifoxamine, milnacipran, minaprine, moclobemide, nefazodone, oxaflozane, piberaline, prolintane, pyrisuccideanol, ritanserin, roxindole, rubidium chloride, sulpiride, tandospirone, thozalinone, tofenacin, toloxatone, tranylcypromine, L-tryptophan, venlafaxine, viloxazine, and zimeldine.

Examples of useful Ca²⁺-channel blockers include, but are not limited to, bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, and perhexiline.

Examples of useful anticancer agents include, but are not limited to, acivicin, aclarubicin, acodazole hydrochloride, acronine, adozelesin, aldesleukin, altretamine, ambomycin, ametantrone acetate, aminoglutethimide, amsacrine, anastrozole, anthramycin, asparaginase, asperlin, azacitidine, azetepa, azotomycin, batimastat, benzodepa, bicalutamide, bisantrene hydrochloride, bisnafide dimesylate, bizelesin, bleomycin sulfate, brequinar sodium, bropirimine, busulfan, cactinomycin, calusterone, caracemide, carbetimer, carboplatin, carmustine, carubicin hydrochloride, carzelesin, cedefingol, chlorambucil, cirolemycin, cisplatin, cladribine, crisnatol mesylate, cyclophosphamide, cytarabine, dacarbazine, dactinomycin, daunorubicin hydrochloride, decitabine, dexormaplatin, dezaguanine, dezaguanine mesylate, diaziquone, docetaxel, doxorubicin, doxorubicin hydrochloride, droloxifene, droloxifene citrate, dromostanolone propionate, duazomycin, edatrexate, eflornithine hydrochloride, elsamitrucin, enloplatin, enpromate, epipropidine, epirubicin hydrochloride, erbulozole, esorubicin hydrochloride, estramustine, estramustine phosphate sodium, etanidazole, etoposide, etoposide phosphate, etoprine, fadrozole hydrochloride, fazarabine, fenretinide, floxuridine, fludarabine phosphate, fluorouracil, flurocitabine, fosquidone, fostriecin sodium, gemcitabine, gemcitabine hydrochloride, hydroxyurea, idarubicin hydrochloride, ifosfamide, ilmofosine, interleukin II (including recombinant interleukin II or rIL2), interferon alfa-2a, interferon alfa-2b, interferon alfa-n1, interferon alfa-n3, interferon beta-Ia, interferon gamma-I b, iproplatin, irinotecan hydrochloride, lanreotide acetate, letrozole, leuprolide acetate, liarozole hydrochloride, lometrexol sodium, lomustine, losoxantrone hydrochloride, masoprocol, maytansine, mechlorethamine hydrochloride, megestrol acetate, melengestrol acetate, melphalan, menogaril, mercaptopurine, methotrexate, methotrexate sodium, metoprine, meturedepa, mitindomide, mitocarcin, mitocromin, mitogillin, mitomalcin, mitomycin, mitosper, mitotane, mitoxantrone hydrochloride, mycophenolic acid, nocodazole, nogalamycin, ormaplatin, oxisuran, paclitaxel, pegaspargase, peliomycin, pentamustine, peplomycin sulfate, perfosfamide, pipobroman, piposulfan, piroxantrone hydrochloride, plicamycin, plomestane, porfimer sodium, porfiromycin, prednimustine, procarbazine hydrochloride, puromycin, puromycin hydrochloride, pyrazofurin, riboprine, rogletimide, safingol, safingol hydrochloride, semustine, simtrazene, sparfosate sodium, sparsomycin, spirogermanium hydrochtoride, spiromustine, spiroplatin, streptonigrin, streptozocin, sulofenur, talisomycin, tecogalan sodium, tegafur, teloxantrone hydrochloride, temoporfin, teniposide, teroxirone, testolactone, thiamiprine, thioguanine, thiotepa, tiazofurin, tirapazamine, toremifene citrate, trestolone acetate, triciribine phosphate, trimetrexate, trimetrexate glucuronate, triptorelin, tubulozole hydrochloride, uracil mustard, uredepa, vapreotide, verteporfin, vinblastine sulfate, vincristine sulfate, vindesine, vindesine sulfate, vinepidine sulfate, vinglycinate sulfate, vinleurosine sulfate, vinorelbine tartrate, vinrosidine sulfate, vinzolidine sulfate, vorozole, zeniplatin, zinostatin, zorubicin hydrochloride.

Examples of other anti cancer drugs include, but are not limited to, 20-epi-1,25 dlhydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol; 9-dioxamycin; diphenyl spiromustine; docetaxel; docosanol; dolasetron; doxifluridine; droloxifene; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; 4-ipomeanol; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor-1 based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras famesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Therapeutic agents useful for treating or preventing an addictive disorder include, but are not limited to, methadone, desipramine, amantadine, fluoxetine, buprenorphine, an opiate agonist, 3-phenoxypyridine, or a serotonin antagonist.

Examples of useful anti-anxiety agents include, but are not limited to, benzodiazepines, such as alprazolam, chlordiazepoxide, clonazepam, clorazepate, diazepam, halazepam, lorazepam, oxazepam, and prazepam; non-benzodiazepine agents, such as buspirone; and tranquilizers, such as barbituates.

Examples of other useful anti-diarrheal agents include, but are not limited to, loperamide, diphenoxylate with atropine, clonidine, octreotide, and cholestyramine.

A 4-Oxadiazolyl-piperidine Compound and the other therapeutic agent can act additively or, in one embodiment, synergistically. In one embodiment, a 4-Oxadiazolyl-piperidine Compound is administered concurrently with another therapeutic agent; for example, a composition comprising both an effective amount of a 4-Oxadiazolyl-piperidine Compound and an effective amount of another therapeutic agent can be administered. Alternatively, a composition comprising an effective amount of a 4-Oxadiazolyl-piperidine Compound and a different composition comprising an effective amount of another therapeutic agent can be concurrently administered. In another embodiment, an effective amount of a 4-Oxadiazolyl-piperidine Compound is administered prior or subsequent to administration of an effective amount of another therapeutic agent so that the benefit of the combination is achieved. In this embodiment, the 4-Oxadiazolyl-piperidine Compound is administered while the other therapeutic agent exerts its therapeutic effect, or the other therapeutic agent is administered while the 4-Oxadiazolyl-piperidine Compound exerts its preventive or therapeutic effect for treating or preventing pain or diarrhea.

A composition of the invention is prepared by a method comprising admixing a 4-Oxadiazolyl-piperidine Compound or a pharmaceutically acceptable salt thereof with a pharmaceutically acceptable carrier or excipient. Admixing can be accomplished using methods known for admixing a compound (or salt) and a pharmaceutically acceptable carrier or excipient. In one embodiment the composition is prepared such that the 4-Oxadiazolyl-piperidine Compound is present in the composition in an effective amount.

The usefulness of the compounds according to the invention in the treatment of the conditions can be demonstrated by binding to the µ ORL-1, δ and K-receptors.

In one embodiment of the invention, the 4-Oxadiazoylyl-piperidine Compounds will have a Ki (nM) of about 300 or less, for binding to the µ-opioid receptors. In another embodiment the compounds will have a Ki (nM) of about 100 or less. In still another embodiment the compounds will have a Ki (nM) of about 10 or less. In yet another embodiment the 4-Oxadiazoylyl-piperidine Compounds will have a Ki (nM) of about 6 or less. In a further embodiment the compounds will have a Ki (nM) of about 2 or less. In still another embodiment the compounds will have a Ki (nM) of about 1 or less. In yet another embodiment the 4-Oxadiazoylyl-piperidine Compounds will have a Ki (nM) of about 0,1 or less.

µ GTP EC₅₀ is the concentration of a compound providing 50% of the maximal response for the compound at a µ receptor. 4-Oxadiazolyl-piperidine Compounds typically having a µ GTP EC₅₀ (nM) of about 5000 or less stimulate µ opioid receptor function. In one embodiment, the 4-Oxadiazolyl-piperidine Compounds will have a µ GTP EC₅₀ (nM) of about 1000 or less. In still another embodiment, the 4-Oxadiazolyl-piperidine Compounds will have a µ GTP EC₅ₒ (nM) of about 100 or less. In still another embodiment, the 4-Oxadiazolyl-piperidine Compounds will have a µ GTP EC₅₀ (nM) of about 20 or less. In still another embodiment, the 4-Oxadiazolyl-piperidine Compounds will have a µ GTP EC₅₀ (nM) of about 10 or less. In still another embodiment, the 4-Oxadiazolyl-piperidine Compounds will have a µ GTP EC₅₀ (nM) of about 8 or less. In still another embodiment, the 4-Oxadiazolyl-piperidine Compounds will have a µ GTP EC₅₀ (nM) of about 2 or less. In still another embodiment, the 4-Oxadiazolyl-piperidine Compounds will have a µ GTP EC₅₀ (nM) of about I or less. In still another embodiment, the 4-Oxadiazolyl-piperidine Compounds will have a µ GTP EC₅₀ (nM) of about 0.1 or less.

µ GTP Emax % is the maximal effect elicited by a compound relative to the effect elicited by [D-Ala2, N-methyl-Phe4, Gly-015]-enkephalin ("DAMGO"), a standard µ agonist. Generally, the µ GTP Emax (%) value measures the efficacy of a compound to treat or prevent pain or diarrhea. Typically the 4-Oxadiazolyl-piperidine Compounds will have a µ GTP Emax (%) of greater than 50%. In one embodiment the 4-Oxadiazolyl-piperidine Compounds will have a µ GTP Emax (%) of greater than 75%. In one embodiment the 4-Oxadiazolyl-piperidine Compounds will have a µ GTP Emax (%) of greater than 88%. In still another embodiment the 4-Oxadiazolyl-piperidine Compounds will have a µ GTP Emax (%) of greater than 100%. In still another embodiment the 4-Oxadiazolyl-piperidine Compounds will have a µ GTP Emax (%) of greater than 110%. In one embodiment the 4-Oxadiazolyl-piperidine Compounds will have a µ GTP Emax (%) of greater than 115%.

Typically, the 4-Oxadiazolyl-piperidine Compounds will have a Ki (nM) of about 10,000 or less for ORL-1 receptors. In one embodiment, the 4-Oxadiazolyl-piperidine Compounds will have a Ki (nM) of about 2000 or less. In one embodiment, the 4-Oxadiazolyl-piperidine Compounds will have a Ki (nM) of about 1500 or less. In another embodiment, the 4-Oxadiazolyl-piperidine Compounds will have a Ki (nM) of about 1000 or less. In one embodiment, the 4-Oxadiazolyl-piperidine Compounds will have a Ki (nM) of about 700 or less. In still another embodiment, the 4-Oxadiazolyl-piperidine Compounds will have a Ki (nM) of about 100 or less. In still another embodiment, the 4-Oxadiazolyl-piperidine Compounds will have a Ki (nM) of about 10 or less.

ORL-1 GTP EC₅₀ is the concentration of a compound providing 50% of the maximal response for the compound at an ORL-1 receptor. 4-Oxadiazolyl-piperidine Compounds having an ORL-1 GTP EC₅₀ (nM) of about 10,000 or less stimulate ORL-1 opioid-receptor function. In one embodiment, the 4-Oxadiazolyl-piperidine Compounds will have an ORL-1 GTP EC₅₀ (nM) of about 1000 or less. In still another embodiment, the 4-Oxadiazolyl-piperidine Compounds will have an ORL-1 GTP EC₅₀ (nM) of about 100 or less. In still another embodiment, the 4-Oxadiazolyl-piperidine Compounds will have an ORL-I GTP EC₅₀ (nM) of about 50 or less. In still another embodiment, the 4-Oxadiazolyl-piperidine Compounds will have an ORL-1 GTP EC₅₀ (nM) of about 10 or less.

ORL-1 GTP Emax % is the maximal effect elicited by a compound relative to the effect elicited by nociceptin, a standard ORL-1 agonist. Generally, the ORL-1 GTP Emax (%) value measures the efficacy of a compound to treat or prevent pain or diarrhea. Typically the 4-Oxadiazolyl-piperidine Compounds have an ORL-1 GTP Emax (%) of greater than 50%. In one embodiment the 4-Oxadiazolyl-piperidine Compounds will have an ORL-1 GTP Emax (%) of greater than 75%. In still another embodiment the 4-Oxadiazolyl-piperidine Compounds will have an ORL-1 GTP Emax (%) of greater than 88%. In still another embodiment the 4-Oxadiazolyl-piperidine Compounds will have an ORL-1 GTP Emax (%) of greater than 100%.

Typically, the 4-Oxadiazolyl-piperidine Compounds will have a Ki (nM) of about 10,000 or less for δ receptors. In one embodiment, the 4-Oxadiazolyl-piperidine Compounds will have a Ki (nM) of about 4000 or less. In one embodiment, the 4-Oxadiazolyl-piperidine Compounds will have a Ki (nM) of about 2500 or less. In another embodiment, the 4-Oxadiazolyl-piperidine Compounds will have a Ki (nM) of about 1000 or less. In one embodiment, the 4-Oxadiazolyl-piperidine Compounds will have a Ki (nM) of about 500 or less. In still another embodiment, the 4-Oxadiazolyl-pipefidine Compounds will have a Ki (nM) of about 350 or less. In still another embodiment, the 4-Oxadiazolyl-piperidine Compounds will have a Ki (nM) of about 250 or less. In still another embodiment, the 4-Oxadiazolyl-piperidine Compounds will have a Ki (nM) of about 100 or less. In still another embodiment, the 4-Oxadiazolyl-piperidine Compounds will have a Ki (nM) of about 10 or less.

δ GTP EC₅₀ is the concentration of a compound providing 50% of the maximal response for the compound at an δ receptor. 4-Oxadiazolyl-piperidine Compounds having an δ GTP EC₅₀ (nM) of about 10,000 or less stimulate δ opioid-receptor function. In one embodiment, the 4-Oxadiazolyl-piperidine Compounds will have an δ GTP EC₅₀ (nM) of about 1000 or less. In still another embodiment, the 4-Oxadiazolyl-piperidine Compounds will have an δ GTP EC₅₀ (nM) of about 100 or less. In still another embodiment, the 4-Oxadiazolyl-piperidine Compounds will have an δ GTP EC₅₀ (nM) of about 90 or less. In still another embodiment, the 4-Oxadiazolyl-piperidine Compounds will have an δ GTP EC₅₀ (nM) of about 50 or less. In still another embodiment, the 4-Oxadiazolyl-piperidine Compounds will have an δ GTP EC₅₀ (nM) of about 25 or less. In still another embodiment, the 4-Oxadiazolyl-piperidine Compounds will have an δ GTP EC₅₀ (nM) of about 10 or less.

δ GTP Emax % is the maximal effect elicited by a compound relative to the effect elicited by met-enkephalin. In one embodiment the 4-Oxadiazolyl-piperidine Compounds have an δ GTP Emax (%) of greater than 50%. In one embodiment the 4-Oxadiazolyl-piperidine Compounds will have an δ GTP Emax (%) of greater than 75%. In still another embodiment the 4-Oxadiazolyl-piperidine Compounds will have an δ GTP Emax (%) of greater than 90%. In still another embodiment the 4-Oxadiazolyl-piperidine Compounds will have an δ GTP Emax (%) of greater than 100%. In still another embodiment the 4-Oxadiazolyl-piperidine Compounds will have an δ GTP Emax (%) of greater than 110%.

In one embodiment the 4-Oxadiazolyl-piperidine Compounds will have a Ki (nM) of about 10,000 or less for κ receptors. In one embodiment, the 4-Oxadiazolyl-piperidine Compounds will have a Ki (nM) of about 5000 or less. In one embodiment, the 4-Oxadiazolyl-piperidine Compounds will have a Ki (nM) of about 1000 or less. In another embodiment, the 4-Oxadiazolyl-piperidine Compounds will have a Ki (nM) of about 500 or less. In one embodiment, the 4-Oxadiazolyl-piperidine Compounds will have a Ki (nM) of about 400 or less. In still another embodiment, the 4-Oxadiazolyl-piperidine Compounds will have a Ki (nM) of about 200 or less. In still another embodiment, the 4-Oxadiazolyl-piperidine Compounds will have a Ki (nM) of about 100 or less. In still another embodiment, the 4-Oxadiazolyl-piperidine Compounds will have a Ki (nM) of about 50 or less. In still another embodiment, the 4-Oxadiazolyl-piperidine Compounds will have a Ki (nM) of about 10 or less. In still another embodiment, the 4-Oxadiazolyl-pipen'dine Compounds will have a Ki (nM) of about I or less.

κ GTP EC₅₀ is the concentration of a compound providing 50% of the maximal response for the compound at an κ receptor. In one embodiment the 4-Oxadiazolyl-piperidine Compounds will have a κ GTP EC₅₀ (nM) of about 10,000 or less. In one embodiment, the 4-Oxadiazolyl-piperidine Compounds will have a κ GTP EC₅₀ (nM) of about 5000 or less. In one embodiment, the 4-Oxadiazolyl-piperidine Compounds will have a κ GTP EC₅₀ (nM) of about 2000 or less.In one embodiment, the 4-Oxadiazolyl-piperidine Compounds will have a κ GTP EC₅₀ (nM) of about 1000 or less. In still another embodiment, the 4-Oxadiazolyl-piperidine Compounds will have a κ GTP EC₅₀ (nM) of about 100 or less. In still another embodiment, the 4-Oxadiazolyl-piperidine Compounds will have a κ GTP EC₅₀ (nM) of about 50 or less. In still another embodiment, the 4-Oxadiazolyl-piperidine Compounds will have a κ GTP EC₅₀ (nM) of about 25 or less. In still another embodiment, the 4-Oxadiazolyl-piperidine Compounds will have a κ GTP EC₅₀ (nM) of about 10 or less.

κ GTP Emax % is the maximal effect elicited by a compound relative to the effect elicited by U69,593. In one embodiment the 4-Oxadiazolyl-piperidine Compounds have an κ GTP Emax (%) of greater than 50%. In one embodiment the 4-Oxadiazolyl-piperidine Compounds will have an κ GTP Emax (%) of greater than 75%. In still another embodiment the 4-Oxadiazolyl-piperidine Compounds will have an κ GTP Emax (%) of greater than 90%. In still another embodiment the 4-Oxadiazolyl-piperidine Compounds will have an κ GTP Emax (%) of greater than 100%.

### 4.4.2 Kits

The invention further encompasses kits that can simplify the administration of a 4-Oxadiazolyl-piperidine Compound to an animal.

A typical kit of the invention comprises a unit dosage form of a 4-Oxadiazolyl-piperidine Compound. In one embodiment, the unit dosage form is provided in a container, in one embodiment a sterile container, containing an effective amount of a 4-Oxadiazolyl-piperidine Compound and a pharmaceutically acceptable carrier or excipient. The kit can further comprise a label or printed instructions instructing the use of the 4-Oxadiazolyl-piperidine Compound to treat or prevent pain or diarrhea. The kit can also further comprise a unit dosage form of another therapeutic agent, for example, provided in a container containing an effective amount of the other therapeutic agent. In one embodiment, the kit comprises a container containing an effective amount of a 4-Oxadiazolyl-pipefidine Compound and an effective amount of another therapeutic agent. Examples of other therapeutic agents include, but are not limited to, those listed above.

Kits of the invention can further comprise a device useful for administering the unit dosage form. Examples of such devices include, but are not limited to, syringes, drip bags, patches, enema bags, and inhalers.

The following examples are set forth to assist in understanding the invention and should not, of course, be construed as specifically limiting the invention described and claimed herein. Such variations of the invention, including the substitution of all equivalents now known or later developed, which would be within the purview of those skilled in the art, and changes in formulation or minor changes in experimental design, are to be considered to fall within the scope of the invention incorporated herein.

### 5. Examples

The following examples relate to the synthesis of illustrative 4-Oxadiazolyl-pipefidine Compounds of the present invention as well as to the synthesis of intermediates useful in the synthesis of illustrative 4-Oxadiazolyl-piperidine Compounds of the present invention.

### 5.1 Example 1: Synthesis of Compound 16

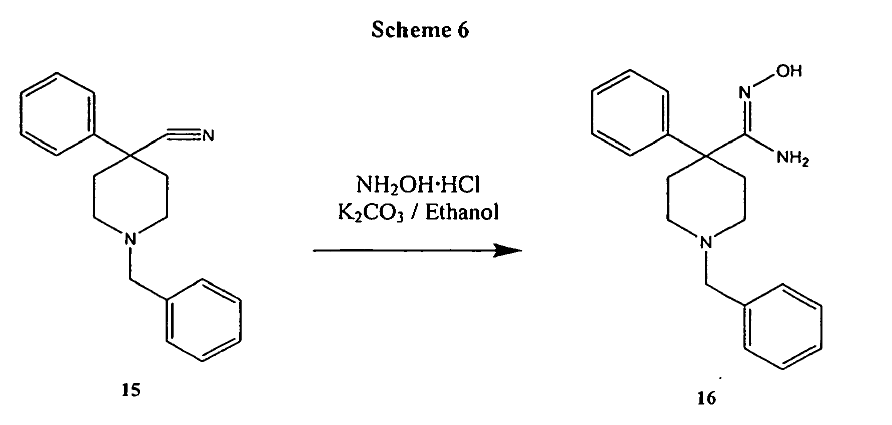

To a suspension of 50 g (719.53 mmol) of hydroxylamine hydrochloride (Aldrich; St. Louis, MO) in ethanol (500 ml) at 0 °C was added 99.0 g (719.53 mmol) of potassium carbonate. The resulting suspension was stirred at 0 °C for 15 min and 40.0 g (144.73 mmol) of the nitrile 15 (Acros Organics, Morris Plains, NJ) was added. The resulting reaction mixture was stirred at room temperature for 0.5 h and then refluxed for 12 h. After this period, the reaction mixture was allowed to cool to room temperature and the inorganic salts were filtered. The filtrate was concentrated with a rotary evaporator and the residue was purified by flash chromatography using a gradient of ethyl acetate / hexane as eluent to give 14.1 g of the amidoxime 16: *mlz* 310, ¹H NMR (CDCl₃) 8 9.07 (bs, 1 H), 7.47-7.18 (m, 10 H + CHCl₃), 4.23 (s, 2 H), 3.46 (m, 2 H), 2.62-2.46 (m, 4 H), 2.41-2.30 (m, 2 H), 2.24-2.11 (m, 2 H).

### 5.2 Example 2: Synthesis of Compound 18

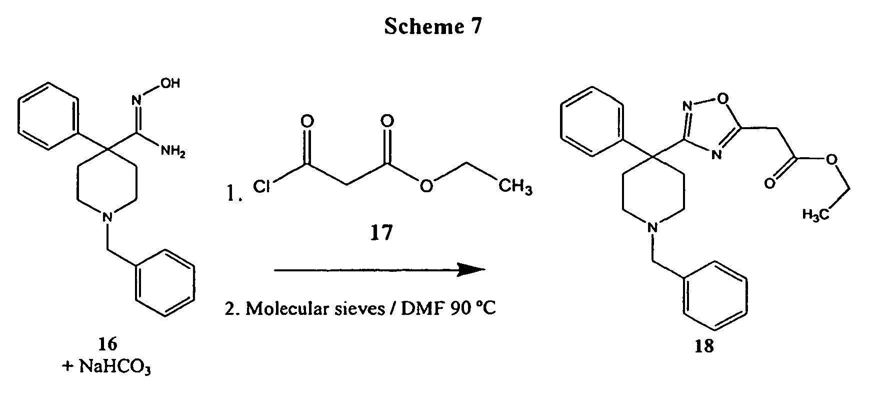

The amidoxime 16 (11.02 g, 55.64 mmol) and NaHC0₃ (6.58 g, 78.41 mmol) were suspended in THF (500 ml) under nitrogen atmosphere. The mixture was cooled to 0 °C and ethyl malonyl chloride 17 (Aldrich) was added dropwise over 20 min (5.6 ml, 6.44 g, 42.77 mmol). The resulting reaction mixture was stirred at 0 °C for 30 min and refluxed for 2 h. After this period, the reaction mixture was allowed to cool to room temperature and the inorganic salts were filtered. The excess acid chloride in the filtrate was quenched with 50 ml of methanol and the resulting solution was concentrated with rotary evaporator. The residue was redissolved in DMF (100 ml) and was heated to 90 °C for 10h in the presence of 2.0 g of 4 Å molecular sieves. The reaction mixture was cooled to room temperature and volatiles were removed *in vacuo* to give a crude sample that was purified by flash chromatography using a gradient of ethyl acetate / hexane as eluent to give 2.5 g of the oxadiazole 18: *m*/*z* 406, ¹H NMR (CDCl₃) δ 7.41-7.16 (m, 10 H + CHCl₃), 4.2 (q, J = 7.23, 2 H), 3.92 (s, 2 H), 3.47-3.43 (m, 2 H), 2.86-2.75 (m, 2 H), 2.74-2.64 (m, 2 H), 2.35-2.17 (m, 4 H), 1.23 (t, *J* = 7.02, 3 H).

### 5.3 Example 3: Synthesis of Compound 20

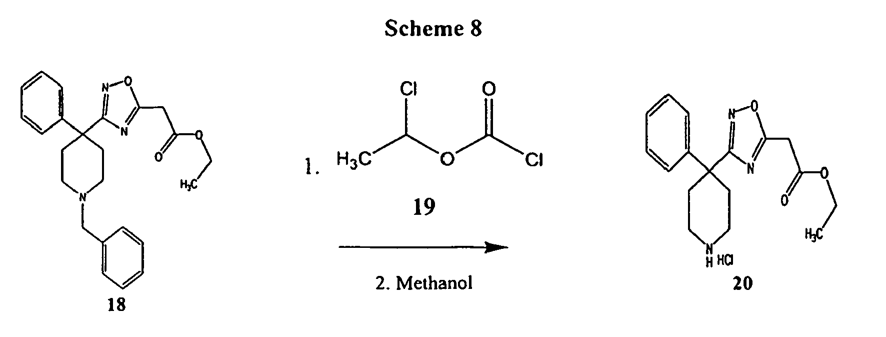

The piperidinyloxadiazole 18 (0.735 g, 1.82 mmol) was dissolved in 100 ml of dichloroethane and 0.3 ml (2.18 mmol) of triethylamine was added under nitrogen atmosphere. The resulting solution was cooled to 0 °C and 1-chloroethyl chloroformate 19 (Aldrich) was added (0.24 ml, 2.18 mmol). The resulting reaction mixture was stirred at 0 °C for 10 min and refluxed for 4 h. After this period, the volatiles were removed with a rotary evaporator and then the resulting residue was redissolved in 100 ml of methanol. The resulting solution was stirred at reflux for 2 h and the volatiles were removed using a rotary evaporator. The residue was redissolved in DCM and concentrated again by rotary evaporator to give 0.5 g of 20: m/z 316.

### 5.4 Example 4: Synthesis of Compound 22

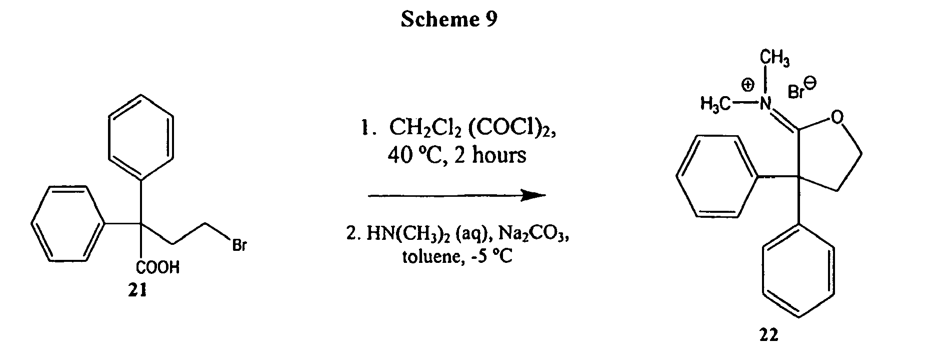

A 50 g (156.7 mmol) portion of 4-Bromo-2,2-diphenyl butyric acid 21 (Aldrich) was suspended in dichloromethane (250 mL). Oxalyl chloride (14.4 ml, 164.5 mmol) was added and the mixture heated under reflux under argon for 2h. The reaction mixture was cooled to room temperature and volatiles were then removed *in vacuo* to give the crude acid chloride, which was used immediately. Sodium carbonate (19.9 g, 188.04 mmol) was dissolved in water (200 mL) and the solution cooled to -5 °C (ice-acetone). Aqueous dimethylamine 40% w/w 7.9 M (24 mL, 188.04 mmol) was added, followed by toluene (200 mL). The acid chloride in toluene (250 mL) was added over 15 min keeping the temperature below 0 °C during the addition, and the resulting mixture stirred for an additional 1 h at this temperature. The organic layer was separated (discarded to remove impurities) and the aqueous layer extracted with dichloromethane (5 x 500 mL), dried (MgS0₄) and the solvent evaporated *in vacuo* to leave an off-white solid which was left on the rotary evaporator at 50 °C for 20 min. The solid was triturated with ethyl acetate (250 mL) to give 22 (37.5 g, 69.4%) as a white solid: ¹H NMR (CDCl₃) δ 7.56-7.36 (10H, m), 4.86 (2H, t, J = 7.0 Hz), 3.82 (3H, s), 3.47 (2H, t, J = 7.0 Hz), 2.96 (3H, s).

### 5.5 Example 5: Synthesis of Compound 23

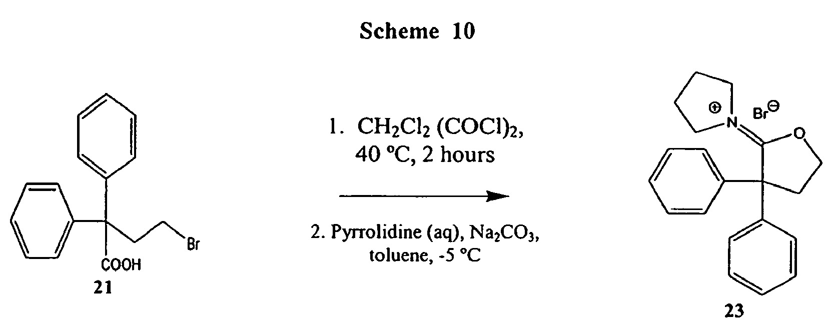

To a suspension of 100 g (313.293 mmol) of 4-Bromo-2,2-diphenyl butyric acid 21 (Aldrich) in dichloromethane (300 mL) was added oxalyl chloride (328.958 mmol *i.e.* 164.48 ml of a 2M solution in dichloromethane (Aldrich)) and the mixture heated under reflux under argon for 18 h. The reaction mixture was cooled to room temperature and volatiles were then removed *in vacuo* to give the crude acid chloride, which was redissolved in 400 ml of toluene. To a suspension of pyrrolidine (26.74 g, 375.95 mmol) in water (400 ml) was added sodium carbonate (39.84 g, 375.95 mmol), the resulting solution was cooled to -5 °C (ice-acetone). The acid chloride in toluene (400 mL) was added over 40 min keeping the temperature below 0 °C during the addition, and the resulting mixture stirred for an additional 1 h at this temperature. The organic layer was decanted and the aqueous layer extracted with dichloromethane (3 x 500 mL), dried (MgS0₄) and the solvent evaporated *in vacuo* to leave an off-white solid which was left on the rotary evaporator at 50 °C for 20 min. The solid was triturated with ethyl acetate (250 mL) to give 23 (74.5 g, 63 %) as a white solid: ¹H NMR (CDCl₃) δ 7.57-7.50 (m, 4 H), 7.48-7.42 (m, 6 H), 4.92-4.85 (m, 2 H), 4.39-4.32 (m, 2 H), 3.55-3.47 (m, 2 H), 2.91-2.83 (m, 2 H), 2.19-2.08 (m, 2 H), 2.06-1.94 (m, 2 H).

### 5.6 Example 6: Synthesis of Compound AFL

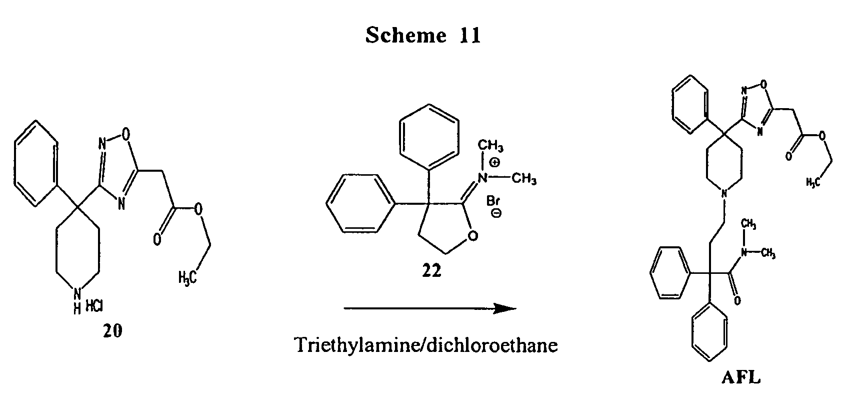

A 0.3 ml (4.36 mmol) portion of triethylamine was added to a 1,2-dichloroethane (50 ml) solution of 20 (0.63 g, 1.82 mmol) at room temperature under nitrogen atmosphere. The resulting solution was cooled to 0 °C and 22 (0.75 g, 2.18 mmol) was added. The reaction mixture was stirred at 0 °C for 2 h and room temperature for 8 h. After this period, the volatiles were removed by rotary evaporator and the residue was purified by flash chromatography using a gradient of ethyl acetate / hexane as eluent to give 0.7 g of AFL: *mlz* 581, ¹H NMR (CD₃0D) δ 7.41-7.15 (m, 15 H), 4.11-4.02 (m, 2 H), 3.99-3.92 (m, 2 H), 3.51-3.33 (m, 2 H), 2.94-2.74 (m, 7 H), 2.63-2.45 (m, 4 H), 2.33-2.16 (m, 5 H), 1.15-1.09 (m, 3 H).

### 5.7 Example 7: Synthesis of Compound AGE

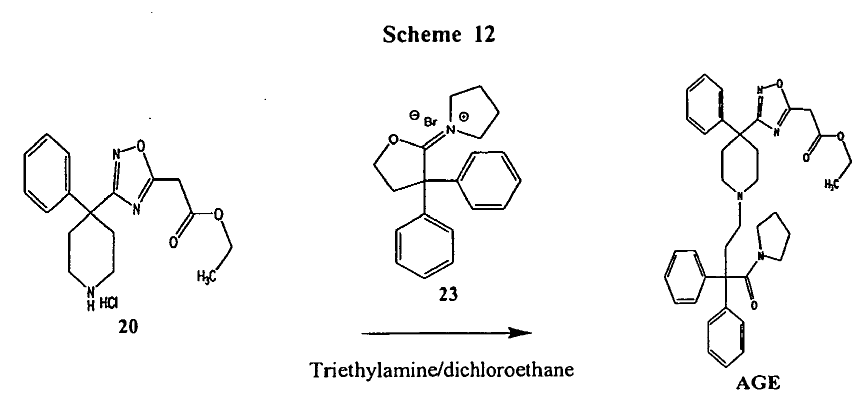

A 2.88 ml (20.72 mmol) portion of triethylamine was added to a 1,2-dichloroethane (50 ml) solution of 20 (1.75 g, 5.1 mmol) at room temperature under nitrogen atmosphere. The resulting solution was cooled to 0 °C and 23 (2.2 g, 6.0 mmol) was added. The reaction mixture was stirred at 0 °C for 2 h and room temperature for 8 h. After this period, the volatiles were removed by rotary evaporator and the residue was purified by flash chromatography using a gradient of ethyl acetate / hexane as eluent to give 1.2 g of **AGE**: m/z 607, is NMR (CD₃0D) δ 7.34-7.00 (m, 15), 3.96 (q, J = 7.23, 2 H), 3.87 (bs, 2 H), 3.43-3.29 (m, 4 H), 2.84-2.65 (m, 4 H), 2.57-2.39 (m, 4 H), 2.26-2.09 (m, 4 H), 1.56-1.45 (m, 2 H), 1.43-1.29 (m, 2 H), 1.00 (t, J = 7.23, 3 H).

### 5.8 Example 8: Synthesis of Compound 24

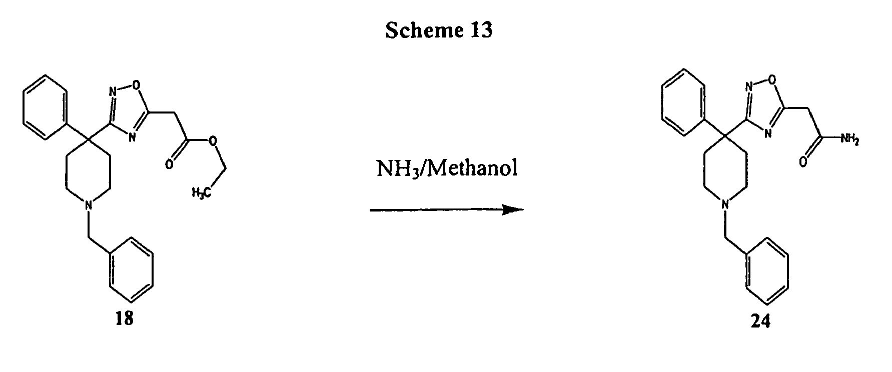

A 0.1 g (0.25 mmol) sample of 18 was dissolved in 8.0 ml of ammonia in methanol (7N, Aldrich). The resulting solution was stirred at reflux for 40 min. The reaction mixture was cooled to room temperature and volatiles were then removed *in vacuo* to give a crude sample. The crude was purified by preparative TLC using 2 % methanol in DCM to give 50 mg of 24 as white solid: m/z 377, ¹H NMR (CDCl₃) δ 7.41-7.18 (m, 10 H + CHCl₃) 7.02 (bs, 1 H), 5.53 (bs, 1 H), 3.85 (s, 2 H), 3.46 (bs, 2 H), 2.86-2.76 (m, 2 H), 2.73-2.62 (m, 2 H), 2.39-2.15 (m, 4 H).

### 5.9 Example 9: Synthesis of Compound AFE

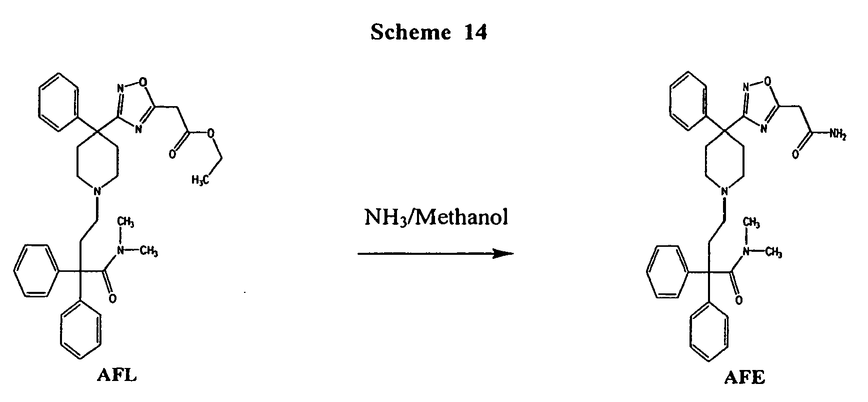

A 0.31 g (0.55 mmol) sample of AFL was dissolved in 20.0 ml of ammonia in methanol (7N, Aldrich). The resulting solution was stirred at reflux for 40 min. The reaction mixture was cooled to room temperature and volatiles were then removed *in vacuo* to give a crude sample. The crude was purified by flash chromatography using a gradient of ethyl acetate / hexane as an eluent to give 0.25 g of AFE as a white solid: *m*/*z* 552, ¹H NMR (CDCl₃) δ 7.46-7.17 (m, 15 H + CHCl₃), 6.93 (bs, 1 H), 5.80 (bs, 1 H), 3.90 (bs, 2 H), 3.66-3.55 (m, 2 H), 2.98 (bs, 3 H), 2.91-2.64 (m, 6 H), 2.62-2.47 (m, 4 H), 2.29 (bs, 3 H).

### 5.10 Example 10: Synthesis of Compound AFX

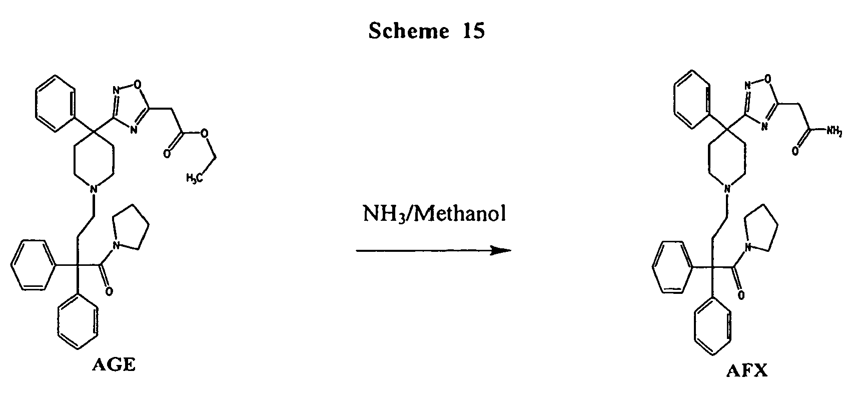

A 0.30 g (0.50 mmol) sample of **AGE** was dissolved in 20.0 ml of ammonia in methanol (7N, Aldrich). The resulting solution was stirred at reflux for 40 min. The reaction mixture was cooled to room temperature and volatiles were then removed *in vacuo* to give a crude sample. The crude was purified by flash chromatography using a gradient of ethyl acetate / hexane as an eluent to give 0.251 g of AFX as a white solid: *m*/*z* 578, ¹H NMR (CD₃0D) δ 7.44-7.17 (m, 15 H), 3.84 (bs, 2 H), 3.55-3.37 (m, 4 H), 2.99-2.81 (m, 4 H), 2.68-2.54 (m, 4 H), 2.39-2.21 (m, 4 H), 1.67-1.57 (m, 2 H), 1.54-1.43 (m, 2 H).

### 5.11 Example 11: µ- and ORL-I-Receptor-Bindine Affinity Assays

The following example will demonstrate that 4-Oxadiazolyl-piperidine Compounds bind to µ- or ORL-1-receptors and, accordingly, are useful for treating or preventing pain or diarrhea.

### 5.11 Materials and Methods

### ORL-1 Receptor Membrane Preparation

All reagents are obtained from Sigma (St. Louis, MO) unless noted otherwise. Membranes from recombinant HEK-293 cells expressing the human opioid receptor-like (ORL-1 ) receptor (Perkin Elmer, Boston, MA) are prepared by lysing cells in ice-cold hypotonic buffer (2.5 mM MgCl₂, 50 mM HEPES, pH 7.4) (10 mL/10 cm dish), followed by homogenization with a tissue grinder/teflon pestle. Membranes are collected by centrifugation at 30,000 x g for 15 min at 4 °C, and pellets are resuspended in hypotonic buffer to a final concentration of 1-3 mg/mL. Protein concentrations are determined using the BioRad (Hercules, CA) protein assay reagent with bovine serum albumen as standard. Aliquots of the ORL- I receptor membranes are stored at -80 °C.

### µ- and ORL-1 -Receptor-Binding-Assay Procedures

Radioligand dose-displacement binding assays for ORL-1 and µ receptors use 0.1 nM [³H]-nociceptin or 0.2 nM [³H]-diprenorphine (NEN, Boston, MA), respectively, with 5-20 mg membrane protein/well in a final volume of 500 ml binding buffer (10 mM MgCl₂, 1 mM EDTA, 5% DMSO, 50 mM HEPES, pH 7.4). Reactions are carried out in the absence or presence of increasing concentrations of unlabeled nociceptin (American Peptide Company, Sunnyvale, CA) or naloxone, for ORL-1 and µ, respectively. All reactions are conducted in 96-deep well polypropylene plates for 1-2 h at room temperature. Binding reactions are terminated by rapid filtration onto 96-well Unifilter GF/C filter plates (Packard, Meriden, CT) presoaked in 0.5% polyethylenimine using a 96-well tissue harvester (Brandel, Gaithersburg, MD) followed by three filtration washes with 500 µL of ice-cold binding buffer. Filter plates are subsequently dried at 50 °C for 2-3 h. BetaScint scintillation cocktail (Wallac, Turku, Finland) is added (50 µl/well), and plates are counted using a Packard Top-Count for 1 min/well. The data are analyzed using the one-site competition curve fitting functions in GraphPad PRISM v. 3.0 (San Diego, CA).

### δ Opioid receptor binding assay procedure

Radioligand dose-displacement assays used 0.2 nM [³H]-Naltrindole (NEN; 33.0 Ci/mmole) with 10-20 µg membrane protein (recombinant delta opioid receptor expressend in CHO-K 1 cells; Perkin Elmer) in a final volume of 500 µl binding buffer (5 mM MgCl₂, 5% DMSO, 50 mM Trizma base, pH 7.4). Non-specific binding was determined in the presence of 25 µM unlabeled naloxone. All reactions were performed in 96-deep well polypropylene plates for I h at room temperature. Binding reactions were determined by rapid filtration onto 96-well Unifilter GF/C filter plates (Packard) presoaked in 0.5% polyethylenimine (Sigma). Harvesting was performed using a 96-well tissue harvester (Packard) followed by five filtration washes with 500 µl ice-cold binding buffer. Filter plates were subsequently dried at 50 °C for 1-2 hours. Fifty µl/well scintillation cocktail (MicroScint, Packard) was added and plates were counted in a Packard Top-Count for 1 min/well.

### κ Opioid receptor binding assay procedure

Membranes from recombinant HEK-293 cells expressing the human kappa opioid receptor (kappa) (cloned in house) were prepared by lysing cells in ice cold hypotonic buffer (2.5 mM MgCl₂, 50 mM HEPES, pH 7.4) (10 ml/10 cm dish) followed by homogenization with a tissue grinder/teflon pestle. Membranes were collected by centrifugation at 30,000 x g for 15 min at 4 °C and pellets resuspended in hypotonic buffer to a final concentration of 1-3 mg/ml. Protein concentrations were determined using the BioRad protein assay reagent with bovine serum albumen as standard. Aliquots of kappa receptor membranes were stored at -80 °C.

Radioligand dose displacement assays used 0.4-0.8 nM [³H]-U69,593 (NEN; 40 Ci/mmole) with 10-20 µg membrane protein (recombinant kappa opioid receptor expressed in HEK 293 cells; in-house prep) in a final volume of 200 µl binding buffer (5% DMSO, 50 mM Trizma base, pH 7.4). Non-specific binding was determined in the presence of 10 µM unlabeled naloxone or U69,593. All reactions were performed in 96-well polypropylene plates for 1 h at room temperature. Binding reactions were determined by rapid filtration onto 96-well Unifilter GF/C filter plates (Packard) presoaked in 0.5% polyethylenimine (Sigma). Harvesting was performed using a 96-well tissue harvester (Packard) followed by five filtration washes with 200 µl ice-cold binding buffer. Filter plates were subsequently dried at 50 °C for 1-2 hours. Fifty µl/well scintillation cocktail (MicroScint; Packard) was added and plates were counted in a Packard Top-Count for 1 min/well.

### 5.11.2 µ-Receptor-Binding Data

Generally, the lower the Ki value, the more effective the 4-Oxadiazolyl-piperidine Compounds will be at treating or preventing pain or diarrhea. Typically, the 4-Oxadiazolyl-piperidine Compounds will have a Ki (nM) of about 300 or less for binding to µ-opioid receptors. In one embodiment, the 4-Oxadiazolyl-piperidine compounds will have a Ki (nM) of about 100 or less. In another embodiment, the 4-Oxadiazolyl-piperidine Compounds of the present invention will have a Ki (nM) of about 10 or less. In still another embodiment, the 4-Oxadiazolyl-piperidine Compounds will have a Ki (nM) of about 1 or less. In still another embodiment, the 4-Oxadiazolyl-piperidine Compounds will have a Ki (nM) of about 0.1 or less. Compounds **AFL, AGE, AFE,** and **AFX** (i.e illustrative 4-Oxadiazolyl-piperidine Compounds), have, respectively, a Ki (nM) of 1. 1, 1.3, 5.9, and 4.8 for binding to µ-opioid receptors.

### 5.11.3 ORL-1-Receptor-Binding Data

Typically, the 4-Oxadiazolyl-piperidine Compounds will have a Ki (nM) of about 10,000 or less for ORL-1 receptors. ln one embodiment, the 4-Oxadiazolyl-piperidine Compounds will have a Ki (nM) of about 2000 or less. In another embodiment, the 4-Oxadiazolyl-piperidine Compounds will have a Ki (nM) of about 1000 or less. In still another embodiment, the 4-Oxadiazolyl-piperidine Compounds will have a Ki (nM) of about 100 or less. In still another embodiment, the 4-Oxadiazolyl-piperidine Compounds will have a Ki (nM) of about 10 or less.

### δ-Receptor-Binding data

Typically, the 4-Oxadiazolyl-piperidine Compounds will have a Ki (nM) of about 10,000 or less for δ receptors. In one embodiment, the 4-Oxadiazolyl-piperidine Compounds will have a Ki (nM) of about 4000 or less. In one embodiment, the 4-Oxadiazolyl-piperidine Compounds will have a Ki (nM) of about 2500 or less. In another embodiment, the 4-Oxadiazolyl-piperidine Compounds will have a Ki (nM) of about 1000 or less. In one embodiment, the 4-Oxadiazolyl-piperidine Compounds will have a Ki (nM) of about 500 or less. In still another embodiment, the 4-Oxadiazolyl-piperidine Compounds will have a Ki (nM) of about 350 or less. In still another embodiment, the 4-Oxadiazolyl-piperidine Compounds will have a Ki (nM) of about 250 or less. In still another embodiment, the 4-0xadiazolyl-piperidine Compounds will have a Ki (nM) of about 100 or less. In still another embodiment, the 4-Oxadiazolyl-piperidine Compounds will have a Ki (nM) of about 10 or less.

### κ-Receptor Binding data

Typically, the 4-Oxadiazolyl-pipefidine Compounds will have a Ki (nM) of about 10,000 or less for κ receptors. In one embodiment, the 4-Oxadiazolyl-piperidine Compounds will have a Ki (nM) of about 5000 or less. In one embodiment, the 4-Oxadiazolyl-piperidine Compounds will have a Ki (nM) of about 1000 or less. In another embodiment, the 4-Oxadiazolyl-piperidine Compounds will have a Ki (nM) of about 500 or less. In one embodiment, the 4-Oxadiazolyl-piperidine Compounds will have a Ki (nM) of about 400 or less. In still another embodiment, the 4-Oxadiazolyl-piperidine Compounds will have a Ki (nM) of about 200 or less. In still another embodiment, the 4-Oxadiazolyl-piperidine Compounds will have a Ki (nM) of about 100 or less. In still another embodiment, the 4-Oxadiazolyl-piperidine Compounds will have a Ki (nM) of about 50 or less. In still another embodiment, the 4-Oxadiazolyl-piperidine Compounds will have a Ki (nM) of about 10 or less.

### 5.12 Example 12: µ- and ORL-1-Opioid Receptor GTPyS Functional Activity

The following example will demonstrate that 4-Oxadiazolyl-pipefidine Compounds stimulate µ- or ORL-1-receptor function as well as 8 and K-receptor function and, accordingly, will be useful for treating or preventing pain or diarrhea.

### 5.12.1 Materials and Methods

[³⁵S]GTPyS functional assays are conducted using freshly thawed ORL-1 or µ-receptor membranes, as appropriate. Assay reactions are prepared by sequentially adding the following reagents to binding buffer (100 mM NaCl, 10 mM MgCl₂, 20 mM HEPES, pH 7.4) on ice (final concentrations indicated): membrane protein (0.066 mg/mL for ORL-1 receptor and 0.026 mg/mL for µ-receptor), saponin (10 mg/ml), GDP (3 mM) and [³⁵S]GTPyS (0.20 nM; NEN). Aliquots of the prepared membrane solution (190 µL/well) are transferred to 96-shallow well polypropylene plates containing 10 µL of 20x concentrated stock solutions of the agonist nociceptin prepared in dimethyl sulfoxide ("DMSO"). Plates are incubated for 30 min at room temperature with shaking. Reactions are terminated by rapid filtration onto 96-well Unifilter GF/B filter plates (Packard, Meriden, CT) using a 96-well tissue harvester (Brandel, Gaithersburg, MD) followed by three filtration washes with 200 µL of ice-cold binding buffer (10 mM NaH₂P0₄, 10 mM Na₂HP0₄, pH 7.4). Filter plates are subsequently dried at 50 °C for 2-3 h. BetaScint scintillation cocktail (Wallac, Turku, Finland) is added (50 µL/well) and plates are counted using a Packard Top-Count for 1 min/well. Data are analyzed using the sigmoidal dose-response curve fitting functions in GraphPad PRISM, v. 3.0.

The κ and δ opioid receptor functional [³⁵S]GTPγS binding assays were conducted as follows. Kappa or delta opioid receptor membrane solution was prepared by sequentially adding final concentrationsof 0.026 µg/µl membrane protein (kappa: in-house, delta: Perkin Elmer), 10 µg/ml saponin, 3 µM GDP and 0.20 nM [³⁵S]GTPγS to binding buffer (100 mM NaCl, 10 mM MgCl₂, 20 mM HEPES, pH 7.4) on ice. The prepared membrane solution (190 µl/well) was transferred to 96-shallow well polypropylene plates containing 10 µl of 20x concentrated stock solutions of agonist prepared in DMSO. Plates were incubated for 30 min at room temperature with shaking. Reactions were terminated by rapid filtration onto 96-well tissue harvester (Packard) and followed by three filtration washes with 200 µl ice-cold binding buffer (10 mM NaH₂PO₄), 10 mM Na₂HP0₄, pH 7.4). Filter plates were subsequently dried at 50 °C for 2-3 hours. Fifty µl/well scintillation cocktail (MicroScint20, Packard) was added and plates were counted in a Packard Top-Count for 1 min/well.

### 5.12.2 µ-Receptor Function Data

µ GTP EC₅₀ is the concentration of a compound providing 50% of the maximal response for the compound at a µ receptor. 4-Oxadiazolyl-piperidine Compounds typically having a µ GTP EC₅₀ (nM) of about 5000 or less stimulate µ opioid receptor function. In one embodiment, the 4-Oxadiazolyl-piperidine Compounds will have a µ GTP EC₅₀ (nM) of about 1000 or less. In still another embodiment, the 4-Oxadiazolyl-piperidine Compounds will have a µ GTP EC₅₀ (nM) of about 100 or less. In still another embodiment, the 4-0xadiazolyl-piperidine Compounds will have a µ GTP EC₅₀ (nM) of about 10 or less. In still another embodiment, the 4-Oxadiazolyl-piperidine Compounds will have a µ GTP EC₅₀ (nM) of about I or less. In still another embodiment, the 4-Oxadiazolyl-piperidine Compounds will have a µ GTP EC₅₀ (nM) of about 0.1 or less.

µ GTP Emax % is the maximal effect elicited by a compound relative to the effect elicited by [D-Ala2, N-methyl-Phe4, Gly-o15]-enkephalin ("DAMGO"), a standard µ agonist. Generally, the µ GTP Emax (%) value measures the efficacy of a compound to treat or prevent pain or diarrhea. Typically the 4-Oxadiazolyl-piperidine Compounds will have a µ GTP Emax (%) of greater than 50%. In one embodiment the 4-Oxadiazolyl-piperidine Compounds will have a µ GTP Emax (%) of greater than 75%. In still another embodiment the 4-Oxadiazolyl-piperidine Compounds will have a µ GTP Emax (%) of greater than 88%. In still another embodiment the 4-Oxadiazolyl-piperidine Compounds will have a µ GTP Emax (%) of greater than 100%.

### 5.12.3 ORL-1-Receptor Function Data

ORL-1 GTP EC₅₀ is the concentration of a compound providing 50% of the maximal response for the compound at an ORL-1 receptor. 4-Oxadiazolyl-piperidine Compounds having an ORL-1 GTP EC₅₀ (nM) of about 10,000 or less stimulate ORL-1 opioid-receptor function. In one embodiment, the 4-Oxadiazolyl-piperidine Compounds will have an ORL-1 GTP EC₅₀ (nM) of about 1000 or less. In still another embodiment, the 4-Oxadiazolyl-piperidine Compounds will have an ORL-1 GTP EC₅₀ (nM) of about 100 or less. In still another embodiment, the 4-Oxadiazolyl-piperidine Compounds will have an ORL-1 GTP EC₅₀ (nM) of about 50 or less. In still another embodiment, the 4-Oxadiazolyl-piperidine Compounds will have an ORL-1 GTP EC₅₀ (nM) of about 10 or less.

ORL-1 GTP Emax % is the maximal effect elicited by a compound relative to the effect elicited by nociceptin, a standard ORL-1 agonist. Generally, the ORL-1 GTP Emax (%) value measures the efficacy of a compound to treat or prevent pain or diarrhea. Typically the 4-Oxadiazolyl-piperidine Compounds have an ORL-1 GTP Emax (%) of greater than 50%. In one embodiment the 4-Oxadiazolyl-piperidine Compounds will have an ORL-1 GTP Emax (%) of greater than 75%. In still another embodiment the 4-Oxadiazolyl-piperidine Compounds will have an ORL-1 GTP Emax (%) of greater than 88%. In still another embodiment the 4-Oxadiazolyl-piperidine Compounds will have an ORL-1 GTP Emax (%) of greater than 100%.

In the Table 1 below, the respective parameters Ki, GTP EC 50 and GTP Emax for the µ, ORL-1, δ and κ receptor are summarized for the compounds AFE, AFL, AFX and AGE.

**Table 1**

| **Compound** | **µ** | | | **ORL1** | | |
|---|---|---|---|---|---|---|
| | **Ki (nM)** | **GTP EC50 (nM)** | **GTP Emax** | **Ki (nM)** | **GTP EC50 (nM)** | **GTP Emax** |
| | | | | | | |
| AFE | 5.92 | 16.11 | 116.50 | 1840.74 | | |
| | | | | | | |
| AFL | 1.11 | 1.04 | 116.67 | 695.88 | >20 µm | 28.00 |
| | | | | | | |
| AFX | 4.82 | 8.09 | 90.00 | 1293.04 | | |
| | | | | | | |
| AGE | 1.29 | 2.03 | 103.33 | 3846.20 | | |
| | | | | | | |

| **Compound** | **δ** | | | κ | | |
|---|---|---|---|---|---|---|
| | **Ki (nm)** | **GTP EC50 (nM)** | **GTP Emax** | **Ki (nM)** | **GTP EC50 (nM)** | **GTP Emax** |
| | | | | | | |
| AFE | 3619.47 | | | 151.62 | 1256.66 | 44.67 |
| | | | | | | |
| AFL | 223.18 | 84.04 | 114.5 | 32.91 | 1681.48 | 52.67 |
| | | | | | | |
| AFX | 2321.18 | | | 365.15 | 3832.77 | 41.67 |
| | | | | | | |
| AGE | 340.64 | 21.53 | 104.33 | 91.91 | 703.59 | 56.00 |
| | | | | | | |
| | | | | | | |
| | | | | | | |

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

A number of references have been cited, the entire disclosures of which are incorporated herein by reference.

Further embodiments of this invention are as follows:
1. A compound of formula (1): 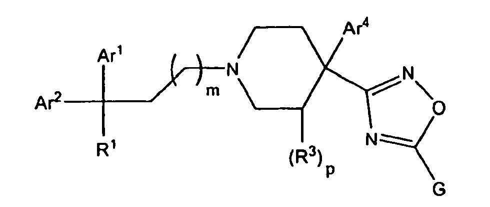 or a pharmaceutically acceptable salt thereof, wherein:
   Ar¹ is -C₃-C₈ cycloalkyl, phenyl, naphthyl, anthryl, phenanthryl or -(5- to 7-membered) heteroaryl, each being unsubstituted or substituted with one or more R₂ groups;
   Ar² is phenyl, naphthyl, anthryl, phenanthryl or -(5- to 7-membered) heteroaryl, each being unsubstituted or substituted with one, two, or three R² groups;
   Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;
   G is -H, -C(O)(CH₂)ₙCO₂R⁴, -C(O)(CH₂),R⁵, -(C₁-C₅ alkylene)C(O)OR⁴, or -(C₁-C₅ alkylene)R⁵;
   R¹ = -H, -C(O)NH₂, -C(O)NHOH, -CO₂R⁴, -CHO, -CN, -(C₁-C₄ alkyl), -C(O)NH(C₁-C₄ alkyl), -C(O)N(C₁-C₄ alkyl)₂, -CF₃, -CHF₂, -CH₂F, 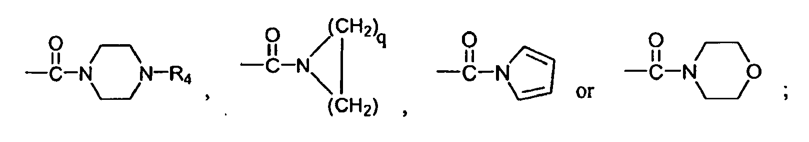
   R² and R³ are each independently -halogen, -C₁-C₃ alkyl, -O(C₁-C₃ alkyl), -NH(C₁-C₃ alkyl), -N(C₁-C₃ alkyl)₂, -CF₃ or -OCF₃;
   R⁴ = -H, -C₁-C₁₀ alkyl, -CH₂O(C₁-C₄ alkyl), -CH₂N(C₁-C₄ alkyl)₂, or -CH₂NH(C₁-C₄ alkyl);
   R⁵ = -NH₂, -NHS0₂R⁴, -C(O)NH₂, -C(O)NHOH, -S0₂NH₂, -C(O)NH(C₁-C₄ alkyl), -C(O)N(C₁-C₄ alkyl)₂, -SO₂NH(C₁-C₄ alkyl), -SO₂N(C₁-C₄ alkyl)₂, -H, -OH, -CN, -C₃-C₈ cycloalkyl, phenyl, naphthyl, anthryl, phenanthryl, or -(5- to 7-membered) heteroaryl, each -C₃-C₈ cycloalkyl, phenyl, naphthyl, anthryl, phenanthryl, or -(5- to 7-membered) heteroaryl being unsubstituted or substituted with one or more R² groups;
      m = an integer ranging from 0 to 4;
      n = an integer ranging from 1 to 4;
      p = 0 or 1; and
      q = an integer ranging from 1 to 6.
2. 2. A compound of formula (II): 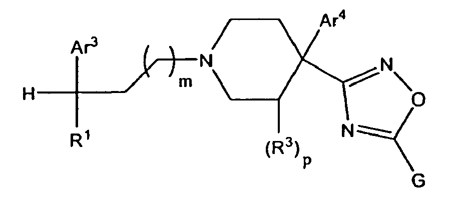 or a pharmaceutically acceptable salt thereof, wherein:
   Ar³ is phenyl, naphthyl, anthryl, phenanthryl, or -(5- to 7-membered) heteroaryl, each being unsubstituted or substituted with one, two, or three R² groups;
   Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;
   G = -H, -C(O)(CH₂)ₙC(O)OR⁴, -C(O)(CH₂)ₙR⁵, -(C₁-C5 alkylene)C(O)OR ⁴, or -(C₁-C₅ alkylene)R⁵;
   R¹ = H, -C(O)NH₂, -C(O)NHOH, -CO₂R₄, -CHO, -CN, -(C₁-C₄ alkyl), -C(O)NH(C₁-C₄ alkyl), -C(O)N(C₁-C₄ alkyl)₂, -CF₃, -CHF₂, -CH₂F, 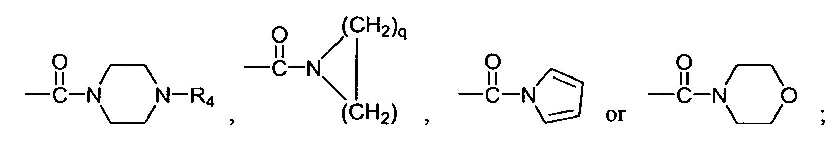
   R² and R³ are each independently halogen, -C₁-C₃ alkyl, -O(C₁-C₃ alkyl), -NH(C₁-C₃ alkyl), -N(C₁-C₃ alkyl)₂, -CF₃, or -OCF₃;
   R⁴ = -H, -C₁-C₁₀ alkyl, -CH₂O(C₁-C₄ alkyl), -CH₂N(C₁-C₄ alkyl)₂, or -CH₂NH(C₁-C₄ alkyl);
   R⁵ = -NH₂, -NHS0₂R ⁴, -C(O)NH₂, -C(O)NHOH, - S0₂NH₂, -C(O)NH(C₁-C₄ alkyl), -C(O)N(C₁-C₄ alkyl)₂, -SO₂NH(C₁-C₄ alkyl), -S0₂N(C₁-C₄ alkyl)₂, -H, -OH, -CN, -C₃-C₈ cycloalkyl, phenyl, naphthyl, anthryl, phenanthryl, or -(5- to 7-membered) heteroaryl, each -C₃-C₈ cycloalkyl, phenyl, naphthyl, anthryl, phenanthryl, or -(5- to 7-membered) heteroaryl, being unsubstituted or substituted with one, two, or three R² groups;
      m = an integer ranging from 0 to 4;
      n = an integer ranging from 1 to 4;
      p = 0 or 1; and
      q = an integer ranging from 1 to 6.
3. The compound of item 1, wherein Ar¹, Ar² , and Ar⁴ are phenyl
   item
4. The compound of item 2, wherein Ar³, and Ar⁴ are phenyl.
5. The compound of item 1 or 2, wherein m = 1 and G = H.
   item
6. The compound of item 1 or 2 wherein R¹ is -C(O)NH₂, -C(O)NH(C₁-C₄ alkyl), or -C(0)N(C₁-C₄ alkyl) (C₁-C₄ alkyl).
7. The compound Item 1 or 2, wherein R¹ is -CN.
8. The compound of item 1 or 2, wherein m = 1, p = 0 and q = 3.
9. The compound of item 1 or 2, wherein G = -(CH₂)₂NHS0₂H.
10. The compound of item 1 or 2, wherein G = -CH₂C(O)NH₂, - CH₂C(O)NH(C₁-C₄ alkyl) or-CH₂C(0)N(C₁-C₄ alkyl) (C₁-C₄ alkyl), preferably -CH₂C(O)NH₂.
11. The compound of item 1 or 2, wherein G = -CH₂C(O)OCH₂CH₃.
12. The compound of item 10 or 11, wherein R¹ = -C(O)N(CH₃)₂
13. The compound of item 10 or 11, wherein R¹ is 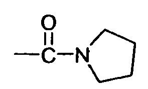
14. The compound of item 1 or 2, wherein G = -(CH₂)₂C(0)OCH₂CH₃.
   item
15. The compound of item 1 or 2, wherein G = -(CH₂)₄C(O)OCH₂CH₃-item
16. The compound of item or 2, wherein p = 1.
   item
17. A composition comprising a compound of item 1 or 2 and a
   pharmaceutically acceptable carrier or excipient.
18. The composition of item 17, further comprising at least one compound selected from the group consisting of an opioid analgesic, a non-opioid analgesic, and an anti-emetic agent.
19. A method for treating pain in an animal, comprising administering to an
   item animal in need thereof an effective amount of a compound of item 1 or 2, preferably further comprising administering an effective amount of at least one compound selected from the group consisting of an opioid analgesic, a non-opioid analgesic, and an anti-emetic agent.
20. A method for stimulating opioid-receptor function in a cell, comprising contacint a cell capable of expressing an opioid receptor with an effective
   item amount of a compound of item 1 or 2.
   item
21. The method of item 20, wherein the receptor is selected from the group consisting of a κ-opioid receptor, a µ-opioid receptor, a δ-opioid receptor, and an ORL-1 receptor.
22. A method for preparing a composition, the method comprising admixing a compound of item 1 or 2 and a pharmaceutically acceptable carrier or excipient.
23. A kit comprising a container containing the composition of item 17, preferably further comprising an anti-diarrheal agent.
24. Use of a compound of item1 or 2 or a pharmaceutically acceptable salt thereof in the preparation of a medicament in the treatment or prevention of pain or diarrhea.
25. A compound of formula 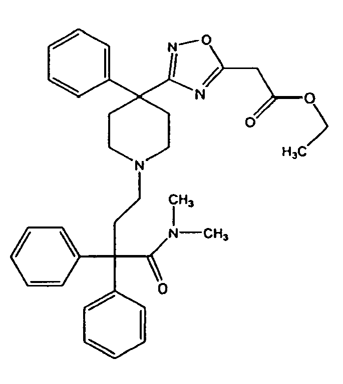 or a pharmaceutical salt thereof.
26. A compound of formula 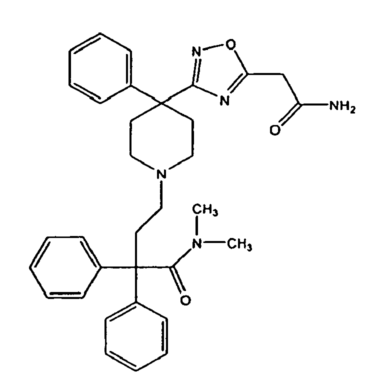 or a pharmaceutical salt thereof.
27. A compound of formula 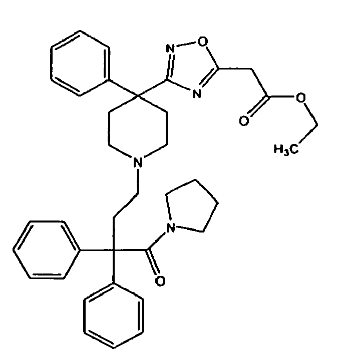 or a pharmaceutical salt thereof.
28. A compound of formula 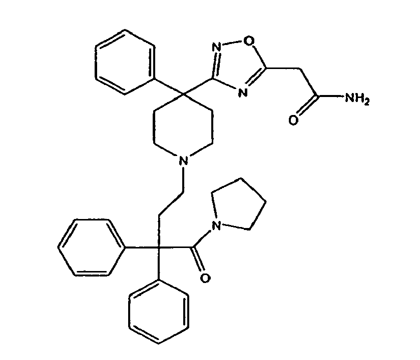 or a pharmaceutical salt thereof.
29. Use of a compound of item 1 or 2 or a pharmaceutically acceptable salt thereof as a medicament.
30. Use of a compound for item 1 or 2 or a pharmaceutically acceptable salt thereof in the preparation of a medicament for stimulating opioid-receptor function in a cell comprising contacting a cell capable of expressing an opioid receptor with an effective amount of the compound of item 1 or 2 or a pharmaceutically acceptable salt thereof.

## Claims

1. A compound of formula (I): 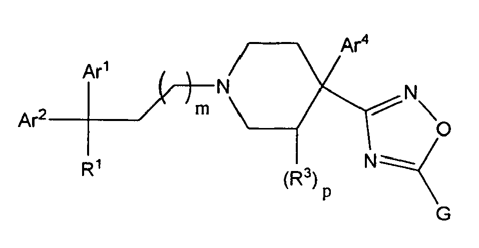 or a pharmaceutically acceptable salt thereof, wherein:
Ar¹ is -C₃-C₈ cycloalkyl, phenyl, naphthyl, anthryl, phenanthryl or -(5- to 7-membered) heteroaryl, each being unsubstituted or substituted with one or more R₂ groups;
Ar² is phenyl, naphthyl, anthryl, phenanthryl or -(5- to 7-membered) heteroaryl, each being unsubstituted or substituted with one, two, or three R²groups;
Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;
G is -H, -C(O)(CH₂)ₙCO₂R⁴, -C(O)(CH₂)ₙR⁵, -(C₁-C₅ alkylene)C(O)OR⁴, or -(C₁-C₅ alkylene)R⁵;
R¹ = -H, -C(O)NH₂, -C(O)NHOH, -CO₂R⁴, -CHO, -CN, -(C₁-C₄ alkyl), -C(O)NH(C₁-C₄ alkyl), -C(O)N(C₁-C₄ alkyl)₂, -CF₃, -CHF₂, -CH₂F, 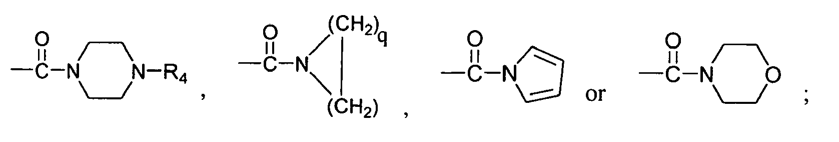
R² and R³ are each independently -halogen, -C₁-C₃ alkyl, -0(C₁-C₃ alkyl), -NH(C₁-C₃ alkyl), -N(C₁-C₃ alkyl)₂, -CF₃ or -OCF₃;
R⁴ = -H, -C₁-C₁₀ alkyl, -CH₂O(C₁-C₄ alkyl), -CH₂N(C₁-C₄alkyl)₂, or -CH₂NH(C₁-C₄ alkyl);
R⁵ = -NH₂, -NHS0₂R ⁴, -C(O)NH₂, -C(O)NHOH, -SO₂NH₂, -C(O)NH(C₁-C₄ alkyl), -C(O)N(C₁-C₄ alkyl)₂, -S0₂NH(C₁-C₄ alkyl), -S0₂N(C₁-C₄ alkyl)₂, -H, -OH, -CN, -C₃-C₈ cycloalkyl, phenyl, naphthyl, anthryl, phenanthryl, or -(5- to 7-membered) heteroaryl, each -C₃-C₈ cycloalkyl, phenyl, naphthyl, anthryl, phenanthryl, or -(5- to 7-membered) heteroaryl being unsubstituted or substituted with one or more R² groups;
m = an integer ranging from 0 to 4;
n = an integer ranging from 1 to 4;
p = 0 or 1; and
q = an integer ranging from 1 to 6.

2. A compound of formula (II): 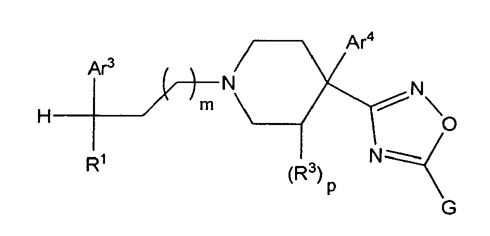 or a pharmaceutically acceptable salt thereof, wherein:
Ar³ is phenyl, naphthyl, anthryl, phenanthryl, or -(5- to 7-membered) heteroaryl, each being unsubstituted or substituted with one, two, or three R²groups;
Ar⁴ is -C₃-C₈ cycloalkyl, phenyl, naphthyl, anthryl, phenanthryl or -(5- to 7-membered) heteroaryl, each being unsubstituted or substituted with one, two, or three R² groups;
G = -H, -C(O)(CH₂)ₙC(O)OR⁴, -C(O)(CH₂)ₙR ⁵, -(C₁-C₅ alkylene)C(O)OR⁴, or -(C₁-C₅ alkylene)R⁵ ;
R¹ = H, -C(O)NH₂, -C(O)NHOH, -CO₂R₄, -CHO, -CN, -(C₁-C₄ alkyl), -C(O)NH(C₁-C₄ alkyl), -C(O)N(C₁-C₄ alkyl)₂, -CF₃, -CHF₂, -CH₂F, 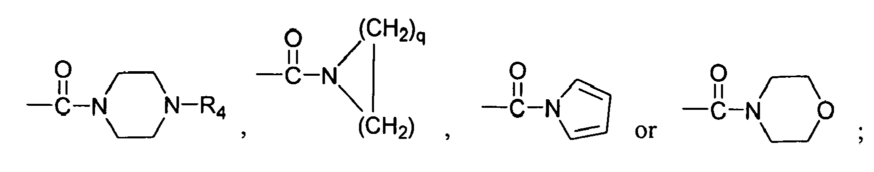
R² and R³ are each independently halogen, -C₁-C₃ alkyl, -O(C₁-C₃ alkyl), -NH(C₁-C₃ alkyl), -N(C₁-C₃ alkyl)₂, -CF₃, or -OCF₃;
R⁴ = -H, -C₁-C₁₀ alkyl, -CH₂O(C₁-C₄ alkyl), -CH₂N(C₁-C₄ alkyl)₂, or -CH₂NH(C₁-C₄ alkyl);
R⁵ = -NH₂, -NHSO₂R⁴, -C(O)NH₂, -C(O)NHOH, - S0₂NH₂, -C(O)NH(C₁-C₄ alkyl), -C(O)N(C₁-C₄ alkyl)₂, -SO₂NH(C₁-C₄ alkyl), -SO₂N(C₁-C₄ alkyl)₂, -H, -OH, -CN, -C₃-C₈ cycloalkyl, phenyl, naphthyl, anthryl, phenanthryl, or -(5- to 7-membered) heteroaryl, each -C₃-C₈ cycloalkyl, phenyl, naphthyl, anthryl, phenanthryl, or -(5- to 7-membered) heteroaryl, being unsubstituted or substituted with one, two, or three R² groups;
m = an integer ranging from 0 to 4;
n = an integer ranging from 1 to 4;
p = 0 or 1; and
q = an integer ranging from 1 to 6.

3. The compound of claim 1, wherein Ar¹, Ar² , and Ar⁴ are phenyl.

4. The compound of claim 2, wherein Ar³, and Ar⁴ are phenyl.

5. The compound of claim 1 or 2, wherein m = 1 and G = H.

6. The compound of claim 1 or 2 wherein R¹ is
(a) -C(O)NH₂, -C(O)NH(C₁-C₄ alkyl), or -C(O)N(C₁-C₄ alkyl) (C₁-C₄ alkyl), or
(b) -CN

7. The compound of claim 1 or 2, wherein m = 1, p = 0 and q = 3.

8. The compound of claim 1 or 2, wherein
(a) G = -(CH₂)₂NHS0₂H, or
(b) G = -CH₂C(O)NH₂, -CH₂C(O)NH(C₁-C₄ alkyl) or -CH₂C(O)N(C₁-C₄ alkyl) (C₁-C₄ alkyl), and preferably is -CH₂C(O)NH₂, or
(c) G = -CH₂C(O)OCH₂CH₃

9. The compound of claim 8, option (b) or (c), wherein
(a) R¹ = -C(O)N(CH₃)₂ or
(b) R¹ is

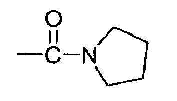

10. The compound of claim 1 or 2, wherein G = -(CH₂)₂C(O)OCH₂CH₃ or G = -(CH₂)₄C(O)OCH₂CH₃.

11. The compound of claim 1 or 2, wherein p = 1.

12. A composition comprising a compound of claim 1 or 2 and a pharmaceutically acceptable carrier or excipient and preferably the composition further comprising at least one compound selected from the group consisting of an opioid analgesic, a non-opioid analgesic, and an anti-emetic agent..

13. A method for preparing a composition, the method comprising admixing a compound of claim 1 or 2 and a pharmaceutically acceptable carrier or excipient.

14. A kit comprising a container containing the composition of claim 12, preferably further comprising an anti-diarrheal agent.

15. Use of a compound of claim 1 or 2 or a pharmaceutically acceptable salt thereof in the preparation of a medicament in the treatment or prevention of pain or diarrhea, and the use preferably further comprising administering an effective amount of at least one compound selected from the group consisting of an opioid analgesic, a non-opioid analgesic, and an anti-emetic agent.

16. Use of a compound of claim 1 or 2 or a pharmaceutically acceptable salt thereof as a medicament.

17. Use of a compound for claim 1 or 2 or a pharmaceutically acceptable salt thereof in the preparation of a medicament for stimulating opioid-receptor function in a cell comprising contacting a cell capable of expressing an opioid receptor with an effective amount of the compound of claim 1 or 2 or a pharmaceutically acceptable salt thereof and preferably the receptor is selected from the group consisting of a κ-opioid receptor, a µ-opioid receptor, a δ-opioid receptor or an ORL-1 receptor.

18. Compound according to claim 1 or 2 or a pharmaceutically acceptable salt thereof for use in the treatment of pain or diarrhea or the the stimulation of an opioid-receptor function in a cell.
